(19) Europäisches Patentamt
European Patent Office
Office européen des brevets

(11) EP 4 238 491 A1

(12) **EUROPEAN PATENT APPLICATION**
published in accordance with Art. 153(4) EPC

(43) Date of publication:
**06.09.2023 Bulletin 2023/36**

(21) Application number: **21886360.3**

(22) Date of filing: **28.10.2021**

(51) International Patent Classification (IPC):
***A61B 5/02*** *(2006.01)* ***A61B 5/0245*** *(2006.01)*
***A61B 5/11*** *(2006.01)*

(52) Cooperative Patent Classification (CPC):
**A61B 5/02; A61B 5/0245; A61B 5/11**

(86) International application number:
**PCT/JP2021/039924**

(87) International publication number:
**WO 2022/092243 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.10.2020 JP 2020180963**
**28.06.2021 JP 2021107109**

(71) Applicant: **Delta Tooling Co., Ltd.**
**Hiroshima-shi, Hiroshima 736-0084 (JP)**

(72) Inventors:
- **FUJITA, Etsunori**
**Hiroshima-shi, Hiroshima 736-0084 (JP)**
- **OGURA, Yumi**
**Hiroshima-shi, Hiroshima 736-0084 (JP)**
- **MAEDA, Shinichiro**
**Hiroshima-shi, Hiroshima 736-0084 (JP)**
- **KOJIMA, Shigeyuki**
**Hiroshima-shi, Hiroshima 736-0084 (JP)**
- **HORIKAWA, Masahiro**
**Hiroshima-shi, Hiroshima 736-0084 (JP)**
- **NOBUHIRO, Yoshika**
**Hiroshima-shi, Hiroshima 736-0084 (JP)**
- **KANEKO, Shigehiko**
**Kawaguchi-shi, Saitama 333-0854 (JP)**
- **YOSHIZUMI, Masao**
**Hiroshima-shi, Hiroshima 730-0014 (JP)**

(74) Representative: **Hoffmann Eitle**
**Patent- und Rechtsanwälte PartmbB**
**Arabellastraße 30**
**81925 München (DE)**

(54) **BIOLOGICAL SIGNAL ANALYSIS DEVICE, COMPUTER PROGRAM, AND RECORDING MEDIUM**

(57) To make it possible to grasp a biological condition, in particular, a condition ascribable to the movement of the heart. According to the present invention, it is possible to find a boundary frequency between cardiac apex beat and heart sound. This makes it possible to find not only heart sound but also cardiac apex beat, from a biological signal which is a collection of biological sound and in-vivo vibration. The boundary frequency is represented by a quadratic function of heart rate, and the use of correlation data based on the quadratic function makes it possible to find heart rate or a boundary frequency in real time. As a result, for example, if heart rate is known, the level of a boundary frequency is known. This facilitates inferring a health condition, for example, whether or not the heart is under stress, from a broader viewpoint than conventionally.

EP 4 238 491 A1

FIG. 12

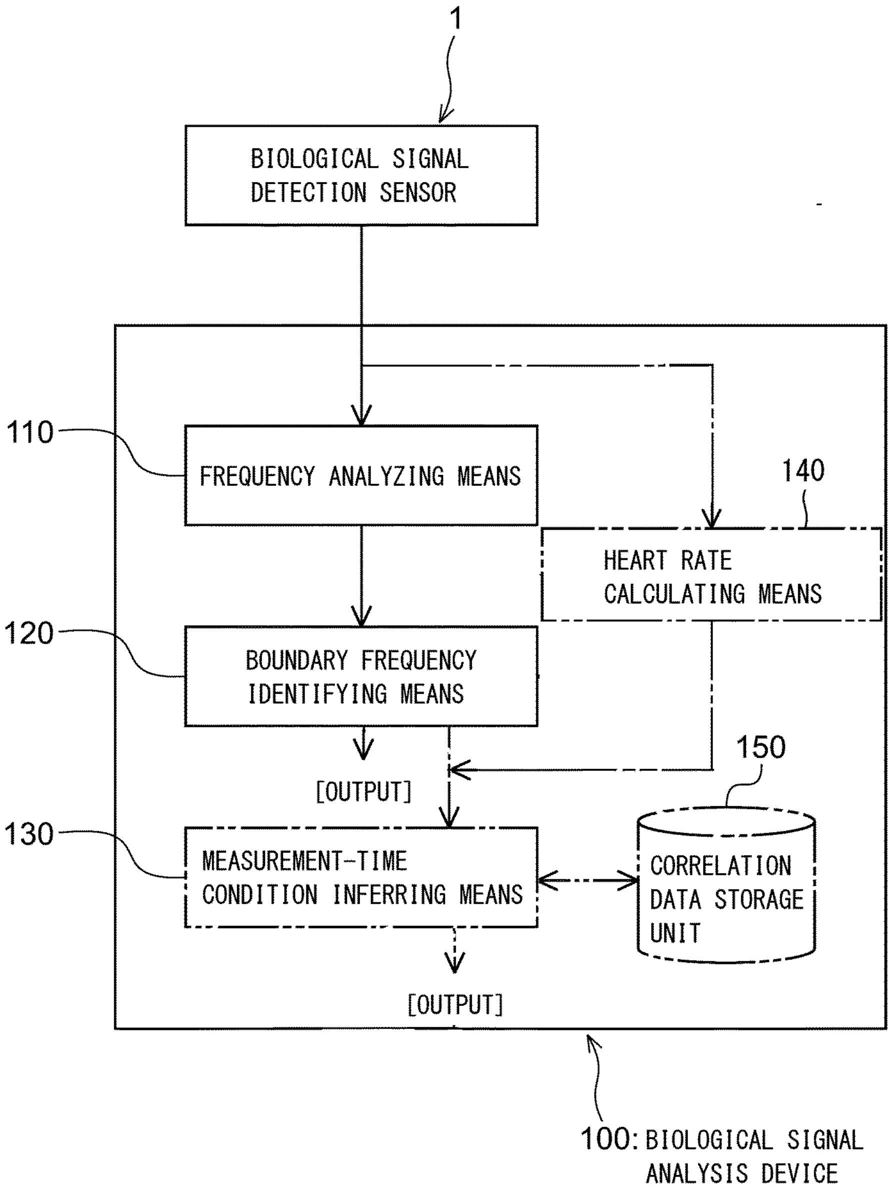
1
BIOLOGICAL SIGNAL DETECTION SENSOR
110
FREQUENCY ANALYZING MEANS
140
HEART RATE CALCULATING MEANS
120
BOUNDARY FREQUENCY IDENTIFYING MEANS
[OUTPUT]
150
130
MEASUREMENT-TIME CONDITION INFERRING MEANS
CORRELATION DATA STORAGE UNIT
[OUTPUT]
100: BIOLOGICAL SIGNAL ANALYSIS DEVICE

## Description

### Technical Field

The present invention relates to a technique regarding the inference of a biological condition, and in particular, relates to a biological signal analysis device using an index relating to cardiac apex beat, a computer program, and a recording medium.

### Background Art

Cardiac apex beat reflects the movement of the apex of the left ventricle and is used in the evaluation of the function of the heart, mainly, the left ventricle. Normally, cardiac apex beat is objectively represented by an ACG (Apex cardiogram) recorded by a MCG (Mechanocardiogram) method, but the apex cardiogram is likely to be affected by recording skill because it captures the movement of the chest wall through various tissues between the heart and the chest wall. Further, mechanocardiogram recording devices are often relatively large and have restrictions that their measurement rooms have to be soundproof.

Considering this, Patent Document 1 discloses a bioinformation measurement device for apex cardiogram, including: an ultrasonic transmitting part causing an ultrasonic signal to be incident toward the vicinity of the cardiac apex from the body surface; and a receiving sensor that receives a reflected ultrasonic wave. The device is capable of easily creating an apex cardiogram even when it is not in a special environment such as a soundproof room.

The present inventors have proposed, in Patent Documents 2 to 5 and so on, arts for capturing vibration generated on the dorsal body surface of a person in a non-constraining manner and inferring the state of the person by analyzing the vibration. The vibration generated on the dorsal body surface of a person is vibration propagated from a human body inner part such as the heart and the aorta and contains information on atrial and ventricular systoles and diastoles, information on vascular wall elasticity which serves as an auxiliary pump for circulation, and information on reflected waves.

In Patent Document 2, slide calculation is performed in which a predetermined time width is set in a time-series waveform of a dorsal body surface pulse wave of around 1 Hz extracted from vibration (biological signal) propagated through the body surface, to find a frequency slope time-series waveform, and from the tendency of its variation, for example, based on whether its amplitude is on the increase or on the decrease, a biological condition is inferred. It is also disclosed that power spectra of frequencies respectively corresponding to a function regulation signal, a fatigue reception signal, and an activity regulation signal that belong to a predetermined range from ULF band (ultra-low-frequency band) to VLF band (very-low-frequency band) are found through frequency analysis of the biological signals, and the state of a person is determined from time-series variations of the respective power spectra.

Patent Documents 3, 4 disclose a means for determining a homeostasis function level. Further, Patent Document 5 discloses a sound/vibration information collection mechanism including a resonance layer including a natural oscillator having a natural frequency corresponding to sound/vibration information of a biological signal.

Further, Non-patent Document 1 teaches that heart sound is composed of random vibrations. Non-patent Document 2 discloses that waveforms of the aforesaid apex cardiogram are classified and the result is used for diagnosis. Non-patent Document 3 gives an explanation regarding the fluctuation of cardiac cycle.

### Prior Art Document

#### Patent Document

Patent Document 1: WO2012/165660
Patent Document 2: Japanese Patent Application Laid-open No. 2011-167362
Patent Document 3: Japanese Patent Application Laid-open No. 2014-117425
Patent Document 4: Japanese Patent Application Laid-open No. 2014-223271
Patent Document 5: Japanese Patent Application Laid-open No. 2016-26516

#### Non-patent Document

Non-patent Document 1: Nobuhiro, Y et al. Development of Physical Condition Fluctuation Prediction Model Using Trunk Biosignals, Proceeding of Asia-Pacific Vibration Conference 2019 (2019)
Non-patent Document 2: Yoshikawa, J. et al. Physical Examination in Cardiology, Bunkodo Shoten Co., Ltd., 79-95 (2005)
Non-patent Document 3: Kobayashi M., Musha T.: 1/f fluctuation of heart beat period. IEEE Transactions on biomedical engineering. BME, 29, 45-457 (1982)

## Summary of the Invention

### Problems to Be Solved by the Invention

What Patent Document 1 discloses is a device using 192 ultrasonic oscillators, out of which twelve ultrasonic oscillators compose each set, and it scans while shifting the sets in sequence, to create an apex cardiogram. Further, for creating a more accurate apex cardiogram, it also discloses a device using a plurality of arrays of ultrasonic oscillators, the arrays each being composed of 192 ultrasonic oscillators. In Patent Document 1, it is difficult to obtain a desired apex cardiogram unless many ultrasonic oscillators are thus used. Therefore, a product unavoidably becomes very expensive and is not practical. Further, to create a clear apex cardiogram, care has to be taken to the detection of the rib position, a transmission frequency is limited, the positions of receiving elements are limited, and the device needs to satisfy these conditions, which also leads to a complicated structure and a higher manufacturing cost.
Further, Patent Document 1 states as a merit that the device requires only one person for the measurement while conventional mechanocardiogram recording devices require a plurality of people for handling. However, the scanning with the ultrasonic oscillators requires the operation by a doctor or an engineer, and thus the measurement takes a lot of time and trouble, and only data during the scanning can be obtained. That is, the device is not suitable for capturing cardiac apex beat of a measurement subject over long hours.
On the other hand, the sensor that captures the vibration generated on the body surface in a non-constraining manner as is disclosed in Patent Documents 2 to 5 is composed of a three-dimensional knitted fabric, a film surrounding the three-dimensional knitted fabric, a microphone, and so on, and is capable of obtaining biological signals through the body surface only by being in contact with the person. That is, the device can obtain biological signal data only by being attached to the body of the person, without any operation by a doctor or the like. However, in Patent Documents 2 to 5, an autonomic nervous function and heart rate variability are mainly analyzed, and an analysis regarding cardiac apex beat which is the movement of the apex of the left ventricle is not done.
Further, the sensors used in Patent Documents 2 to 5 are suitable for passively capturing acoustic vibration of 20 to 100 Hz propagated from the dorsal body surface of a person but are not suitable for extracting cardiac apex beat which is in-vivo vibration of 10 Hz or lower.
Diagnosing using the apex cardiogram of Non-patent Document 3 requires professional knowledge for reading the waveform of the apex cardiogram, and the determination result may differ depending on each determiner, and thus there is still room for improvement in terms of determination accuracy and promptness. Therefore, the diagnosis using the apex cardiogram is only for auxiliary use. Moreover, such an apex cardiogram is used for diagnosis as information relating to cardiac apex beat, though auxiliary, but the inference of various health conditions (the presence/absence of a disease such as heart disease, the identification of a disease, whether the physical condition is good or not, and so on) of a person using an index relating to cardiac apex beat, other than the apex cardiogram, has not been done.
The present invention was made in consideration of the above, and has an object to provide a biological signal analysis device that can be manufactured easily and at a low cost, is capable of long-time measurement, is capable of extracting information regarding cardiac apex beat by analyzing biological signals collected by a biological signal detection sensor, and further is capable of inferring various health conditions of a person, and to provide a computer program and a recording medium.

### Means for Solving the Problems

To solve the aforesaid problems, the present inventors have developed a biological signal detection sensor that includes a three-dimensional knitted fabric, a microphone, and so on and is capable of obtaining biological signal data of 0.5 to 80 Hz. The biological signal detection sensor only passively captures acoustic vibration propagated from the body surface, and the captured biological signal data contains various biological sounds and in-vivo vibrations. As a result of widening a collectable frequency band to a low-frequency band, the biological signal data contains data regarding cardiac apex beat, but we faced a new problem that the data of the cardiac apex beat is hidden behind data of heart sound and thus the extraction of only the data of the cardiac apex beat is difficult. Under such circumstances, as a result of intensive studies for solving such a problem, the present inventors have completed the present invention.
Specifically, the biological signal analysis device of the present invention includes:

a frequency analyzing means that frequency-analyzes biological signal data obtained by a biological signal detection sensor through a body surface; and
a boundary frequency identifying means that finds a boundary frequency between vibration generated by cardiac apex beat and vibration generated by heart sound in the biological signal data, from a result of the frequency analysis of the biological signal data which result is obtained from the frequency analyzing means.

Preferably, the boundary frequency identifying means includes a means that finds, in the result of the frequency analysis, a power spectrum sudden changing point which is a boundary between harmonic vibration and random vibration, and identifies the boundary frequency based on the sudden changing point.
Preferably, the boundary frequency identifying means includes a means that finds the power spectrum sudden changing point, in consideration of a result of a frequency analysis of heart sound data measured simultaneously.
Preferably, the boundary frequency identifying means includes:

a log-log plot means that represents, in log-log axes, a waveform resulting from addition averaging of the results of the frequency analyses of the biological signal data and the heart sound data, using a log difference method; and
a sudden changing point identifying means that finds a fluctuation changing point from the waveform represented in log-log axes, and identifies the fluctuation changing point as the power spectrum sudden changing point.

Preferably, as the frequency analyzing means, short time Fourier transform is employed, and
the boundary frequency identifying means includes a means that finds the power spectrum sudden changing point from an analysis result of the short time Fourier transform.
Preferably, the frequency analyzing means includes a means that outputs the analysis result of the short time Fourier transform as image data showing time, frequency, and a degree of power spectrum variation, and
the boundary frequency identifying means includes a means that finds the power spectrum sudden changing point from the image data.
Preferably, the biological signal analysis device further includes:

a correlation data storage unit in which correlation data relating to the boundary frequency is stored; and
a measurement-time condition inferring means that infers a measurement-time health condition of a measurement subject, by referring to the correlation data.

Preferably, the correlation data is correlation data of the boundary frequency and heart rate, and
the measurement-time condition inferring means includes a means that infers a measurement-time boundary frequency of the measurement subject by collating a measurement-time heart rate of the measurement subject with the correlation data of the boundary frequency and the heart rate.
Preferably, the correlation data is correlation data of the boundary frequency and a fluctuation characteristic of heart rate variability, and
the measurement-time condition inferring means includes a means that infers a measurement-time fluctuation characteristic of heart rate variability of the measurement subject by collating a measurement-time boundary frequency of the measurement subject with the correlation data of the boundary frequency and the fluctuation characteristic of the heart rate variability.
Preferably, the biological signal analysis device further includes:

a cardiac apex beat waveform extracting means that filters the biological signal data, with an upper limit value being set to the boundary frequency identified by the boundary frequency identifying means, to find a waveform of the vibration generated by the cardiac apex beat; and
a waveform classifying means that classifies the waveform of the vibration generated by the cardiac apex beat, and the measurement-time condition inferring means includes a means that causes the waveform classifying means to classify a measurement-time waveform of the vibration generated by the cardiac apex beat of the measurement subject, which waveform is obtained by the cardiac apex beat waveform extracting means, and infers the measurement-time health condition of the measurement subject based on data of a result of the classification of the measurement-time waveform.

Preferably, correlation data of a result of the waveform classification and health condition is stored in the correlation data storage unit in advance, and
the measurement-time condition inferring means includes a means that causes the waveform classifying means to classify the measurement-time waveform of the vibration generated by the cardiac apex beat of the measurement subject, which waveform is obtained by the cardiac apex beat waveform extracting means, and infers the measurement-time health condition of the measurement subject by collating the result of the classification of the measurement-time waveform with the pre-stored correlation data of the waveform classification result and the health condition.

Preferably, the biological signal analysis device includes a model creating means that uses information on waveforms and the health conditions as training data, the waveforms being output by the cardiac apex beat waveform extracting means to be classified by the waveform classifying means, to create, by machine learning, an inference model for inferring the health condition from the waveform information, and

the measurement-time condition inferring means includes a means that receives the information of the measurement-time waveform of the vibration generated by the cardiac apex beat of the measurement subject, and outputs a value of the measurement-time health condition based on the obtained information of the measurement-time waveform, using the inference model created by the model creating means.

Preferably, the waveform classifying means uses the following means (A) and (B):

(A) a means that classifies the waveform by a mathematical approach using Fourier series expansion; and

(B) a means that classifies the waveform based on a combination of one or two or more of the following data (1) to (4) obtained by engineering approaches:

(1) data indicating a shape of a time waveform in a predetermined time range of the waveform of the vibration generated by the cardiac apex beat;

(2) data of a graph of the time waveform, in which frequency and power spectrum are taken on a horizontal axis and a vertical axis, the data being obtained by the frequency analyzing means;

(3) image data in a predetermined time range that is the analysis result of the short time Fourier transform of the time waveform and shows the time, the frequency, and the degree of the power spectrum variation; and

(4) data regarding a type and a period of a waveform, in a predetermined time range, which data is extracted from a correlogram,

to classify waveforms of vibrations generated by the cardiac apex beat of healthy people into five, and

infers whether the measurement-time health condition of the measurement subject is a healthy condition or not based on whether or not the result of the classification of the measurement-time waveform of the measurement subject corresponds to any one of the five results of the waveform classification.

Preferably, the biological signal detection sensor includes:

a three-dimensional knitted fabric;

a housing film that sealingly covers a periphery of the three-dimensional knitted fabric;

a microphone disposed on an outer side of the housing film;

a case covering the microphone; and

an external disturbance mixture preventing member that functions to prevent external disturbance from mixing into the microphone in the case.

Preferably, the external disturbance mixture preventing member is gel.

Further, the present invention provides a computer program causing a computer to process biological signal data that is obtained by a biological signal detection sensor through a body surface, to function as a biological signal analysis device, the computer program causing the computer to execute:

a procedure that frequency-analyzes the biological signal data; and

a procedure that identifies a boundary frequency between vibration generated by cardiac apex beat and vibration generated by heart sound in the biological signal data, from a result of the frequency analysis.

Preferably, the procedure that identifies the boundary frequency finds, in the result of the frequency analysis, a power spectrum sudden changing point which is a boundary between harmonic vibration and random vibration, and identifies the boundary frequency based on the sudden changing point.

Preferably, the procedure that identifies the boundary frequency finds the power spectrum sudden changing point, in consideration of a result of a frequency analysis of heart sound data measured simultaneously.

Preferably, the procedure that identifies the boundary frequency represents, in log-log axes, a waveform resulting from addition averaging of the results of the frequency analyses of the biological signal data and the heart sound data, using a log difference method, finds a fluctuation changing point from the waveform represented in log-log axes, and identifies the fluctuation changing point as the power spectrum sudden changing point.

Preferably, in the frequency analyzing procedure, short time Fourier transform is employed, and

the procedure that identifies the boundary frequency finds the power spectrum sudden changing point from an analysis result of the short time Fourier transform.

Preferably, the frequency analyzing procedure outputs the analysis result of the short time Fourier transform as image data showing time, frequency, and a degree of power spectrum variation, and
the procedure that identifies the boundary frequency finds the power spectrum sudden changing point from the image data.

Preferably, the computer is caused to execute a procedure that infers a measurement-time health condition of a measurement subject, by referring to correlation data that relates to the boundary frequency and is stored in a correlation data storage unit.

Preferably, the correlation data is correlation data of the boundary frequency and heart rate, and
the procedure that infers the measurement-time condition infers a measurement-time boundary frequency of the measurement subject by collating a measurement-time heart rate of the measurement subject with the correlation data of the boundary frequency and the heart rate.

Preferably, the correlation data is correlation data of the boundary frequency and a fluctuation characteristic of heart rate variability, and
the procedure that infers the measurement-time condition infers a measurement-time fluctuation characteristic of heart rate variability of the measurement subject by collating a measurement-time boundary frequency of the measurement subject with the correlation data of the boundary frequency and the fluctuation characteristic of the heart rate variability.

Preferably, the computer is further caused to execute:

> a procedure that filters the biological signal data, with an upper limit value being set to the boundary frequency identified by the boundary frequency identifying means, to find a waveform of the vibration generated by the cardiac apex beat; and
> a procedure that classifies the waveform of the vibration generated by the cardiac apex beat, and
> the procedure that infers the measurement-time condition causes the procedure that classifies the waveform to classify a measurement-time waveform of the vibration generated by the cardiac apex beat of the measurement subject, and infers the measurement-time health condition of the measurement subject based on a result of the classification of the measurement-time waveform.

Preferably, correlation data of a result of the waveform classification and health condition is stored in the correlation data storage unit, and
the procedure that infers the measurement-time condition causes the procedure that classifies the waveform to classify the measurement-time waveform of the vibration generated by the cardiac apex beat of the measurement subject, and infers the measurement-time health condition of the measurement subject by collating the result of the classification of the measurement-time waveform with the correlation data of the waveform classification result and the health condition.

Preferably, information on waveforms and the health conditions are used as training data, the waveforms being output by the execution of the procedure that extracts the cardiac apex beat waveform to be classified by the procedure that classifies the waveform, and an inference model for inferring the health condition is created from the waveform information by machine learning, and
the procedure that infers the measurement-time health condition receives the information of the measurement-time waveform of the vibration generated by the cardiac apex beat of the measurement subject, and outputs a value of the measurement-time health condition based on the obtained information of the measurement-time waveform, using the inference model.

Preferably, the procedure that classifies the waveform uses the following procedures (A) and (B):

> (A) a procedure that classifies the waveform by a mathematical approach using Fourier series expansion; and
> (B) a procedure that classifies the waveform based on a combination of one or two or more of the following data (1) to (4) obtained by engineering approaches:
>
> > (1) data indicating a shape of a time waveform in a predetermined time range of the waveform of the vibration generated by the cardiac apex beat;
> > (2) data of a graph of the time waveform, in which frequency and power spectrum are taken on a horizontal axis and a vertical axis, the data being obtained by the frequency analyzing means;
> > (3) image data in a predetermined time range which is the analysis result of the short time Fourier transform of the time waveform and shows the time, the frequency, and the degree of the power spectrum variation; and
> > (4) data regarding a type and a period of a waveform, in a predetermined time range, which data is extracted from a correlogram,
>
> to classify waveforms of vibrations generated by the cardiac apex beat of healthy people into five, and

infers whether the measurement-time health condition of the measurement subject is a healthy condition or not based on whether or not the result of the classification of the measurement-time waveform of the measurement subject corresponds to any one of the five results of the waveform classification.

Further, the present invention provides a recording medium in which the above-described computer program is recorded.

Effect of the Invention

According to the present invention, it is possible to find a boundary frequency between cardiac apex beat and heart sound. The boundary frequency which is a new index regarding the cardiac apex beat correlates with the fluctuation characteristic of heart rate variability, and the use of the boundary frequency enables the inference of health conditions (the presence/absence of a disease such as a heart disease, the identification of a disease, whether or not the physical condition is good or not, and so on). Further, according to the present invention, since the boundary frequency can be represented by a function of heart rate, the use of correlation data based on this makes it possible to find the boundary frequency in real time.

Further, in the present invention, the waveforms of vibrations generated by cardiac apex beat found by using the aforesaid boundary frequency are classified, and the waveforms are associated with health conditions of people (the presence/absence of a disease such as a heart disease, the identification of a disease, whether or not the physical condition is good or poor, and so on), which enables the inference of a health condition. This makes it possible to automate the evaluation of a waveform of an apex cardiogram which has been done by a professional such as a doctor or the like. In particular, using the waveform information of the cardiac apex beat and the health conditions as training data to create, by machine learning, an inference model for the inference of a health condition from the waveform information makes it possible to improve inference accuracy of the health condition.

Brief Description of Drawings

[FIGs. 1] FIG. 1(a) is an external perspective view illustrating a biological signal detection sensor (4SR) according to a first embodiment of the present invention, FIG. 1(b) is an external perspective view in which an air pack and a gel pack are separately illustrated, and FIG. 1(c) is a sectional view.

[FIG. 2] FIG. 2 is a view illustrating the structure of the biological signal detection sensor (4SR) according to the first embodiment of the present invention as compared with a conventional biological signal detection sensor (3SR).

[FIGs. 3] FIG. 3(a) is a chart illustrating mechanically reproduced sound (PCG reproduction input) of PCG (Phonocardiogram) that is output from a speaker and input to the biological signal detection sensor (4SR) of the embodiment and the conventional biological signal detection sensor (3 SR), and acoustic vibration data transmitted by a three-dimensional knitted fabric, FIG. 3(b) is a chart illustrating frequency components forming a resonance waveform of the PCT reproduction input and a modulated PCG reproduction input, FIG. 3(c) is a chart illustrating Lissajous figures showing spring characteristics and damping characteristics of the biological signal detection sensor (4SR) of the embodiment and the conventional biological signal detection sensor (3SR), FIG. 3(d) illustrates Bode plots, and FIG. 3(e) is a chart illustrating a spring constant of a structure (sealed air pack) of the biological signal detection sensor (4SR) of the embodiment in which the three-dimensional knitted fabric is sealingly housed in a housing film.

[FIGs. 4] FIGs. 4 are charts illustrating amplification effects when the waveform of PCG is input, (a) illustrating output waveforms of the biological signal detection sensor (4SR) of the embodiment and the conventional biological signal detection sensor (3 SR), (b) illustrating the frequency analysis results of these, and (c) illustrating a gain of 4SR/3SR.

[FIGs. 5] FIGs. 5 are charts illustrating APW (Acoustic Pulse Wave) amplification effects, (a) illustrating output waveforms of the biological signal detection sensor (4SR) of the embodiment and the conventional biological signal detection sensor (3 SR), (b) illustrating the frequency analysis results of these, and (c) illustrating a gain of 4SR/3 SR.

[FIG. 6] FIG. 6 is an explanatory view of an experiment for comparing the biological signal detection sensor (4SR) of the first embodiment and a structure having only an air pack without a gel pack.

[FIGs. 7] FIGs. 7(a) to (c) illustrate the experiment results of 4SR, and FIGs. 7(d) to (f) illustrate the experiment results of the structure only with the air pack 1A (4SR without gel pack). FIG. 7(g) is an enlarged chart of (d), and FIG. 7(h) is an enlarged chart of (e). FIG. 7(i) illustrates Bode plots, FIG. 7(j) illustrates load-deflection characteristics of 4SR and the structure only with the air pack 1A, and FIG. 7(k) is a chart illustrating amplitude ratios of these (4SR/4SR without gel pack).

[FIGs. 8] FIGs. 8(a) to (d) are charts illustrating the results of the three-second measurement of time-series waveforms of F-APW, R-APW, L-APW, and PCG of a subject whose average heart rate is 58/min.

[FIGs. 9] FIGs. 9(a) to (d) are charts illustrating the results of the three-second measurement of time-series waveforms of F-APW, R-APW, L-APW, and PCG of a subject whose average heart rate is 71/min.

[FIGs. 10] FIGs. 10(a) to (d) are charts illustrating the results of the three-second measurement of time-series waveforms of F-APW, R-APW, L-APW, and PCG of a subject whose average heart rate is 79/min.

[FIGs. 11] FIGs. 11(a) to (d) are charts illustrating the results of the three-second measurement of time-series waveforms of F-APW, R-APW, L-APW, and PCG of a subject whose average heart rate is 93/min.

[FIG. 12] FIG. 12 is a diagram schematically illustrating the configuration of a biological signal analysis device according to the first embodiment.

[FIG. 13] FIG. 13 is an explanatory flowchart of a process of extracting a boundary frequency.

[FIGs. 14] FIGs. 4(a) to (d) are charts illustrating the frequency analysis results of CAB (Cardiac Apex Beat) and CAS (Cardiac Acoustic Sound) of the subjects whose average heart rates are 58/min, 71/min, 79/min, and 93 min.

[FIG. 15] FIG. 15 is a chart illustrating processed waveforms of Front CAB (0.5-BF), Front CAB (5-BF), Rear CAB (0.5-BF), Lumbar CAB (0.5-BF), PCG, Front CAS (BF-50), Rear CAS (BF-50), and Lumbar CAS (BF-50) obtained from the subject whose average heart rate is 58/min.

[FIG. 16] FIG. 16 is a chart illustrating processed waveforms of Front CAB (0.5-BF), Front CAB (5-BF), Rear CAB (0.5-BF), Lumbar CAB (0.5-BF), PCG, Front CAS (BF-50), Rear CAS (BF-50), and Lumbar CAS (BF-50) obtained from the subject whose average heart rate is 71/min.

[FIG. 17] FIG. 17 is a chart illustrating processed waveforms of Front CAB (0.5-BF), Front CAB (5-BF), Rear CAB (0.5-BF), Lumbar CAB (0.5-BF), PCG, Front CAS (BF-50), Rear CAS (BF-50), and Lumbar CAS (BF-50) obtained from the subject whose average heart rate is 79/min.

[FIG. 18] FIG. 18 is a chart illustrating processed waveforms of Front CAB (0.5-BF), Front CAB (5-BF), Rear CAB (0.5-BF), Lumbar CAB (0.5-BF), PCG, Front CAS (BF-50), Rear CAS (BF-50), and Lumbar CAS (BF-50) obtained from the subject whose average heart rate is 93/min.

[FIGs. 19] FIGs. 19(a) to (d) are charts each illustrating all of ECG, F-APW, and PCG of each of the four subjects whose average heart rates are 58/min, 71/min, 79/min, and 93/min.

[FIGs. 20] FIGs. 20(a) to (d) are charts each illustrating all of ECG, Front CAB, and Front CAS of each of the four subjects whose average heart rates are 58/min, 71/min, 79/min, and 93/min.

[FIGs. 21] FIGs. 20(a) to (d) are charts illustrating power spectral densities (PSD) of Front CAB and Front CAS of the four subjects whose average heart rates are 58/min, 71/min, 79/min, and 93/min.

[FIGs. 22] FIGs. 22 are charts illustrating the STFT analysis results of the subject whose average heart rate in the ten-second measurement time is 93/min, (a) illustrating the analysis result of F-APW, and (b) illustrating the analysis result of PCG.

[FIGs. 23] FIG. 23(a) is a chart illustrating the STFT analysis result of F-APW$\times$PCG$^{-1}$ of the subject whose average heart rate in the ten-second measurement time is 93/min, and FIG. 23(b) is an enlarged chart of FIG. 23(a).

[FIGs. 24] FIG. 24(a) is a chart illustrating the STFT analysis result of F-APW$\times$PCG of the subject whose average heart rate in the ten-second measurement time is 93/min, and FIG. 24(b) is a chart illustrating power spectra of F-APW$\times$PCG$^{-1}$, F-APW, PCG, and F-APW$\times$PCG.

[FIGs. 25] FIG. 25(a) is a chart illustrating power spectra of F-APW$\times$PCG$^{-1}$, F-APW, PCG, and F-APW$\times$PCG of the subject whose average heart rate is 58/min, and FIG. 25(b) is the STFT analysis result of F-APW$\times$PCG$^{-1}$.

[FIGs. 26] FIG. 26(a) is a chart illustrating power spectra of F-APW$\times$PCG$^{-1}$, F-APW, PCG, and F-APW$\times$PCG of the subject whose average heart rate is 71/min, and FIG. 26(b) is the STFT analysis result of F-APW$\times$PCG$^{-1}$.

[FIGs. 27] FIG. 27(a) is a chart illustrating power spectra of F-APW$\times$PCG$^{-1}$, F-APW, PCG, and F-APW$\times$PCG of the subject whose average heart rate is 79/min, and FIG. 27(b) is a chart illustrating the STFT analysis result of F-APW$\times$PCG$^{-1}$.

[FIGs. 28] FIG. 28(a) is a BF-HR correlation chart of fifty subjects, and FIG. 28(b) is a correlation chart of a disappearing order of higher harmonics with HR.

[FIGs. 29] FIG. 29(a) is a chart illustrating a correlation between a heart rate variability fluctuation characteristic and heart rate, FIG. 29(b) is a chart illustrating a correlation between the heart rate variability fluctuation characteristic and BF, and FIG. 29(c) is a chart illustrating a correlation between heart rate and BF of one subject, FIG. 29(d) is a chart illustrating a correlation between heart rate and BF of another subject, and FIG. 29(e) is a chart illustrating a correlation between heart rate and BF of still another subject.

[FIG. 30] FIG. 30 is a schematic diagram illustrating the configuration of a biological signal analysis device according to a second embodiment.

[FIG. 31] is an explanatory schematic diagram of another mode of the biological signal analysis device of the second embodiment.

[FIGs. 32] FIGs. 32(a) to (e) are explanatory charts of the typical waveform analysis results of Front CAB (0.5-BF) in the case of a pseudo sine wave.

[FIGs. 33] FIGs. 33(a) to (e) are explanatory charts of the typical waveform analysis results of Front CAB (0.5-BF) in the case of a triangular wave.

[FIGs. 34] FIGs. 33(a) to (e) are explanatory charts of the typical waveform analysis results of Front CAB (0.5-BF) in the case of a cosine wave.

[FIGs. 35] FIGs. 35(a) to (e) are explanatory charts of the typical waveform analysis results of Front CAB (0.5-BF) in the case of a Gauss wave.

[FIGs. 36] FIGs. 36(a) to (e) are explanatory charts of the typical waveform analysis results of Front CAB (0.5-BF) in the case of a pseudo full-wave rectified wave.

[FIG. 37] FIG. 37 is a chart illustrating the results of waveform studies using two kinds of STFT, power spectrum, and correlogram.

[FIGs. 38] FIGs. 38(a) to (e) are charts illustrating the frequency analysis results of five subjects, sorted by measurement position.

[FIG. 39] FIG. 39 is a chart illustrating amplitude ratios at the respective measurement positions.

[FIG. 40] FIG. 40 is a BF-HR correlation chart when heart rate variability occurs in subjects under respiratory depression in a resting state.

[FIGs. 41] FIG. 41(a) is a BF-HR correlation chart when the subjects are in a supine posture, FIG. 41(b) is a BF-HR correlation chart when the subjects are in a left lateral recumbent posture, and FIG. 41(c) is a BF-HR correlation chart when the subjects are in a right lateral recumbent posture.

Modes for Carrying out the Invention

The present invention will be hereinafter described in more detail based on embodiments of the present invention illustrated in the drawings.

(First Embodiment)

- Biological signal detection sensor

First, the structure of a biological signal detection sensor 1 used in this embodiment will be described based on FIGs. 1(a) to (c). The biological signal detection sensor 1 of this embodiment is a stacked structure of an air pack 1A and a gel pack 1B. The air pack 1A includes a three-dimensional knitted fabric (3D net) 10 and a housing film 20 in which the three-dimensional knitted fabric (3D net) 10 is sealingly housed. In the gel pack 1B, a microphone 30 is disposed and fixed in its case 40, and gel 50 is filled around the microphone 30.

The three-dimensional knitted fabric 10 is formed of a pair of ground knitted fabrics disposed apart from each other and connecting yarns connecting the ground knitted fabrics. For example, the ground knitted fabrics each can be formed to have a flat knitted fabric structure (fine meshes) continuous both in a wale direction and a course direction using yarns of twisted fibers or to have a knitted fabric structure having honeycomb (hexagonal) meshes. The connecting yarns impart certain rigidity to the three-dimensional knitted fabric so that one of the ground knitted fabrics and the other ground knitted fabric are kept at a predetermined interval. Therefore, applying tension in the planar direction makes it possible to cause string vibration of the yarns of the facing ground knitted fabrics forming the three-dimensional knitted fabric or of the connecting yarns connecting the facing ground knitted fabrics. Accordingly, cardiovascular sound/vibration being a biological signal causes the string vibration and is propagated in the planar direction of the three-dimensional knitted fabric.

As the material of the yarns forming the ground knitted fabrics or of the connecting yarns of the three-dimensional knitted fabric, various materials are usable, and examples thereof include synthetic fibers and regenerated fibers such as polypropylene, polyester, polyamide, polyacrylonitrile, and rayon, and natural fibers such as wool, silk, and cotton. These materials each may be used alone or any combination of these may be used. Preferably, the material is a polyester-based fiber represented by polyethylene terephthalate (PET), polybutylene terephthalate (PBT), and the like, a polyamide-based fiber represented by nylon 6, nylon 66, and the like, a polyolefin-based fiber represented by polyethylene, polypropylene, and the like, or a combination of two kinds or more of these fibers. Further, the shapes of the ground yarns and the connecting yarns are not limited either, and round cross-section yarns, modified cross-section yarns, hollow yarns, or the like may be used. Carbon yarns, metallic yarns, and so on are also usable.

The following products are usable as the three-dimensional knitted fabric, for instance.

(a) product number: 49013D (manufactured by Sumie Textile Co., Ltd.), 10 mm thickness material:

front-side ground knitted fabric ... twisted yarn of two polyethylene terephthalate fiber false twisted yarns with 450 decitexes/108 f

rear-side ground knitted fabric ... twisted yarn of two polyethylene terephthalate fiber false twisted yarns with 450 decitexes/108 f
connecting yarn ................ polytrimethylene terephthalate monofilament with 350 decitexes/1 f

(b) product number: AKE70042 (manufactured by Asahi Kasei Corporation), 7 mm thickness
(c) product number: T28019C8G (manufactured by Asahi Kasei Corporation), 7 mm thickness

The three-dimensional knitted fabric 10 is covered with the housing film 20. In this embodiment, the housing film 20 is composed of two films 21, 22 made of a synthetic resin, they are arranged to cover the front surface and the rear surface of the three-dimensional knitted fabric 10, and peripheral edge portions of the films 21, 22 are bonded by welding. Consequently, the three-dimensional knitted fabric 10 is sealingly housed in the housing film 20. In bonding the peripheral edge portions of the films 21, 22, the peripheral edge portions are preferably bonded such that the three-dimensional knitted fabric 10 is slightly pressed in the thickness direction by the films 21, 22. This increases the tension of the three-dimensional knitted fabric 10 to more facilitate the generation of the string vibration of the yarns forming the three-dimensional knitted fabric 10.

The case 40 is attached to the outer side of the housing film 20, and the microphone 30 is disposed in the case 40. The gel 50 as an external disturbance mixture preventing member is filled around the microphone 30 in the case 40. The case 40 is made of a synthetic resin and has a function of preventing acoustic vibration propagated to the microphone 30 from spreading out, and the gel 50 inhibits external vibration from being captured by the microphone 30. A cord 30a that carries detected acoustic vibration data is connected to the microphone 30.

The biological signal detection sensor 1 is set in contact with any of various parts of a person, for example, the back, the chest, or the lumbar when used. Vibration of the body surface is propagated to the housing film 20 and the three-dimensional knitted fabric 10 to be captured by the microphone 30, but the biological signal detection sensor 1 may be attached to the surface of clothes when used, not limited to being directly pasted on the skin surface.

Here, the detection performance of the novel biological signal detection sensor 1 employed in this embodiment will be described as compared with the conventional signal detection sensor disclosed in Patent Document 2 and so on by the present inventors. Note that in the below, the biological signal detection sensor 1 of this embodiment will be called 4SR (Sound Sensing System using Stochastic Resonance), and the conventional biological signal detection sensor 1000 will be called 3SR (Sound Sensing System using Resonance).

(Evaluation of sensing performance of 4SR in frequency band of heart sound)

FIG. 2 illustrates the overview and component configuration of an experimental device used for comparing the sensing performances of 4SR (biological signal detection sensor 1) and 3SR (conventional biological signal detection sensor 1000). The three photographs on each of the left and right of the experimental device in the middle illustrate an accelerometer (a-1) and constituent components (a-2 to a-6) of 3SR and 4SR, and under the experimental device, overview photographs of 3SR (a-7) and 4SR (a-8) are shown. A cross section of the three-dimensional knitted fabric (hereinafter, it may be called a 3D net) with a 10 mm thickness used in 3SR, 4SR is shown in the photograph (a-4). The photographs (Section: a-a, Section: b-b) are cutaway views of the 3D net for 3SR and 4SR.

First, the basic principle of amplitude amplification of 4SR will be described using the photograph (a-4) of the cross section of the 3D net. In the 3D net used in this experiment, the upper and lower surfaces are base fabric layers formed of multifilament yarns, and piles formed of a monofilament yarn in the middle are knitted to the upper and lower base fabric layers to be friction-connected to the monofilament yarns. When the weight of a subject is applied to the upper surface of the 3D net, the piles knitted in an X-shape (hereinafter called X-piles) of the 3D net disposed in the air pack bend, and body movement causes the piles to rub against one another, so that sound and vibration are generated. The sound and vibration cause a stochastic resonance phenomenon. Further, tension is generated when the X-piles bend, so that a natural oscillator is formed. The natural frequency of the X-piles under tension is around 20 Hz. Therefore, owing to the two resonance mechanisms of the stochastic resonance and the string vibration, 4SR which is a sealed air pack is thought to amplify the vibration of 0.5 to 80 Hz.

Next, the performance of amplifying heart sound will be studied. As illustrated in FIG. 3(a), heart sound used for the study was first sound with a first sound splitting interval on PCG being set to 0.03 seconds. This heart sound was measured from a subject whose heart rate was 56/min, and after being subjected to filtering was mechanically reproduced by a speaker. As the speaker, P1000K manufactured by FOSTEX Corporation was used without any enclosure. P1000K is a conical full-range unit with a 10 cm diameter. A 600 g weight was put on a vibration plate of P1000K so that the weight of the vibration plate became 1005 g, and the lowest resonant frequency was set to a low frequency of 82 Hz to 52 Hz. Consequently, 52 Hz to 16 Hz was a reproduction frequency band. The lowest resonant frequency f0 is found by the following.
[Formula 1]

$$f0 = 0.16\sqrt{s0/m0} \quad \cdot\cdot\cdot (1)$$

s0 represents the strength of a spring, and m0 represents the weight of the vibration plate.

The mechanically reproduced sound (hereinafter, called Reproduction PCG) output from the speaker passes through a 3D net placed on the upper surface of the accelerometer, and thereafter is measured by the microphones of 3SR and 4SR. The microphone 1010 of 3SR together with a 3D net 1020 is wrapped with an elastomer film 1030 and beads 1040. Damping is caused when the air in a slot flows to make the air around the microphone flow due to spring force of the internal 3D net. The magnitude of the damping is determined by a slit width in a peripheral portion of a cord 1010a attached to the microphone as illustrated in the photograph (a-7). Since 3SR has the microphone 1010 arranged in the elastomer film 1030, the cord 1010a has to be drawn out of the elastomer film 1030, and a gap is unavoidably formed in this portion. In 4SR, on the other hand, since the three-dimensional knitted fabric (3D net) 10 is structured such that the air pack 1A housed in the housing film 20 is separated from the gel pack 1B where the microphone 30 is disposed, the cord 30a of the microphone 30 is drawn out from the gel pack 1B, which does not obstruct the sealability of the air pack 1A. That is, in 4SR, the whole periphery of the three-dimensional knitted fabric (3D net) 10 is sealed with the elastomer film (housing film 20), and when a load is applied to 4SR, a uniform air pressure is applied to the elastomer film, so that tension is generated in the elastomer film and an environment where acoustic vibration is easily propagated is formed (see FIG. 3 (c)).

FIG. 3(a) illustrates the PCG reproduction input and the acoustic vibration data propagated by the 3D net as described above, and the acoustic vibration data propagated by the 3D net is composed of a waveform due to the resonance effect of the natural oscillator of the 3D net and a subsequent modulated PCG reproduction input. The area of the Lissajous figure in FIG. 3(c) represents energy consumed during one cycle of the vibration, and a damping ratio of 3SR found from damping capacity was 0.53 and that of 4SR was 0.20.

(Evaluation of sensing performance of 4SR in frequency band of cardiac apex beat)

In ACG of a healthy person, cardiac apex beat is composed of short-duration pulsation recognized only in early systole (a systolic wave is composed of a short-duration, sharp positive wave and a subsequent dicrotic limb, and an A-wave originating in left atrial contraction is recognized as a trace) and is described as tapping, and its good recording in ACG is difficult. Therefore, a subject (heart rate: 62/min) who was in a sitting posture so that cardiac apex beat described as tapping could be sensed by palpation was selected, and F-APW (biological information measured from the anterior chest surface: Front Acoustic Pulse Wave) was measured using 3SR and 4SR, was recorded in a data logger, and filtered, and the following analysis was conducted.

The input waveform recorded in the data logger is frequency-analyzed, a ratio of the power spectra of 4SR and 3SR ($PSD_{4SR}/PSD_{3SR}$) at each frequency is defined as a gain, and sensing performance is evaluated based on the gain. On the anterior chest surface at a position corresponding to the left fifth intercostal space, 4SR is placed 6 cm left of the chest midline, and 3SR is placed 10 cm left thereof (corresponding to the vicinity of V4 induction of an electrocardiogram). Since the installation positions of the microphones for PCG and the 4SR sensing systems are places where vesicular breathing sound is likely to mix, F-APW is measured for ten seconds while the breath is held. Note that the procedure of the measurement experiment was to continue breathing at rest for five minutes, and hold the breath after two-second inspiration. As a subject, a healthy male in his forties without any underlying disease in the cardiocirculatory system was selected.

(APW Measurement)

Next, APW is measured. All the measurements were conducted in a sitting posture. 4SR for capturing F-APW considered as including cardiac apex beat is placed on the anterior chest surface at a position corresponding to the fifth left intercostal space on the left midclavicular line (near V4) and 10 cm left of the chest midline, and 4SR for R-APW (biological information measured from the posterior chest: Rear Acoustic Pulse Wave) is placed on the posterior chest surface at a position that is the same height as that of 4SR for F-APW which is on the anterior chest surface, and is 6 cm left of the dorsal midline. 4SR for capturing L-APW of the lumbar (biological signal measured from the lumbar: Lumbar Acoustic Pulse Wave) is placed on the third to fourth lumbar vertebrae median which is directly behind the umbilical region. The microphone for PCG is placed at a position corresponding to the cardiac apex. Further, in ECG (Electrocardiogram), II induction is obtained.

The position of the cardiac apex microphone sensor on the anterior chest is an optimal position for recording first sound, third sound, and fourth sound, but at this position, it is difficult to record second sound well. It is said that the second sound is generated by the hyperextension of the semilunar valve caused by arterial blood trying to flow back to the ventricle in late systole and subsequent reaction of the arterial wall (cardiohemic system), and the second sound becomes weak at the cardiac apex.

Since the installation positions of the microphones for PCG and the 4SR sensing system are places where vesicular breathing sound is likely to mix, the result of the ten-second measurement during which the breath is held is used for the calculation. Note that the APW measurement experiments at rest are conducted under the following four conditions: a) a state where natural breathing is continued for five minutes; b) a state where the breath is held for thirty seconds after two-second inspiration; c) a state of thirty-second natural breathing; and d) a state where the breath is held for thirty seconds after two-second inspiration.

Subjects were 70 healthy males and females in a group with a relatively thin chest wall and a group with a relatively thick chest wall and thus have different features.

As for fifty healthy people having a relatively thin chest wall, their ages were distributed in twenties to sixties, and a ratio of the males was 78%. The thin chest wall facilitates the observation of a sine wave or a triangular wave which is the feature of cardiac apex beat. Using these subjects, later-described five waveform types were defined, and BF (Boundary Frequency) between vibration ascribable to cardiac apex beat and vibration ascribable to heart sound was decided.

Using twenty subjects in their twenties who had a relatively thick chest wall and were muscular, the compatibility of the analysis method was evaluated and its extensibility was studied, based on the previously decided five waveform types.

Clinical profiles of the fifty healthy subjects with a relatively thin chest wall are as follows:
average age 38.2 (±10.3) years old, 39 males, 11 females, average BMI value 23 (±3.4), out of them, four have hypertension and ten are smokers.

The clinical profiles of the twenty subjects with a relatively thick chest wall are as follows:
average age 23.4 (±3.6) years old, 20 males, average BMI value 20.8 (±2.4), out of them, one has hypertension and five are smokers.

As data to be analyzed, used was data during ten seconds from a part where RR variation was within 15%, in the first thirty-second period during which the breath was held, and as heart rate, an average value of data in this ten-second period was used.

(Performance evaluation result and APW measurement result of 4SR sensing system)

In FIG. 3(a), the waveform illustrated with the black thin line is Reproduction PCG, and the waveform illustrated with the gray thick line is Acceleration PCG measured by the accelerometer. Reproduction PCG is composed of first sound (an A-wave and a B-wave), a waveform (C-wave) of around 30 Hz, and second sound (a D-wave, an E-wave, and an F-wave), and Acceleration PCG is composed of resonance waveforms (an a-wave and a b-wave, and a d-wave and an e-wave) of Reproduction PCG and subsequent waveforms (a c-wave and an f-wave) with changed amplitudes and frequencies. As shown in the frequency analysis result in FIG. 3(b), the A-wave and the B-wave, and the D wave and the E-wave which are present in a frequency band of 60 Hz or higher became larger in amplitude to be an a-wave and a b-wave, and a d-wave and an e-wave, and the C-wave and the F-wave of around 30 Hz became a c-wave and an f-wave resulting from amplitude and frequency modulation by heterodyne. As a result, at around 30 Hz, the power spectrum of Acceleration PCG was smaller than that of Reproduction PCG.

Next, the damping performance of a mechanical vibration system constituting the sensing system will be studied. FIG. 3(c) illustrates Lissajous figures indicating damping characteristics that the sensors 3SR and 4SR have. The Lissajous figures were drawn using a servo pulser whose pressure plate had a 110 × 110 mm size and whose input had a ±1.0 mm amplitude and a 1.34 Hz excitation frequency. A pre-compressive force is 652 N. The area surrounded by the Lissajous figure is energy consumed in one cycle of vibration and is a damping capacity. Let this be W, then W is represented by the following.
[Formula 2]

$$W = \pi c \omega a^2 \quad \cdot\cdot\cdot (2)$$

[C is damping coefficient, ω is angular frequency, and α is amplitude]
Comparison between the dynamic characteristics of 4SR and 3SR shows that 3SR has a substantially equal spring characteristic to that of 4SR, and in addition, is noticeably influenced by damping due to the air that flows in/out.

Further, amplitude magnification Z/Y used in the evaluation of vibration displacement is given by the following, where Z is relative displacement and Y is displacement of cardiac apex beat.

[Formula 3]

$$\frac{Z}{Y}=\frac{\left(\frac{\omega}{\omega_n}\right)^2}{\sqrt{\left[1-\left(\frac{\omega}{\omega_n}\right)^2\right]^2+\left(2\zeta\frac{\omega}{\omega_n}\right)^2}} \quad \cdot\cdot\cdot(3)$$

Here, the natural angular frequencies of the mechanical vibration systems of the sensors 4SR and 3 SR are represented by the following,

$$\omega_n=\sqrt{\frac{k}{m}}$$

and, a damping ratio is represented by the following.

$$\zeta=\frac{c}{2\sqrt{mk}}$$

FIG. 3(d) illustrates amplitude magnification curves of 4SR and 3SR calculated using Formula (3). In this chart, spring constants of the sensors 4SR are compared.

The following values were substituted.

$$k_{4SR}=485\mathrm{N/m}、m=24\mathrm{kg}、\zeta_{4SR}=0.20、k_{3SR}=9367\mathrm{N/m}、m=24\mathrm{kg}、\zeta_{3SR}=0.53$$

FIG. 3(e) illustrates a load-deflection characteristic that is obtained through the measurement with a digital force gauge (RZ-20) when the sensor (4SR) illustrated in FIG. 2 (a-8) is pressed by a φ15 mm pressure plate. The spring constant was calculated assuming the measurement of cardiac apex beat. In the case of the measurement from the anterior chest, since a pressing force of the sensor was set small, a spring constant in the case where a deflection amount was 1 mm was used. In the measurement from the posterior chest, since the weight is applied to the sensor, a spring constant in the case where the deflection amount was 4 to 5 mm which is a design value of 3 SR was used.

It is seen that, since the natural frequency of the sensing system of 4SR is set low, 4SR is capable of the measurement of a frequency band of 0.45 Hz or higher in the case of the measurement in the range of $Z/Y \approx 1$, and 3 SR is capable of the measurement of a frequency band of 2.10 Hz or higher. Note that, in the case of the damping ratio $\zeta = 0.7$, 4SR is capable of the measurement of 0.99 Hz or higher, and 3SR is capable of the measurement of 3.15 Hz or higher. By thus changing the damping ratio, it is possible to adjust the characteristic of a mechanical filter.

Cardiac auscultation is to capture the characteristic of heart sound in a high frequency range of around 50 to 100 Hz and interpret and confirm it, and it is important to record acoustic vibration of high frequencies. On the other hand, cardiac apex beat is short-duration pulsation that is within a frequency range lower than that of heart sound and is recognized only in early systole, and it is difficult to record a good apex cardiogram if emphasis is put on high-frequency measurement. Since the required specifications of the sensing system thus differ depending on which of heart sound and cardiac apex beat is to be measured, quantitative evaluations of the sensing systems were conducted separately for these. FIG. 4(a) illustrates comparison of acoustic vibration information captured by the sensing system of 3SR and that captured by the sensing system of 4SR (hereinafter, called 3SR data and 4SR data) when Acceleration PCG is input. FIG. 4(b) illustrates the frequency analysis results. The 3SR data and the 4SR data are acoustic vibration information influenced by the stochastic resonance and the resonance by the natural oscillator, and when they are compared, an increase/decrease in the power spectrum is seen in the same frequency band. FIG. 4(c) illustrates a gain of 4SR/3SR

($PSD_{4SR}/PSD_{3SR}$). Differences in the effect of the stochastic resonance around 10 Hz, the resonance effect of the natural oscillator around 20 Hz, and the mechanical vibration characteristics that the sensors have around 20 to 80 Hz appear as a gain change (the maximum 11.6 dB and the minimum 8.0 dB), and the maximum amplitude difference between the waveforms illustrated in FIG. 4(a) became 4.3 times. Note that a sudden drop of the gain at 80 Hz or higher is considered to be due to the damping effect of the gel attached to the 4SR sensing system.

FIG. 5(a) illustrates time-series waveforms of 3SR data and 4SR data when a person instead of the speaker is a sound source, and FIG. 5(b) illustrates the frequency analysis results. FIG. 5(c) illustrates a gain of 4SR/3 SR ($PSD_{4SR}/PSD_{3SR}$). The damping in the 3SR sensing system works at 1.34 Hz or higher, and even though the gain near 1 Hz is 13.4 dB, the gain near 1.34 to 10 Hz decreases to 4.9 dB. The waveform prominently shows the influence of the damping, and as illustrated in FIG. 5(a), the maximum amplitude of the systolic wave appeared as 6.8 times.

Incidentally, the air damping of 3SR had a small influence on random vibration related to heart sound, but as illustrated in FIG. 5(b), it had an influence on the power spectrum of the cardiac apex beat wave of 13 Hz or lower, and its value became small. Further, the damping effect illustrated in FIG. 3(c) is prominently recognized mainly in a fundamental harmonic and a second harmonic. This agrees with the simulation results in FIG. 3(d), from which it is seen that with 3SR, it is not possible to depict the cardiac apex beat wave.

A vibration transmitting mechanism will be discussed. Since tension is generated also in the elastomer film (the housing film denoted by reference sign 20 in 4SR and the elastomer film denoted by reference sign 1030 in 3SR) itself, low-frequency vibration of around 1 Hz is transmitted to the internal 3D net in 4SR through tension variation. As a result, vibration of 13 Hz or higher was measured by 3SR and 4SR as having substantially equal power spectra between 13 to 30 Hz illustrated in FIG. 5(b), due to the effect of the natural oscillator occurring around 20 Hz. This vibration component is thought to be transmitted to the piles of the 3D net. It is also seen from FIG. 5(b) that a frequency band in which the minimum frequency of heart sound and the higher harmonic components of the cardiac apex beat wave mix is present near 10 Hz. Therefore, it has been found out that 4SR is higher in sensitivity than 3SR in the entire band of frequencies to be studied.

Conventionally, the frequency to be analyzed by 3SR was set to 10 to 30 Hz, but as a result of the above experiment result, the vibration frequency of F-APW to be measured by 4SR was set to 0.5 to 80Hz in consideration of a performance ensured range of the microphone, which is 0.1 Hz or higher, and the frequency to be analyzed of heart sound was set to 25 to 45 Hz or 40 to 80 Hz instead of 100 Hz or higher because most of the heart sound gather in a low audible spectrum. Further, the sampling frequency was set to 1000 Hz in 4SR though set to 200 Hz in 3 SR.

Here, a comparative experiment was conducted in which acoustic vibration was measured using the biological signal detection sensor 1 (4SR) of this embodiment and a structure having only the air pack 1A with the gel pack 1B removed from 4SR (4SR without gel pack). FIG. 6 is a view illustrating how the experiment is conducted, and in the experiment, on a urethane base, the biological signal detection sensor 1 (4SR) was arranged on the right and the structure only with the air pack 1A (4SR without gel pack) was arranged on the left. The other experimental conditions are the same as those of the above-described comparative experiment of 3SR and 4SR. FIGs. 7 illustrate the results.

FIGs. 7(a) to (c) illustrate the experiment results of 4SR. FIGs. 7(d) to (f) illustrate the experiment results of the structure only with the air pack 1A (4SR without gel pack). (a) and (d) illustrate time-series waveforms of acoustic vibration information captured when the sound source is a person, (b) and (e) illustrate time-series waveforms with the middles of the amplitudes of the waveforms in (a) and (d) being set as a zero point, and (c) and (f) illustrate time-series waveforms of acoustic vibration information captured when Acceleration PCG is an input. FIG. 7(g) is an enlarged chart of (d), and FIG. 7(h) is an enlarged chart of (e).

As is apparent from these charts, 4SR has higher detection sensitivity to acoustic vibration information than the structure only with the air pack 1A (4SR without gel pack). In particular, when the sound source was a person, 4SR was capable of clearly capturing the acoustic vibration information because an amplitude change of about $\pm 0.2$ [V] occurred, while it was difficult for the structure only with the air pack 1A to capture an amplitude change. Further, as illustrated in FIG. 7(j), in the load-deflection characteristic comparison, a hysteresis loss was smaller in 4SR than in the structure only with the air pack 1A.

Further, the microphone 30 is arranged on a disk-shaped support member (member denoted by reference sign 30b in FIG. 6) made of a thin plate on the bottom surface of the case 40 made of a synthetic resin, and the case 40 has a thickness of about 0.1 to 0.2 mm and is lower in rigidity than the disk-shaped support member 30b. Because of this, the case 40 easily vibrates by reacting to the acoustic vibration and causes the resonance of the acoustic vibration to play a role of something like a vibration plate and an enclosure of a speaker, and also works to enhance the detection sensitivity of the microphone 30 and increase the amplitude. FIG. 7(k) illustrates amplitude ratios of these (4SR/4SR without gel pack). It is seen from this chart that 4SR is higher not only in detection sensitivity to acoustic vibration with a very low frequency such as cardiac apex beat but also in detection sensitivity to vibration in a heart sound spectrum (25 to 80 Hz).

Further, a time waveform of MAV (micro acoustic vibration) output from the trunk and measured by a microphone attached directly to the chest and a time waveform of F-APW (acoustic vibration information influenced by the response of the mechanical vibration system of the aforesaid members composing 4SR and further influenced by the resonance of the natural oscillator and the stochastic resonance) measured from 4SR were frequency-analyzed, and a gain of F-APW/MAV was found through the calculation of their amplitude magnifications. The gain of F-APW/MAV was 42 dB in the frequency band of the fundamental harmonic of cardiac apex beat, was slightly more than 30 dB in a higher harmonic frequency band, and was 20 dB in the lowest frequency band of heart sound of around 20 to 30 Hz, which is a frequency band of the natural oscillator. The effects of the response of the mechanical vibration system and the stochastic resonance appeared as over 40 dB at the maximum and as 10 dB at the minimum. An amplitude difference was slightly less than 200 times. In F-APW, a waveform in a 1 to 1.5 Hz band was observed, and information on heart sound and cardiac apex beat was contained. A time lag of MAV and F-APW was 0.12 seconds.

FIGs. 8 to FIGs. 11 illustrate the three-second measurement results of the time-series waveforms of F-APW, R-APW, L-APW, and PCG of four subjects whose average heart rates are 58, 71, 79, and 93/min. These three types of APW contain information on heart sound and cardiac apex beat. In F-APW, waveforms in a 1 to 1.5 Hz band were observed, and in R-APW and L-APW, waveforms in a 5 to 7 Hz band were observed.

- Biological signal analysis device

Next, a biological signal analysis device 100 having a computer function, in which a computer program for processing data obtained from the biological signal detection sensor 1 of this embodiment, is set will be described based on FIG. 12.

The biological signal analysis device 100 processes time-series data of biological signals obtained by the biological signal detection sensor 1 to obtain a waveform of cardiac apex beat and further infers biological conditions, that is, various health conditions (the presence/absence of a disease such as a heart disease, the identification of a disease, whether a physical condition is good or not, and so on) of a person. The biological signal analysis device 100 is constituted by a computer (including a personal computer, a microcomputer incorporated in a device, and so on) and receives the time-series data of the biological signals transmitted from the microphone 30 of the biological signal detection sensor 1. It includes: a frequency analyzing means 110 that performs predetermined processing using the received time-series data; and a boundary frequency identifying means 120.

As a storage unit, it has a correlation data storage unit 150 in which correlation data of a boundary frequency between vibration ascribable to cardiac apex beat and vibration ascribable to heart sound with heart rate is stored, the correlation data being created through advance measurement.

In more detail, in the biological signal analysis device 100, a computer program causing the execution of procedures functioning as the frequency analyzing means 110 and the boundary frequency identifying means 120 is stored in the storage unit (including not only a recording medium such as an internal hard disk as the computer (biological signal analysis device 100) but also various types of removable recording media and a recording medium of another computer connected through communication means). Note that the biological signal analysis device 100 can also be actualized using an electronic circuit having one recording circuit or more in which the computer program implementing the frequency analyzing means 110 and the boundary frequency identifying means 120 is incorporated.

The computer program can be stored in a recording medium to be provided. The recording medium storing the computer program may be a non-transitory recording medium. The non-transitory recording medium is not limited, and examples thereof include recording media such as a flexible disk, a hard disk, CD-ROM, MO (magneto-optical disk), DVD-ROM, and a memory card. It is also possible to transfer the computer program to the computer through a communication line to install it therein.

Here, since the biological signal detection sensor 1 is capable of capturing heart sound as described above, the frequency analyzing means 110, upon receiving the obtained biological signal data, frequency-analyzes biological signal data obtained through the body surface by the biological signal detection sensor 1.

The boundary frequency identifying means 120 finds BF (Boundary Frequency) between the vibration ascribable to cardiac apex beat and the vibration ascribable to heart sound in the biological signal data, from the frequency analysis result of the biological signal data obtained from the frequency analyzing means 110. The boundary frequency identifying means 120 includes a means that finds a power spectrum sudden changing point which is the boundary between harmonic vibration and random vibration, in the frequency analysis result obtained by the frequency analyzing means 110 and identifies the boundary frequency based on the sudden changing point.

According to this embodiment, finding the boundary frequency makes it possible to distinguish the waveform of cardiac apex beat from the waveform of heart sound to obtain the waveform of cardiac apex beat from the biological signal.

Further, the biological signal analysis device 100 of this embodiment uses the boundary frequency itself as a determination criterion. Specifically, as is apparent from a later-described experiment, the boundary frequency is represented by a quadratic function with heart rate, and from correlation data between these, the boundary frequency can be found based on the heart rate. From the correlation between the boundary frequency and the heart rate, it is seen that the heart rate of the heart in a highly efficient condition is about 70 to 80/min. This is a state where an exercise load of the heart is small, and the boundary frequency in this case is smallest. Therefore, from a measurement-time boundary frequency obtained by measuring the heart rate and using the correlation data, it is possible to infer the state of the heart at this instant.

Preferably, the biological signal analysis device 100 of this embodiment has: the correlation data storage unit 150, indicated by the imaginary line in FIG. 12, in which the correlation data between the boundary frequency and heart rate is stored; and a measurement-time condition inferring means 130 that finds the boundary frequency based on the heart rate of a measurement subject by referring to the correlation data stored in the correlation data storage unit 150 and infers a measurement-time boundary frequency of the measurement subject.

The measurement-time condition inferring means 130 requires information on the heart rate when inferring the condition. As for the heart rate information, a heart rate calculating means 140 indicated by the imaginary line in FIG. 12 is capable of finding the heart rate. In this case, the combination of the biological signal detection sensor 1 and the heart rate calculating means 140 that processes the biological signal data thereof to find the heart rate functions as a heart rate measuring unit. Heart rate measuring methods include electrocardiography, phonocardiography, photoplethysmography, and sphygmomanometry, and a measurement device of any of these types is also usable as the heart rate measuring unit. However, the biological signal detection sensor 1 of this embodiment is excellent in easy measurement because it is capable of the measurement in a non-constraining manner only by being attached to the body surface of a person.

The correlation data stored in the correlation data storage unit 150 is correlation data of the boundary frequency between vibration ascribable to cardiac apex beat and the vibration ascribable to heart sound with the heart rate, which are measured in advance. This correlation data is configured by a quadratic function indicating the correlation between the boundary frequency and the heart rate obtained when the boundary frequency is found. Therefore, collating the measured heart rate with the correlation data stored in the storage unit of the correlation data storage unit 150 makes it possible to find the boundary frequency.

The boundary frequencies stored in the correlation data storage unit 150 are found through an analysis of a lot of biological signal data in advance. The boundary frequency is found as the aforesaid sudden changing point identified by the boundary frequency identifying means 120.

The sudden changing point can be found as follows. First, the biological signal data obtained through the body surface by the biological signal detection sensor 1 is frequency-analyzed, and in consideration of this frequency analysis result and the frequency analysis result of heart sound data measured simultaneously, the boundary frequency between the vibration ascribable to cardiac apex beat and the vibration ascribable to heart sound, in the biological signal data, is extracted.

Specifically, the frequency analysis results of the biological signal data found by the frequency analyzing means 110 are subjected to addition averaging, the obtained waveform is represented in log-log axes using frequency and power spectrum, and the frequency analysis results of the heart sound data are subjected to addition averaging, the obtained waveform is represented in log-log axes using frequency and power spectrum, a fluctuation changing point is found from a waveform of a log difference of the two waveforms represented in log-log axes, this fluctuation changing point is considered as a practical disappearing point where higher harmonic components of the cardiac apex beat become very small, and a frequency at the disappearing point is considered as corresponding to the aforesaid sudden changing point, whereby the boundary frequency can be found.

As the frequency analyzing means 110, a STFT (Short Time Fourier Transform) method can also be employed. In this case, the boundary frequency identifying means 120 finds the aforesaid power spectrum sudden changing point from the analysis result of the short time Fourier transform. Preferably, the analysis result of the short time Fourier transform is output as image data showing time, frequency, and a power spectrum variation degree. This enables the boundary frequency identifying means 120 to find the power spectrum sudden changing point from the image data of the short time Fourier transform. A specific means for identifying the sudden changing point, that is, a means for finding the boundary frequency will be described later.

Here, the biological signal detection sensor 1 is attached to the back, the chest, the lumbar, or the like of the body of a person to capture sound and vibration in the body. The obtained biological signal is a collection of various biological sounds and in-vivo vibrations. On the other hand, cardiac apex beat is vibration hidden behind a heart sound waveform, and it is difficult to separate them. In this embodiment, however, the aforesaid BF (Boundary Frequency) for distinguishing the cardiac apex beat waveform and the heart sound waveform is found, which enables the inference of the biological condition relating to the cardiac apex beat based on the heart rate.

Hereinafter, methods of the frequency analysis by the frequency analyzing means 110 and the identification of the boundary frequency by the boundary frequency identifying means 120 will be described in detail. As described above, this embodiment uses two methods: the method of using the waveform represented in log-log axes to find, in the waveform, the fluctuation changing point based on the frequency at which the harmonic vibration of higher harmonics practically disappears, and considering this fluctuation changing point as the power spectrum sudden changing point;

and the method of finding the power spectrum changing point from the analysis result of the short time Fourier transform.

[Method of extracting boundary frequency (BF)]

(BF extraction method based on disappearing frequency of harmonic vibration of higher harmonics of APW)

Heart sound is random vibration as described in Non-patent Document 1, while the later-described experiment result has made it obvious that a cardiac apex beat wave is mainly composed of harmonic vibration composed of a fundamental harmonic and higher harmonics. Therefore, we have focused on frequency-analyzing the waveform of APW obtained from the 4SR sensing system attached to the anterior chest, performing the addition averaging, and finding the power spectrum changing points of the harmonic vibration of the higher harmonics and the random vibration, that is, the power spectrum sudden changing point. The higher harmonic components of CAB (Cardiac Apex Beat) of the cardiac apex beat wave become smaller in power spectrum as the frequency becomes higher. On the other hand, the power spectrum of the random vibration of CAS (Cardiac Acoustic Sound) of heart sound changes in magnitude without depending on frequency. Here, a frequency at which the power spectrum of the higher harmonic components of CAB becomes small and a variation behavior of CAS changes is called boundary frequency, which will be hereinafter called BF. Note that the occurrence of heart rate variability or blood pressure variability leads to instantaneous variation of the waveform of APW, and one of the possible reasons of the instantaneous variation of the APW waveform is variation of the dominant frequency of PCG.

Because of individual differences of subjects, impedance regarding the transmission characteristic of acoustic vibration of the trunk also differs, which may make it difficult to find BF. Therefore, the frequency at which the higher harmonic components of the cardiac apex beat wave disappear (the frequency at which the power spectrum of the higher harmonic components becomes very small and is practically on a negligible level) is found using the log difference method. In the addition averaging of APW, Fourier transform is performed with a time window of 8.2 seconds and 90% overlapping. The waveform resulting from the addition averaging is represented in log-log axes. This is because the addition theorem is usable for the power spectrum waveform represented in log-log axes. Note that the PCG waveform is used for heart sound. When the log difference method is used, an absolute value of the PCG power spectrum in a frequency band of 0.5 to 20 Hz becomes large and the power spectrum waveform can be reversed in phase. Consequently, the power spectra of the higher harmonic components of the cardiac apex beat and the random vibration of the heart sound are decreased to facilitate finding BF.

(BF extraction method using short time Fourier transform)

The power spectrum STFT (Short Time Fourier Transform) is applied to F-APW, PCG, APW×PCG, and APW×$PCG^{-1}$ to visualize a fluctuation changing point. A formula of the short time Fourier transform used in this analysis is as follows.
[Formula 4]

$$\mathrm{STFT}(\tau, \omega) = \int_{-\infty}^{\infty} x(t) W(t - \tau) e^{-j\omega t} dt \quad \cdots (4)$$

x(t) is measured signal data, and W(t - τ) is a window function. To obtain the frequency resolution of 0.5 Hz or lower, the number of points of the window function is set to $2^{12}$ = 4096. Under the sampling frequency of 1000 Hz, the 4096 points correspond to 4.096 seconds, and 4.096 seconds correspond to 0.244 Hz. As a result, the frequency resolution can be set equal to or less than 0.5 Hz, specifically, set to 0.244 Hz. Then, a shift width of the window function was set to 50 points. t represents measurement time, and τ represents time-series data based on the moving time of the window function. Multiplying the measured signal data by the window function makes it possible to create frequency information that changes with time. Note that the frequency information is generated every 50/1000 = 0.05 seconds.

Next, using BF identified based on the aforesaid two BF extraction methods, a cardiac apex beat waveform (Front CAB) and heart sound (Front CAS) are drawn from F-APW, and similarly Rear CAB, Rear CAS, Lumbar CAB, and Lumbar CAS are drawn from R-APW and L-APW. Further, since it has been found out in a preliminary experiment and a preliminary study that HR correlates with BF, a correlation chart of BF and HR is created using data of fifty subjects for the purpose of studying the correlation between BF and HR.

The BF extraction method based on the frequency at which the higher harmonic components disappear is an approach to decide BF from the frequency, while the BF extraction method using the short time Fourier transform is an approach to decide BF based on the power spectrum threshold. Specifically, the former is a method to decide, as BF, the frequency at which a decreasing change of the power spectrum height shifts to an increasing change as the frequency is changed, and the latter is a method to set 70% of an average value of a power spectrum group present around BF as the threshold to decide BF.

Here, FIGs. 19 to FIGs. 21 illustrate summaries of the comparison of ECG, F-APW, PCG, Front CAB, and Front CAS regarding four subjects whose average HR (heart rates) are 58/min, 71/min, 79/min, and 93/min.

In the case of the subject with HR: 58/min, a systolic wave measured from Front CAB presents a late systolic bulge of ACG, but from ECG and PCG (Front CAS), it is less likely that the subject has dilated cardiomyopathy. It is thought that the cardiac apex beat is influenced by gravitational force to resemble a sustained pattern.

In the case of the subject with HR: 71/min, in ECG, a P-wave is not clear and is too small to be seen. In Front CAB, an A-wave originating in left atrial contraction (atrial bulging wave) as is confirmed in ACG is confirmed, and in Front CAS as well, a low-frequency wave is present earlier than first sound of PCG, which does not correspond to ectopic rhythm.

In the case of the subject with HR: 79 min, a waveform measured from Front CAB presents a sustained pattern, but an A-wave, a RFW (Rapid Filling Wave) waveform, and Front CAS indicate that this does not correspond to a case of left ventricular hypertrophy and leakage from the left ventricle in systole (mitral valve regurgitation or the like).

In the case of the subject with HR: 93/min, ECG presents a low voltage and presents a high heart rate in a resting state, but Front CAB having low-frequency components and high-frequency components and Front CAS in which first sound does not rise nor weaken rule out the possibility of an underlying heart disease.

(Correlation of heart rate variability fluctuation characteristic with heart rate and BF)

A power spectrum slope on a log-log plot is called a fluctuation coefficient here, and a steep changing point of the fluctuation coefficient will be hereinafter called a fluctuation changing point. At the fluctuation changing point, complexity is lost and a breakpoint occurs. The fluctuation coefficient is represented by power spectrum P(f) and frequency (f), which are log P(f) and log(f) on the log-log plot, and in the case where log P(f) and log(f) have a linear relationship, the following formula holds.

[Formula 5]

$$\log P(f)=\kappa-n\log(f)=\kappa+\log(1/f^{n}) \quad \cdot\cdot\cdot (5)$$

[κ is constant and n is fluctuation coefficient]

To evaluate the fluctuation characteristic of heart rate variability found from ECG, experimental data measured from an experimental protocol of APW measurement is used. The fluctuation characteristic is calculated using Formula (5). An obtained value of n is a minus numerical value (about -0.5 to -1.5) but is expressed by an absolute value because Non-patent Document 3 defines it as 1/f fluctuation in a cardiac cycle.

To evaluate the fluctuation of the heart rate variability, data during no breathing, natural breathing, and no breathing each having thirty-second duration, that is, data during totally ninety seconds are used. As the heart rate here, an average value during the ninety seconds is used.

[Results of BF extraction by the extraction methods]

(Result of BF extraction based on disappearing frequency of harmonic vibration of higher harmonics of F-APW)

FIG. 13 is a flowchart illustrating a procedure for extracting BF using F-APW×$PCG^{-1}$, and the following (1) to (10) correspond to the encircled numbers in FIG. 13. Hereinafter, the procedure and focused points of calculation will be described.

(1) F-APW is found from 4SR data without filtering processing and is frequency-analyzed. Hereafter, the result is represented by F-APW.
(2) F-APW is subjected to addition averaging (8.2-second time window with 90% overlapping).
(3) A waveform is extracted from PCG and is frequency-analyzed. Hereinafter, the result will be represented by PCG.
(4) The same addition averaging (8.2-second time window with 90% overlapping) as that in (2) is performed.
(5) F-APW×$PCG^{-1}$ is created using the power spectra F-APW and PCG of F-APW and PCG found in (2) and (4), and a breakpoint is found.
(6) From the breakpoint on a waveform of F-APW×$PCG^{-1}$, the disappearing frequency of higher harmonics of a cardiac apex beat wave having fluctuation and an appearing frequency of a random vibration component of heart sound which becomes white noise are identified.

(7) A BF line is drawn from the disappearing point of the higher harmonics on F-APW×$PCG^{-1}$, and an intersection of the BF line and F-APW is identified as BF.

(8) 0.5 Hz to BF is a CAB band (hereinafter, CAB (0.5-BF)), and BF to 50 Hz is a CAS band (hereinafter, CAS (BF-50)).

(9) A 0.5 Hz to BF bandpass filter is applied to F-APW and it is confirmed that the higher harmonic components of CAB decrease near BF.

(10) A BF to 50 Hz bandpass filter is applied to F-APW and it is confirmed that the power spectrum changes near BF without depending on frequency.

FIGs. 14 illustrate the frequency analysis results of CAB and CAS, sorted by heart rate (HR) (58 to 93/min). From these charts, the disappearance of the higher harmonic components of CAB at BF, the appearance of random vibration of CAS from the vicinity of BF, and HR dependence of BF are understood, which shows that the method of the present invention is capable of separating CAB and CAS to visualize their characteristics.

Next, FIG. 15 to FIG. 18 illustrate the CAB and CAS waveforms drawn by the BF extraction method based on the disappearing frequency of the harmonic vibration of the higher harmonics in APW. These charts illustrate processed waveforms of Front CAB (0.5-BF), Front CAS (5-BF), Rear CAB (0.5-BF), Lumbar CAB (0.5-BF), PCG, Front CAS (BF-50), Rear CAS (BF-50), and Lumbar CAS (BF-50) which are extracted from F-APW, R-APW, and L-APW obtained from the subjects whose average heart rates are 58, 71, 79, and 93/min. The drawing time is 1.5 seconds. The frequency bands of used filters are written in the parentheses. Ranges of the vertical axes and the horizontal axes of CAB and CAS waveforms are equal among measurement positions. It is seen that in Front CAB (0.5-BF) of the four subjects, a cardiac apex beat wave mainly composed of a fundamental harmonic is drawn.

The study of the obtained results shows that Front CAB (5-BF) is formed of the combination of a plurality of 5 to 10 Hz waveforms. On the other hand, Rear CAB (0.5-BF) and Lumbar CAB (0.5-BF) had harmonic vibration waveforms mainly composed of a waveform with a higher frequency than that of the waveform illustrated in Front CAB (0.5-BF), and had waveforms different from that of Front CAB (0.5-BF). Further, heart sound was random vibration formed of a high-frequency waveform in which multiple vibration components and multiple vibration waveforms are combined.

(Result of BF extraction using short time Fourier transform)

FIGs. 22 to FIGs. 27 illustrate the STFT analysis results of the power spectra obtained through the frequency analyses of F-APW and PCG of the subjects whose average heart rates in the ten-second measurement time are 93/min, 58/min, 71/min, and 79/min.

In the STFT charts, the vertical axis represents frequency and the horizontal axis represents the time when STFT is output. The color intensity indicates a degree of power spectrum variation, and by changing a range of the color intensity, it is also possible to express the degree of the fluctuation coefficient represented by Formula (5). The degree of the power spectrum variation is indicated by the intensities of Red, Green, and Blue (RGB). In a place where the power spectrum variation is small and a part where the fluctuation coefficient does not suddenly change, and in random vibration with a small amplitude, planar Red, Green, and Blue appear. Conversely, in a place where the power spectrum variation is large and a part where the fluctuation coefficient suddenly changes, Red, Green, and Blue appear in a line shape.

The aforesaid log difference (F-APW×$PCG^{-1}$) is illustrated in FIGs. 23(a), (b). FIG. 24(b) illustrates the frequency analysis results, where BF corresponding to a breakpoint is 15 Hz.

In FIG. 24(a), the log sum of APW and PCG (APW×PCG) was calculated to emphasize the higher harmonic components and the random vibration present in the frequency band of 0.5 to 30 Hz. In this STFT, the cardiac apex beat wave and heart sound which is random vibration are both emphasized, with BF being the boundary therebetween. Accordingly, BF appeared in a 14 to 17 Hz band where Blue and Yellow mainly appear.

From the two linearly drawn lines of APW×$PCG^{-1}$ and APW×PCG, variation behaviors of the higher harmonic components of the cardiac apex beat and the random vibration of the heart sound were detected, and it was possible to identify the disappearing frequency of the higher harmonic components of the cardiac apex beat, the heart sound appearing frequency, and BF.

Next, the focused points for the studies of the charts in FIGs. 22 to FIGs. 27 will be described.

FIGs. 22 to FIGs. 24 illustrate the STFT analysis results of the subject whose average heart rate in the ten-second measurement time is 93/min.

FIG. 22(a) shows that APW has higher harmonic components up to the ninth order with 1. 6Hz being a fundamental harmonic. BF in this chart is present in 14 to 17 Hz illustrated in Blue because the breakpoint is set as the power spectrum sudden changing point.

In FIG. 22(b), in PCG, vibration components having a large power spectrum change are between 10 to 50 Hz, and when this chart is seen together with FIG. 22(a), it is seen that the fifth harmonic up to random vibration are captured.

FIG. 23(a) is APW×PCG$^{-1}$, in which BF is present in a 14 to 17 Hz band drawn in Red, Blue, and Red because the sudden changing point in a band where the power spectrum variation is small is a breakpoint. FIG. 23(b) is an enlarged chart of APW×PCG$^{-1}$, in which a linear Red line indicating the breakpoint appeared around 15 Hz.

In APW×PCG in FIG. 24(a), a fundamental harmonic of 1.49 Hz is Blue Line, the second harmonic is Green Line, and the third harmonic to higher harmonics up to 15 Hz are Red Lines, which shows that the higher harmonics are larger in power spectrum than the fundamental harmonic. It further shows that a breakpoint is present in the Blue range indicated by Red-Blue-Red around 15 Hz, and the random vibration is present in 15 to 20 Hz where Red starts.

FIG. 24(b) illustrates the power spectra of APW×PCG$^{-1}$, APW, PCG, and APW×PCG. A breakpoint was in APW×PCG$^{-1}$, and the breakpoint was 15 Hz, and BF was present at 15 Hz.

BFs in FIGs. 25 to FIGs. 28 are found by the above-described BF extraction method using APW×PCG$^{-1}$ and STFT. The heart rates of the subjects are 58, 71, and 79/min.

FIG. 25(a) is a case where, contrary to the case in FIG. 22(a), a breakpoint appeared in a chevron-shaped power spectrum, and in FIG. 25(b), BF appeared as the linear Red in a band sandwiched by Red-Green-Red-Blue.

FIG. 26(a) is a case where the breakpoint illustrated in FIG. 22(a) appeared on a ridgeline of the chevron-shaped power spectrum, and in FIG. 27(b), BF appeared as the linear Red sandwiched by Red-Blue-Red-Blue.

FIGs. 27(a), (b) are cases where the power spectrum fluctuation changes. The determination from the frequency results in FIG. 27(a) is difficult, but it is seen that BF can be determined if the STFT method in FIG. 27(b) is used. In FIG. 27(b), BF appeared as the linear Red on the boundary line of Red-Blue.

FIGs. 28(a), (b) are correlation charts between BF and heart rates (BF-HR correlation charts) of fifty subjects, which are found by the BF extraction method from the disappearing frequency of the higher harmonics and are also confirmed by the BF extraction method using STFT. In FIG. 28(a), the vertical axis represents the frequency of BF and the horizontal axis represents HR. When a relationship therebetween is approximated by a quadratic function, the relationship is represented by $y = 0.0173x^2 - 2.5847x + 107.6111$, and its minimum value is at around 10 Hz in the case of 75/min HR. At this time, R2 = 0.8242. The use of this chart makes it possible to find BF without any need for measuring PCG.

Further, BF did not correlate with age, SBP, DBP, or BMI. Further, in the study of BF in a control group of twenty young people, a high correlation was observed between BF found by STFT and BF decided from a BF-HR curve (R2 = 0.85). That is, since MAV (micro acoustic vibration) output from the trunk is collected using the biological signal detection sensor 1 (4SR) of this embodiment, by the analysis by the biological signal analysis device 100, it is possible to identify BF and the cardiac apex beat wave and classify a waveform (a sine wave or a triangular wave) even from a subject in "a thick chest wall group", the identification from which was conventionally difficult.

FIG. 28(b) is a correlation chart found from the disappearing order of higher harmonics and HR. When their relationship is approximated by a quadratic function with the order taken on the vertical axis and with HR taken on the horizontal axis, their relationship is represented by $y = 0.0203x^2 - 3.2258x + 135.4587$, and takes the minimum value at around 10 Hz in the case of 79/mm HR. At this time, R2 = 0.9479. Note that FIGs. 28(a), (b) are composed of data measured in a resting state in the ten-second measurement time in which an RR variation is within 15%.

Therefore, by storing in advance the correlation charts illustrated in FIGs. 28(a), (b) and their quadratic functions in the correlation data storage unit 150, it is possible for the measurement-time condition inferring means 130 to quickly find the measurement-time boundary frequency from the heart rate, and quickly determine and output the measurement-time cardiac condition or health condition of the person according to the boundary frequency.

(Relationship of heart rate variability fluctuation characteristic with heart rate and BF)

A correlation between the fluctuation characteristic of heart rate variability and the heart rate found from ECG in the APW measurement experiment was studied in fifty subjects. As illustrated in FIG. 29(a), their relationship is approximated by a quadratic function, and when HR was 72/min, the fluctuation was 1/f (|n = 1|). On the other hand, when HR was near 50 or 90/min, the fluctuation was $1/f^2$ (|n| = 2).

FIG. 29(b) is the result of a study on a correlation between the fluctuation characteristic of heart rate variability and BF. Their relationship was approximated by a linear function. When BF was around 8 to 12 Hz, the fluctuation was 1/f (|n| = 1), and when BF was about 20 Hz or higher, the fluctuation was $1/f^2$ (|n| = 2).

FIGs. 29(c) to (e) illustrate charts each obtained by dividing ninety-second experiment data of the same subject into nine equal parts, finding BF variation when heart rate varies under respiratory depression at a resting time, and drawing it on a regression curve of a BF-HR correlation chart. In the three subjects, BF shifted on the regression curve as the heart rate varies.

The fluctuation of the heart rate variability is 1/f when the heart rate is 70 to 80/min in the resting state. When this is collated with BF, the fluctuation of the heart rate variability is 1/f when the aforesaid BF is around 8 to 12 Hz, and when BF has a value near this range, it can be inferred that the heart is moving efficiently. Therefore, by storing the correlation chart illustrated in FIG. 29(b) in the correlation data storage unit 150, it is possible for the measurement-time condition inferring means 130 to infer the measurement-time fluctuation characteristic of the heart rate variability quickly from BF obtained by the boundary frequency identifying means 120. At this time, if the fluctuation characteristic can be inferred as 1/f, it is possible to infer that the movement of the heart is good, that is, the subject is healthy in terms of the movement of the heart. As the value of BF more falls out of around 8 to 12 Hz where the fluctuation is 1/f, it can be inferred that the influence of aging, the influence of disease, or the like is larger.

(Second Embodiment)

Next, a second embodiment of the present invention will be described based on FIG. 30. In this embodiment, a biological signal analysis device 100 includes a cardiac apex beat waveform extracting means 160 and a waveform classifying means 170 in addition to the structure of the first embodiment described above.

The cardiac apex beat waveform extracting means 160 filters biological signal data with the upper limit value being set to the boundary frequency (BF) identified by the boundary frequency identifying means 120, and finds a waveform of vibration generated by cardiac apex beat. In this embodiment, for the filtering, a 0.5 Hz to BF bandpass filter is used as described above. The cardiac apex beat waveforms (CAB) and the heart sound waveforms (CAS) are drawn in FIG. 15 to FIG. 18 of the first embodiment. The cardiac apex beat waveforms at this time are found using the 0.5 Hz to BF bandpass filter by the same method as that used by the cardiac apex beat waveform extracting means 160 of this embodiment. Further, the heart sound waveforms are found using a BF to 50 Hz bandpass filter.

The waveform classifying means 170 classifies a waveform of vibration generated by cardiac apex beat (cardiac apex beat waveform) output from the cardiac apex beat waveform extracting means 160. In this embodiment, for the classification, used is a combination of (A) a means that classifies the waveform by a mathematical approach using the Fourier series expansion, and (B) a means that classifies the waveform based on data obtained by an engineering approach. In this embodiment, cardiac apex beat waveforms of healthy people are classified into five using the two approaches, which will be described in detail later.

The means that classifies the waveform based on the data obtained by the engineering approach classifies the waveform using at least one of the following four determination elements. For higher classification accuracy, a combination of two or more of the determination elements is preferably used for the classification.

(1) Data indicating the shape of a time waveform in a predetermined time range of the aforesaid waveform of the vibration generated by the cardiac apex beat (the charts (b) in FIGs. 32 to FIGs. 36)
(2) Data of a graph of the time waveform, in which frequency and power spectrum are taken on the horizontal axis and the vertical axis, the data being obtained by the frequency analyzing means (the charts (c) in FIGs. 32 to FIGs. 36)
(3) Image data in a predetermined time range which is the analysis result of the short time Fourier transform of the time waveform and shows the time, the frequency, and the degree of the power spectrum variation (the charts (d) in FIGs. 32 to FIGs. 36)
(4) Data regarding the type and period of a waveform, in a predetermined time range, which data is extracted from a correlogram (the charts (e) in FIGs. 32 to FIGs. 36)

Out of the above, it is first required that the time waveforms of the healthy people are classified into five waveforms by the mathematical approach using the Fourier series expansion, and as its verification, amplitude, frequency, and time phase are analyzed by the engineering approach, and data corresponding to the five waveforms are found from the shape data of the time waveform of Front CAB (0.5-BF) (the charts (b) in FIGs. 32 to FIGs. 36), data of a graph representing power spectrum on the horizontal axis and the vertical axis (the charts (c) in FIGs. 32 to FIGs. 36), graphic data of the STFT analysis result (the charts (d) in FIGs. 32 to FIGs. 36), and data extracted from the correlogram (the charts (e) in FIGs. 32 to FIGs. 36). This waveform evaluation method will be further described later.

Information on the classification results of the waveforms classified by the waveform classifying means 170 is stored in the correlation data storage unit 150. Here, the information of the waveform classification result is stored in association with information regarding the health condition (the presence/absence of a disease such as a heart disease, the identification of a disease, whether the physical condition is good or not, and so on). To create this data, cardiac apex beat waveforms of as many people as possible are measured and they are associated with the health conditions at the time of the measurement.

The measurement-time condition inferring means 130 causes the waveform classifying means 170 to classify a measurement-time waveform of vibration generated by cardiac apex beat of a measurement subject which waveform is found by the cardiac apex beat waveform extracting means 160, and collates the measurement-time waveform classification result with the information of the waveform classification result stored in advance by accessing the correlation data storage unit 150. Consequently, the health condition corresponding to the measurement-time waveform classification result is output.

As illustrated in FIG. 31, the biological signal analysis device 100 may further include a model creating means 180 that uses, as training data, the health conditions and the waveform information of the cardiac apex beat waveforms which are output by the cardiac apex beat waveform extracting means 160 to be classified by the waveform classifying means 170, to create, by machine learning an inference model for inferring the health condition from the waveform information. Further, in the machine learning, taking the information of the classification result by the waveform classifying means 170 (classification information) into consideration improves the accuracy of the inference model.

In this case, the measurement-time condition inferring means 130 receives the measurement-time waveform information of the vibration generated by the cardiac apex beat of the measurement subject, and by using the inference model created by the model creating means 180, outputs a value of the measurement-time health condition based on the obtained measurement-time waveform information. Such a configuration to combine the machine learning improves the accuracy of inferring the health condition from the waveform information. Note that learning-target feature quantities of the waveform information are amplitude, frequency, time phase, and so on constituting each of the waveforms illustrated in FIGs. 32 to FIGs. 36, but the image data as the STFT analysis results illustrated in (c) in FIGs. 32 to FIGs. 36 contains a large quantity of information, and the learning-target feature quantities are easily extracted therefrom. Therefore, for the capturing of the waveform information, the STFT image data is preferably included in the learning target. Further, the upper chart and the lower chart in (c) of each of FIGs. 32 to FIGs. 36 are waveforms that are obtained by dividing the cardiac apex beat waveform in 0.5 Hz to BF into two parts whose center frequencies are 1.95 and 7.81 Hz, which further increases the learning-target feature quantities to contribute to inference accuracy improvement.

Note that the other configuration including the biological signal detection sensor 1, of the second embodiment is the same as that of the first embodiment.

Hereinafter, the methods of the waveform classification and evaluation will be more specifically described.

[Evaluation method of time waveform of Front CAB]

(Analysis of time waveform of Front CAB)

Next, the evaluation method of the Front CAB waveform will be described.

The aforesaid apex cardiogram (ACG) is known as one of the physical diagnostic tools serving as a criterion of the non-invasive evaluation and as an index of disease condition and treatment, regarding the movement of the cardiovascular system, but a supine posture is not an absolute requirement as a posture during examination, and a sitting posture or a left lateral recumbent posture is sometimes taken. Therefore, if information regarding a cardiac apex beat normal pattern is available for each of the measurement postures including all of a supine posture, a left lateral recumbent posture, and a sitting posture, it is possible to study the relationship with the waveform analysis and the time phase analysis of PCG and ECG.

For this reason, Front CAB (0.5-BF), which is a waveform in a 0.5 to BF frequency band extracted from the frequency analysis results of the F-APW waveform measured by a microphone placed on the chest wall, is generated, and this is compared with a clinically known cardiac apex beat waveform. As focusing points of the waveform analysis, attention is given to "tapping" of an E-wave which is short-duration pulsation recognized only in early systole even by ocular inspection and palpation, and to a long-duration, strong "sustained". Here, for the waveform analysis, the shape of the waveform of Front CAB (0.5-BF) and the time phase when the peak of ACG appears are used.

The evaluation of the amplitudes of the time waveforms in Front CAB (0.5-BF) is performed in the time phase of systole. An A-wave originating in left atrial contraction and a trace of the A-wave are confirmed in Front CAB (0.5-BF). A bottom value after the A-wave is considered as a C point of ACG, and the total amplitude up to the peak value corresponding to the E-wave of ACG is measured and is defined as the amplitude of the systolic wave. This total amplitude from the C point up to the E-wave in ACG is calculated also in the time waveforms of Front CAB (5-BF), Rear CAB (0.5-BF), and Lumbar CAB (0.5-BF). When the amplitudes of the time waveforms measured at the respective positions are compared, that of Front CAB (0.5-BF) is used as a reference value. An amplitude ratio is a ratio in each waveform. The bottom value of the amplitude of the time waveform is present between a P-wave and an R-wave in an electrocardiogram. The peak value of the amplitude of the time waveform is present in a range after the R-wave up to the starting point of second sound of PCG. Next, the time waveforms of Front CAB (0.5-BF) of fifty subjects are classified.

(Higher harmonic component analysis method using short time Fourier transform)

Next, a wave steepness analysis method and a higher harmonic component analysis method using the short time Fourier transform will be described.

Targets of the study are higher harmonics forming the time waveform of Front CAB (0.5-BF), and it was studied at which position of the time waveform the higher harmonic components are superimposed, using Formula (4). In the analysis, the time waveform is divided into those in frequency bands of 1 to 4 Hz and 4 to 16 Hz. The number of window points for the 1 to 4 Hz waveform analysis was $2^9$= 512. The 512 points correspond to 0.512 seconds because the sampling frequency is 1000 Hz, and according to the calculation, a component of the power spectrum of 1.95 Hz appears. A shift width of a Hanning window function is set to 51 points. At this time, frequency information is created every 51/1000 = 0.051 seconds.

The number of window points for capturing an E-wave formed of higher harmonics was set to $2^7 = 128$. Because the sampling frequency is 1000 Hz, 128 points correspond to 0.128 seconds, and a component of the power spectrum appears at 7.8 Hz. A reason why the resolution was set to 0.12 seconds (7.8 Hz) is that the duration of systole in the case of 90/min heart rate is assumed to be 0.26 seconds, and the required resolution of a detection frequency for detecting the peak in the duration of the systole is set to 0.13 seconds. In this case, the shift width of the Hanning window function was set to 12 points. At this time, the frequency information is created every 12/1000 = 0.012 seconds.

(Vibration discrimination using autocorrelation function)

A correlogram is drawn using ACF (Autocorrelation function), and waveform distinction is performed. In the correlogram, the vertical axis represents ACF and the horizontal axis represents time. ACF in each time interval T is calculated, and the type and period of the waveform are captured from the correlogram. Note that ACF, that is, $R_x(\tau)$ is represented by the following.
[Formula 6]

$$R_x(\tau) = \lim_{T\to\infty}\frac{1}{T}\int_0^T x(t)x(t+\tau)dt = \lim_{T\to\infty}\frac{1}{T}\int_{-\frac{T}{2}}^{\frac{T}{2}} x(t)\,x(t+\tau)dt \quad \cdot\cdot\cdot (6)$$

$\tau$ represents time interval, that is, lag, and T represents total interval.

A normalized autocorrelation function takes the maximum value 1 when $\tau = 0$, and this value is a mean square value. When the ACF value is 0.2 or less, it is in an interval of 95% or higher confidence of the absence of autocorrelation. In the vibration type analysis, ten-second data is used for determination.

[Evaluation result of time waveforms of Front CAB].

Using Front CAB (0.5-BF) measured from the fifty subjects, normal patterns in a sitting posture were classified and verified. FIGs. 32 to FIGs. 36 illustrate the results. The waveforms of Front CAB (0.5-BF) are illustrated in the charts (b) in FIGs. 32 to FIGs. 36 and correspond to one-second time waveforms of ECG illustrated in the charts (a) in FIGs. 32 to FIGs. 36. The waveforms of Front CAB (0.5-BF) are classified into five waveforms. Waveforms similar to a ventricular systolic wave starting from an A-wave (atrial systolic wave: marked with the arrowhead) and an E-wave (marked with *) in an apex cardiogram are recognized.

The method of the classification into the five waveform patterns is based on the Fourier series expansion. However, the mathematical expression is not suitable for intuitive understanding. Therefore, in studying the waveforms of Front CAB, the characteristics of the waveforms of Front CAB were classified, using PSD (power spectral density) when focus is on amplitude, using STFT when focus is on amplitude and phase, and using a correlogram when the presence/absence of periodicity is studied.

In this specification, the five waveforms resulting from the classification are named a pseudo sine wave (S-wave), a pseudo triangular wave group (T-wave (triangular wave), C-wave (cosine wave), G-wave (Gauss wave)), and a pseudo full-wave rectified wave (R-wave). Out of these, the S wave and the G-wave correspond to "sustained" and the T-wave, the C-wave, and the R-wave correspond to "tapping", as is said in ocular inspection/palpation, in view of the duration of a systolic wave. Note that the prefix "pseudo" is appended because they have similar waveform shapes to those of a sine wave, a triangular wave, and a full-wave rectified wave though not complete sine wave, triangular wave, and full-wave rectified wave.

What are necessary for the waveform analysis are a waveform of a fundamental harmonic, and amplitudes, frequencies, and phases of waveforms near the bottom and the peak.

In (c) in FIGs. 32 to FIGs. 36, the power spectral densities (PSD) were found by frequency analyses, and relationships between amplitude and frequency were classified. In (e) of FIGs. 32 to FIGs. 36, the types of vibration were classified using the correlogram. Based on these parameters, the time waveforms of Front CAB (0.5-BF) were classified into five typical waveforms.

In more detail, based on PSD, the classification into the following waveform patterns is possible.

First, a systolic E-wave indicated by the S-wave of Front CAB resembles a sine wave, with less contribution of higher harmonic components. That is, only a fundamental frequency component is dominant (the "X" pattern illustrated in the charts).

Next, a systolic E-wave indicated by the T-wave, the C-wave, and the G-wave of Front CAB is in the process of converging to a triangular wave and is classified as a triangular wave. That is, odd-order frequency components are dominant, and their amplitudes become smaller in proportion to a reciprocal of the square of the order as the order becomes higher (the "Y" pattern illustrated in the charts).

In the R-wave of Front CAB, because the amplitude of a fundamental frequency component is small and has a small difference from the amplitude of a component of a frequency that is an integral multiple of the frequency of the fundamental frequency component, a triangular wave part is replaced by a full-wave rectified waveform of a sine wave. In corresponding PSD, an even-order frequency component is dominant, and the amplitude becomes smaller as the order becomes higher (the "Z" pattern illustrated in the charts).

STFT is suitable for studying the influence of phase information, and combining PSD and STFT improves the accuracy of the waveform classification. Using the correlogram with PSD and STFT to study periodicity between the waveforms of the A-wave and the E-wave makes it possible to classify a given waveform as a variant of a normal waveform from the viewpoint of amplitude, phase, and periodicity.

The STFT charts (d) in FIGs. 32 to FIGs. 36 are obtained by dividing the power spectrum information in a range from the fundamental harmonic up to 5 Hz appearing in (c) of FIGs. 32 to FIGs. 36 into two parts whose center frequencies are 1.95 Hz and 7.81 Hz. This doubles the information amount regarding the power spectrum to facilitate pattern matching (in (d), the upper charts correspond to 1.95 Hz and the lower charts correspond to 7.8 Hz). The information on the fundamental harmonic and higher harmonics of 1 to 5 Hz in (c) in FIGs. 32 to FIGs. 36, is drawn between 1 and 5 Hz in STFT. Note that the information on the time waveform is represented by Red, Green, and Blue contour lines.

In the waveform I (S-wave), there are three time zones where the fundamental harmonic appears, there are two time zones where 5 to 10 Hz components appear, and an appearance interval of an atrial bulging wave and a systolic wave is short, which indicates a square wave. In the waveform II (T-wave), the appearance of 5 Hz or lower components concentrates in a 2.3 to 2.5 second period in STFT, 5 to 10 Hz components concentrate in a 2.5 to 2.7 second period, and these components are roughly within a systolic time phase, which indicates a triangular wave. Similarly, the waveform III (C-wave) is also within the systolic time phase, and an interval between an atrial bulging wave and a systolic wave is short, which indicates a triangular wave. In the waveform IV (G-wave), a peak-to-peak interval of the wave in 5 to 10 Hz is close to that of the waveform I (S-wave), its shape is a combined shape of the S-wave and the T-wave, and it as a whole presents the feature of a triangular wave. In the waveform V (R-wave), a peak value in STFT in 5 Hz or lower presents a declining contour line, which indicates that the power spectra of the fundamental harmonic up to higher harmonics are equal in magnitude, further there are three or more peak values in not lower than 5 Hz nor higher than 10 Hz, and the appearance time phase is the same as that of a component of 5 Hz or lower, which indicates the feature of a full-wave rectified waveform.

Using the correlograms (e) in FIGs. 32 to FIGs. 36, the types of vibrations (periodicity between the waveforms of the A-wave and the E-wave) were classified based on how a notch of a sine wave appeared, with an autocorrelation function of not lower than 0.5 nor higher than 0.2 used as a reference value. Here, as the waveforms of the correlograms of Front CAB (0.5-BF), "i" which is the sine wave, "ii" which is a waveform with one notch on the sin wave, and "v" which is a waveform with two or more notches on the sine wave are extracted. The other waveforms are classified into "iii" in which one side of the envelope is decayed, and iv in which both sides of the envelope is decayed.

[Theoretical ground and objective classification criteria of waveform classification]

Here, the mathematical approaches to a cardiac apex beat wave using the Fourier series expansion will be described in detail. It has been shown that cardiac apex beat waves of healthy people are classified into five by these mathematical approaches, and PSD, STFT, and the correlogram described above serve as engineering approach-based supports for the classification into five.

First, let a given periodic function be f(t), then f(t) can be expanded to the following Fourier series.

Note that T is period, and $\omega = 2\pi/T$.

[Formula 7]

$$f(t) = a_0 + \sum_{n=1}^{\infty}(a_n \cos n\omega t + b_n \sin n\omega t) \cdot\ \cdot\ \cdot (7)$$

The S-wave corresponds to a state where a first-order component is dominant in Formula (7) because its waveform shape resembles that of a sine wave. However, since it also includes some higher harmonic components, it can be interpreted from the STFT analysis result that its main component is a fundamental frequency accompanied by the high-order frequency components.

This agrees with the observation result of the short duration "Sustained" in systole.

It is thought that the T-wave can be expanded to the Fourier series as follows because it resembles a triangular wave.

[Formula 8]

$$f(t) = \frac{8V_m}{\pi^2} \sum_{n=0}^{\infty} \frac{\cos(2n+1)\omega t}{(2n+1)^2} \cdot \cdot \cdot (8)$$

Formula (8) shows that an expansion coefficient sharply decreases as the order of n becomes higher.

It also shows that the order that appears as a spectrum is an odd order.

This can explain the shape of PSD.

The influence of waveforms of higher harmonics other than the sine wave accounts for the result that the autocorrelation coefficient shown by the correlogram has a peak value when t = 0 and the absolute value of the autocorrelation coefficient is smaller than 1 at the other times.

This means that a short-duration, sharp waveform originating in ventricular contraction in early systole is superimposed on the sine wave, which agrees with the observation result of short-duration "Tapping" in early systole.

Of the C-wave, a waveform originating in ventricular contraction can also be discussed in the same manner as is discussed about the T-wave. Its difference from the T-wave is that in the Fourier series expansion of Formula (8), a first-order component is more dominant in the C-wave than in the T-wave.

It is seen from the result of the STFT analysis that a waveform originating in ventricular contraction is formed mainly of a fundamental frequency and a second harmonic and of frequency components up to the seventh harmonic, and the correlogram agrees with the observation result of short-duration "Tapping" in early systole similarly to the T-wave.

From the STFT analysis, the G-wave is considered as a combination of the S-wave and the T-wave, and a first-order component is dominant therein. Since it more resembles the S-wave rather than the T-wave, its duration in systole is long, which agrees with the observation result of "Sustained".

In the R-wave, as for a waveform originating in ventricular contraction, the waveform itself is not unimodal but multimodal, but under the hypothesis that it resembles a full-wave rectified waveform, it can be expanded to the following Fourier series.

[Formula 9]

$$f(t) = V_m \left[\frac{2}{\pi} - \frac{4}{\pi} \sum_{n=1}^{\infty} \frac{\cos 2n\omega t}{4n^2-1}\right] \cdot \cdot \cdot (9)$$

From Formula (9), it is considered that the R-wave starts with the second-order component with an even-order harmonic being superimposed thereon. An expansion coefficient becomes smaller as n becomes larger. It is thought that the R-wave has the shape illustrated in the PSD chart because the R-wave also includes the first-order component.

The analysis of the correlogram shows a reason why components other than the first-order component are dominant as in T-wave. This agrees with the observation result of short-duration "tapping "in early systole.

As described above, from the correlograms, the waveforms are classified into the S-wave and the G-wave which have the primitive period of the sine wave, and classified into the T-wave, the C-wave, and the R-wave in which the higher harmonics are prominent. Further, when attention is paid to the difference in the autocorrelation function with respect to lag, the S-wave and the G-wave can be distinguished on the + side of the correlogram, and the T-wave and the C-wave can be distinguished on the - side of the correlogram, and the R-wave can be distinguished by the notch indicating multimodality.

That is, in the classification using the correlogram, attention is paid to sinusoidal periodicity for the S-wave and the G-wave, while attention is paid to distorted wave periodicity for the T-wave, the C-wave, and the R-wave. The periodicities of systolic waves are classified by the autocorrelation function on the + side of the correlogram, and the periodicities of the atrial bulging waves and other waveforms are classified by the autocorrelation function on the - side of the correlogram. It was possible to sort the S-wave and the G-wave according to a difference in autocorrelation with respect to lag, and sort the T-wave, the C-wave, and the R-wave according to the value (0, -0.2, and -0.5 in the above example) of the autocorrelation coefficient on the - side. Then, for the irregularities of the atrial bulging wave and the other waveforms, the drawn shape (notch) of the autocorrelation coefficient was used. Since the R-wave is multimodal, it had two notches or more.

Using the charts (b) to (d) in FIGs. 32 to FIGs. 36, the waveforms were identified by the pattern matching of fifty cases. As a result, generation ratios of the pseudo sine wave (S-wave), the triangular wave (T-wave), the cosine wave (C-wave), the Gauss wave (G-wave), and the pseudo-full wave rectified wave (R-wave) were 4/50 = 80%, 8/50 = 16%, 15/50 = 30%, 11/50 = 22%, and 12/50 = 24% respectively.

FIG. 37 illustrates the results of the waveform studies using the power spectrum, two types of STFT, and the correlogram which are illustrated in the charts (b) to (e) in FIGs. 32 to FIGs. 36, without using the waveform identification decided in the charts (a) in FIGs. 32 to FIGs. 36. The thick letters in the charts indicate the number of cases where the relevant waveform can be verified. Based on STFT and the correlogram, waveforms could be verified in 43 cases except for one case of the triangular waveform, out of the fifty cases. By the use of the power spectrum, the waveforms could be verified in 38 cases out of the fifty cases. Note that the P value was $P = 2.2 \times 10^{-16}$ in the STFT verification, $P = 7.9 \times 10^{-5}$ in the power spectrum verification, and $P = 2.2 \times 10^{-16}$ in the correlogram verification. Since STFT and correlogram (ACF) are in an inverse Fourier transform relation according to the Wiener-Khintching theorem, the result that the P values in both are equal supports the validity of the analysis method. Therefore, a possibility has been found out that combining the three types of verification can improve the accuracy of the waveform verification of Front CAB (0.5-BF), and can increase the accuracy of the classification of the waveforms which have been conventionally evaluated based on the shape of the waveform of ACG, and it has been found out that the 4SR sensing system has waveform resolution necessary for finding abnormality, for example, for discriminating between normal heart and hypertrophied/dilated heart.

Therefore, in this embodiment, when the classification result of the cardiac apex beat wave of a measurement subject obtained by the waveform classifying means 170 corresponds to any of the aforesaid five patterns which are waveform patterns of healthy people, the measurement-time condition inferring means 130 determines that the subject is healthy and outputs this result, and when the classification result does not correspond to any of these, it determines that there is an abnormality finding regarding a heart disease and outputs this result.

FIGs. 38(a) to (e) illustrate the frequency analysis results of Front CAB (0.5-BF), Front CAB (5-BF), Rear CAB (0.5-BF), and Lumbar CAB (0.5-BF) of five subjects, as typical examples of the five waveforms defined in the charts (a) in FIGs. 32 to FIGs. 36. FIG. 39 illustrates a mean value and a standard deviation of an amplitude ratio of CAB at each measurement position based on the frequency analysis results. The amplitude ratio indicates a difference in the magnitude of the amplitude at each measurement position from the amplitude of Front CAB (0.5-BF) to serve as an index indicating measurement easiness. The amplitude ratio is calculated using the maximum amplitude value of a ventricular systolic wave during a period from a starting point of a left ventricular pressure rise in the systolic wave, described in the above section of (Time waveform analysis of Front CAB). The amplitude ratio of Front CAB (5-BF) is found from a ratio of the amplitude of Front CAB (5-BF) to the amplitude of Front CAB (0.5-BF). Similarly, the amplitude ratio of Rear CAB (0.5-BF) is found from a ratio of the amplitude of Rear CAB (0.5-BF) to the amplitude of Front CAB (0.5-BF), and the amplitude ratio of Lumbar CAB (0.5-BF) is found from a ratio of the amplitude of Lumbar CAB (0.5-BF) to the amplitude of Front CAB (0.5-BF). Here, the amplitude ratio of Front CAB (5-BF) to that of the Front CAB (0.5-BF) was 0.2 because it does not include a 0.5 Hz to 5 Hz frequency component. The amplitude ratio of Rear CAB (0.5-BF) was 1.1, and the amplitude ratio of Lumbar CAB (0.5-BF) was 2.6. The mean value and the standard deviation were calculated using the amplitude ratios of the fifty subjects.

Next, the power spectra in FIGs. 38(a) to (e) and the amplitudes in FIG. 39 will be studied. In Rear CAB (0.5-BF) and Lumbar CAB (0.5-BF), the power spectra increased around 5 Hz. Specifically, in Rear CAB (0.5-BF), the power spectrum became 1.8 to 4.8 times around 5 Hz, and in Lumbar CAB (0.5-BF), the power spectrum became 3.7 to 7.9 times around 5 Hz. The difference in the power spectrum of the higher harmonics of around 5 Hz is due to the length of the aorta, and this indicated that a liquid column resonance phenomenon due to the aorta and pressure pulsation occurred.

From the above, according to the present invention, the following specific effects were obtained.

The conventional 3SR is a sensing system that is developed for on-vehicle use and damps external vibration by the air damping effect of the air pack to extract first sound and second sound of heart sound including 20 Hz or higher frequency components and periodic components of heart rate variability. On the other hand, 4SR of the present invention is developed for use in rooms, and it has been found out that 4SR is suitable as a sensor for capturing the period and the amplitude of cardiac apex beat of around 1 to 2 Hz.

The waveform of F-APW was similar to that of a cardiac apex beat wave. Then, from F-APW, it was possible to generate waveforms of a cardiac apex beat wave and heart sound. It is considered that the waveforms of R-APW and L-APW became different due to a resonance phenomenon caused by pressure pulsation. However, from R-APW and L-APW whose waveforms were different from that of F-APW, it is also possible to extract vibration information (CAB) regarding the movement of the heart including cardiac apex beat and heart sound (CAS) by using the vibration analysis, and the possibility of clinical significance is suggested.

By combining the BF extraction method based on the disappearing frequency of the higher harmonics in F-APW and the BF extraction method using the STFT method, the BF-heart rate correlation chart is created, BF can be represented by the quadratic function of heart rate according to the actual measurement results, and it is possible to decide BF based on the heart rate in real time only with data of F-APW, without using PCG. Further, a possibility is suggested that the use of the visualization method by the STFT method enables not only the vibration analysis of the vibration of the heart and pressure pulsation of the aorta but also diagnosis using a change degree.

The experiment result of the fluctuation of heart rate variability showed that when the heart rate was 70 to 80/min in the resting state, the fluctuation was 1/f and the movement of the heart is efficient. The experiment result of the fluctuation of heart rate variability showed that when BF was around 10 Hz, the fluctuation of the heart rate variability was 1/f and the movement of the heart is efficient. The use of these characteristics has a possibility that a change in the circulatory organ function caused by drug can be quantified.

As described above, BF is decided based on the heart rate (for ten seconds in this case). The tendency of the value of the fluctuation (for ninety seconds) of the heart rate variability obtained from BF agreed with the tendency of the value of the fluctuation (for ninety seconds) of heart rate variability directly obtained from the heart rate (for ninety seconds). The result found when their relational formula was substituted supported the validity of BF.

Further, by applying the 0.5 Hz to BF band-pass filtering to APW, it was possible to draw a waveform resembling that of ACG, and it was possible to classify Front CAB (0.5-BF) representing the cardiac apex beat roughly into the five waveforms, by the frequency analysis, the time-series analysis method using the STFT method, and the waveform analysis method using the correlogram.

When heart rate variability occurs under respiratory depression in the resting state, BF shifts on the regression curve of the BF-HR correlation chart (see FIG. 40), which suggests the possibility that the movement of the heart can be analyzed, and pressure pulsation, the state of the movement of the heart, and the ventricular blood flow can be evaluated, using the above-described vibration analysis method and BF-HR correlation chart.

Further, in a supine posture and left and right lateral recumbent postures, the same BF-HR correlation charts were obtained as those obtained in the sitting posture (see FIGs. 41). Therefore, a possibility is suggested that the inference can be made only with one graph because the same BF-HR correlation chart is obtained irrespective of posture, and since the measurement method using 4SR is capable of easily obtaining data, it has high practicability and is applicable for making the ocular inspection/palpation objective, and accordingly APW has a potential to be a physical diagnostic tool serving as criteria for the non-invasive evaluation of the movement of the cardiovascular system and as an index of a disease and treatment.

According to the present invention, signals belonging to two frequency bands are separated through the identification of BF.

Specifically, since signals corresponding to cardiac apex beat and heart sound can be separated in APW, it is possible to quantitatively recognize the results of different types of operations of cardiac activity by observing one physiological signal, which makes it possible to obtain useful complementary information regarding the cardiac condition. Further, since the signal separation increases waveform resolution, there is a possibility that, for example, a frequency as the boundary between two frequency bands, higher harmonic components of Front CAB near the boundary, and a dominant frequency component of Front CAS serve as new parameters of the cardiocirculatory system.

Further, Front CAB has pressure information of a systolic wave in an apex cardiogram, and Front CAS has time phase information relating to the cardiac cycle, and the combination of the pressure and the time phase information is thought to be usable for diagnosis from the complementary information.

For example, first heart sound can be identified from Front CAS, and a notch corresponding to the first sound of an anacrotic limb of a systolic wave can be observed both in a wide range and a narrow range in STFT illustrated in (b), (c) in FIGs. 32 to FIGs. 36. Changing a window width in a 0 to 20 Hz band makes it possible to change an observable range, and adjusting contrast facilitates the discrimination of the notch. This is usable for determining whether there is a notch or not in a systolic pressure wave, and this notch is usable for identifying a cause of cardiac activity abnormality and thus usable for diagnosis. Front CAB which is cardiac vibration information and Front CAS which is cardiac acoustic information indicate dynamic characteristics of mainly the atria, the ventricles, the valves, blood, and the aorta. Then, the result of their complementarity appears as fluctuation of blood pressure, heart rate, and respiratory rate. It is also possible to quantify a change occurring while a medicine is applied to a patient under medication. For example, in diagnosis of left ventricular failure, the comparison of an alarming-level dynamic characteristic of the left ventricle with the left ventricular dynamic characteristics of healthy people in the same age group is also useful in the diagnosis of the presence of the left ventricular failure. Further, associating this with conduction path and speed characteristics and with ECG information such as potential has a possibility that the health condition of cardiac muscle cells can be inferred. From the information of Front CAB and Front CAS, it may also be possible to monitor the cardiac condition and provide data relating to function evaluation. As a result, it may be possible to simulate the size of the ventricles, the thickness of the cardiac wall, and the dynamic characteristic of the heart to describe the movement by a numerical formula.

Measuring data along the aorta makes it possible to separate the data into those originating in cardiac apex beat and heart sound in hemodynamics of the aorta, and may make it possible to obtain new findings regarding the hemodynamics of the aorta.

As is apparent from the above, it is seen that by extracting APW (Acoustic Pulse Wave) of 0.5 to 80 Hz and applying predetermined filtering, it is possible to generate an ACG-equivalent waveform (Cardiac Apex Beat: CAB) and a heart sound-equivalent waveform (Cardiac Acoustic Sound: CAS).

Further, CAB and CAS are different in the generation source and transmission route, and vibration resulting from the superposition of these two vibrations is APW. Considering that systolic performance is a characteristic unique to the cardiac muscles and is independent of afterload and preload, the waveform of CAB in systole has information regarding the influence of chemical stimulation and hormone at the time of contraction. Considering that CAS has movement information of blood ejected from the ventricle in systole, APW has information on preload and afterload and on systolic performance.

Therefore, APW, CAB, and CAS have a lot of information regarding sound, vibration, and pressure, and observing them together with a polarization phenomenon that ECG has enables diagnosis of cardiac failure.

It has been also found out that the real-time measurement of APW, CAB, and CAS additionally provides clinically significant information on acoustic vibration and pressure, and the engineering approach having been used for diagnosis of a failure of a machine is applicable to health diagnosis. Further, the 4SR system of the present invention that measures APW serves as a physical diagnostic tool capable of repeatedly obtaining biological information non-invasively over a long period, and is expected to have wide applications including AI analysis including the aforesaid mechanical learning.

Explanation of Reference Signs

| | |
|---|---|
| 1 | biological signal detection sensor (4SR) |
| 10 | three-dimensional knitted fabric |
| 20 | housing film |
| 30 | microphone |
| 40 | case |
| 50 | gel |
| 100 | biological signal analysis device |
| 110 | frequency analyzing means |
| 120 | boundary frequency identifying means |
| 130 | measurement-time condition inferring means |
| 140 | heart rate calculating means |
| 150 | correlation data storage unit |
| 160 | cardiac apex beat waveform extracting means |
| 170 | waveform classifying means |
| 180 | model creating means |
| 1000 | biological signal detection sensor (3SR) |

**Claims**

**1.** A biological signal analysis device comprising:

a frequency analyzing means that frequency-analyzes biological signal data obtained by a biological signal detection sensor through a body surface; and

a boundary frequency identifying means that finds a boundary frequency between vibration generated by cardiac apex beat and vibration generated by heart sound in the biological signal data, from a result of the frequency analysis of the biological signal data which result is obtained from the frequency analyzing means.

**2.** The biological signal analysis device according to claim 1,
wherein the boundary frequency identifying means includes a means that finds, in the result of the frequency analysis, a power spectrum sudden changing point which is a boundary between harmonic vibration and random vibration, and identifies the boundary frequency based on the sudden changing point.

**3.** The biological signal analysis device according to claim 2,
wherein the boundary frequency identifying means includes a means that finds the power spectrum sudden changing point, in consideration of a result of a frequency analysis of heart sound data measured simultaneously.

**4.** The biological signal analysis device according to claim 3,
wherein the boundary frequency identifying means includes:

a log-log plot means that represents, in log-log axes, a waveform resulting from addition averaging of the results of the frequency analyses of the biological signal data and the heart sound data, using a log difference method; and
a sudden changing point identifying means that finds a fluctuation changing point from the waveform represented in log-log axes, and identifies the fluctuation changing point as the power spectrum sudden changing point.

**5.** The biological signal analysis device according to claim 2 or 3,

wherein as the frequency analyzing means, short time Fourier transform is employed, and
wherein the boundary frequency identifying means includes a means that finds the power spectrum sudden changing point from an analysis result of the short time Fourier transform.

**6.** The biological signal analysis device according to claim 5,

wherein the frequency analyzing means includes a means that outputs the analysis result of the short time Fourier transform as image data showing time, frequency, and a degree of power spectrum variation, and
wherein the boundary frequency identifying means includes a means that finds the power spectrum sudden changing point from the image data.

**7.** The biological signal analysis device according to any one of claims 1 to 6, further comprising:

a correlation data storage unit in which correlation data relating to the boundary frequency is stored; and
a measurement-time condition inferring means that infers a measurement-time health condition of a measurement subject, by referring to the correlation data.

**8.** The biological signal analysis device according to claim 7,

wherein the correlation data is correlation data of the boundary frequency and heart rate, and
wherein the measurement-time condition inferring means includes a means that infers a measurement-time boundary frequency of the measurement subject by collating a measurement-time heart rate of the measurement subject with the correlation data of the boundary frequency and the heart rate.

**9.** The biological signal analysis device according to claim 7,

wherein the correlation data is correlation data of the boundary frequency and a fluctuation characteristic of heart rate variability, and
wherein the measurement-time condition inferring means includes a means that infers a measurement-time fluctuation characteristic of heart rate variability of the measurement subject by collating a measurement-time boundary frequency of the measurement subject with the correlation data of the boundary frequency and the fluctuation characteristic of the heart rate variability.

**10.** The biological signal analysis device according to claim 7, further comprising:

a cardiac apex beat waveform extracting means that filters the biological signal data, with an upper limit value being set to the boundary frequency identified by the boundary frequency identifying means, to find a waveform of the vibration generated by the cardiac apex beat; and
a waveform classifying means that classifies the waveform of the vibration generated by the cardiac apex beat, wherein the measurement-time condition inferring means includes a means that causes the waveform classifying means to classify a measurement-time waveform of the vibration generated by the cardiac apex beat of the measurement subject, which waveform is obtained by the cardiac apex beat waveform extracting means, and infers the measurement-time health condition of the measurement subject based on data of a result of the classification of the measurement-time waveform.

**11.** The biological signal analysis device according to claim 10,

wherein correlation data of a result of the waveform classification and health condition is stored in the correlation data storage unit in advance, and
wherein the measurement-time condition inferring means includes a means that causes the waveform classifying means to classify the measurement-time waveform of the vibration generated by the cardiac apex beat of the measurement subject, which waveform is obtained by the cardiac apex beat waveform extracting means, and infers the measurement-time health condition of the measurement subject by collating the result of the classification of the measurement-time waveform with the pre-stored correlation data of the waveform classification result and the health condition.

**12.** The biological signal analysis device according to claim 10, comprising:

a model creating means that uses information on waveforms and the health conditions as training data, the waveforms being output by the cardiac apex beat waveform extracting means to be classified by the waveform classifying means, to create, by machine learning, an inference model for inferring the health condition from the waveform information,
wherein the measurement-time condition inferring means includes a means that receives the information of the measurement-time waveform of the vibration generated by the cardiac apex beat of the measurement subject, and outputs a value of the measurement-time health conditions based on the obtained information of the measurement-time waveform, using the inference model created by the model creating means.

**13.** The biological signal analysis device according to any one of claims 10 to 12,
wherein the waveform classifying means uses the following means (A) and (B):

(A) a means that classifies the waveform by a mathematical approach using Fourier series expansion; and
(B) a means that classifies the waveform based on a combination of one or two or more of the following data (1) to (4) obtained by engineering approaches:

(1) data indicating a shape of a time waveform in a predetermined time range of the waveform of the vibration generated by the cardiac apex beat;
(2) data of a graph of the time waveform, in which frequency and power spectrum are taken on a horizontal axis and a vertical axis, the data being obtained by the frequency analyzing means,
(3) image data in a predetermined time range that is the analysis result of the short time Fourier transform of the time waveform and shows the time, the frequency, and the degree of the power spectrum variation; and
(4) data regarding a type and a period of a waveform, in a predetermined time range, which data is extracted from a correlogram,

to classify waveforms of vibrations generated by the cardiac apex beat of healthy people into five, and
infers whether the measurement-time health condition of the measurement subject is a healthy condition or not based on whether or not the result of the classification of the measurement-time waveform of the measurement subject corresponds to any one of the five results of the waveform classification.

**14.** The biological signal analysis device according to claim 13, wherein the image data is divided based on a plurality of frequencies, and a plurality of image data are created in the predetermined time range.

**15.** The biological signal analysis device according to any one of claims 1 to 14,
wherein the biological signal detection sensor includes:

a three-dimensional knitted fabric;
a housing film that sealingly covers a periphery of the three-dimensional knitted fabric;
a microphone disposed on an outer side of the housing film;
a case covering the microphone; and
an external disturbance mixture preventing member that functions to prevent external disturbance from mixing into the microphone in the case.

**16.** The biological signal analysis device according to claim 15, wherein the external disturbance mixture preventing member is gel.

**17.** A computer program causing a computer to process biological signal data that is obtained by a biological signal detection sensor through a body surface, to function as a biological signal analysis device, the computer program causing the computer to execute:

a procedure that frequency-analyzes the biological signal data; and
a procedure that identifies a boundary frequency between vibration generated by cardiac apex beat and vibration generated by heart sound in the biological signal data, from a result of the frequency analysis.

**18.** The computer program according to claim 17,
wherein the procedure that identifies the boundary frequency finds, in the result of the frequency analysis, a power spectrum sudden changing point which is a boundary between harmonic vibration and random vibration, and identifies the boundary frequency based on the sudden changing point.

**19.** The computer program according to claim 18,
wherein the procedure that identifies the boundary frequency finds the power spectrum sudden changing point, in consideration of a result of a frequency analysis of heart sound data measured simultaneously.

**20.** The computer program according to claim 19,
wherein the procedure that identifies the boundary frequency represents, in log-log axes, a waveform resulting from addition averaging of the results of the frequency analyses of the biological signal data and the heart sound data, using a log difference method, finds a fluctuation changing point from the waveform represented in log-log axes, and identifies the fluctuation changing point as the power spectrum sudden changing point.

**21.** The computer program according to claim 18 or claim 19,

wherein in the frequency analyzing procedure, short time Fourier transform is employed, and
wherein the procedure that identifies the boundary frequency finds the power spectrum sudden changing point from an analysis result of the short time Fourier transform.

**22.** The computer program according to claim 21,

wherein the frequency analyzing procedure outputs the analysis result of the short time Fourier transform as image data showing time, frequency, and a degree of power spectrum variation,
and
wherein the procedure that identifies the boundary frequency finds the power spectrum sudden changing point from the image data.

**23.** The computer program according to any one of claims 17 to 22, the computer program causing the computer to execute a procedure that infers a measurement-time health condition of a measurement subject, by referring to correlation data that relates to the boundary frequency and is stored in a correlation data storage unit.

**24.** The computer program according to claim 23,

wherein the correlation data is correlation data of the boundary frequency and heart rate, and
wherein the procedure that infers the measurement-time condition infers a measurement-time boundary frequency of the measurement subject by collating a measurement-time heart rate of the measurement subject with the correlation data of the boundary frequency and the heart rate.

**25.** The computer program according to 23,

wherein the correlation data is correlation data of the boundary frequency and a fluctuation characteristic of heart rate variability, and
wherein the procedure that infers the measurement-time condition infers a measurement-time fluctuation characteristic of heart rate variability of the measurement subject by collating a measurement-time boundary frequency of the measurement subject with the correlation data of the boundary frequency and the fluctuation characteristic of the heart rate variability.

**26.** The computer program according to claim 23, further causing the computer to execute:

a procedure that filters the biological signal data, with an upper limit value being set to the boundary frequency identified by the boundary frequency identifying means, to find a waveform of the vibration generated by the cardiac apex beat; and

a procedure that classifies the waveform of the vibration generated by the cardiac apex beat,
wherein the procedure that infers the measurement-time condition causes the procedure that classifies the waveform to classify a measurement-time waveform of the vibration generated by the cardiac apex beat of the measurement subject, and infers the measurement-time health condition of the measurement subject based on a result of the classification of the measurement-time waveform.

**27.** The computer program according to claim 26,

wherein correlation data of a result of the waveform classification and health condition is stored in the correlation data storage unit, and
wherein the procedure that infers the measurement-time condition causes the procedure that classifies the waveform to classify the measurement-time waveform of the vibration generated by the cardiac apex beat of the measurement subject, and infers the measurement-time health condition of the measurement subject by collating the result of the classification of the measurement-time waveform with the correlation data of the waveform classification result and the health condition.

**28.** The computer program according to claim 26,

wherein information on waveforms and the health conditions are used as training data, the waveforms being output by the execution of the procedure that extracts the cardiac apex beat waveform to be classified by the procedure that classifies the waveform, and an inference model for inferring the health condition is created from the waveform information by machine learning, and
wherein the procedure that infers the measurement-time condition receives the information of the measurement-time waveform of the vibration generated by the cardiac apex beat of the measurement subject, and outputs a value of the measurement-time health condition based on the obtained information of the measurement-time waveform, using the inference model.

**29.** The computer program according to any one of claims 26 to 28,
wherein the procedure that classifies the waveform uses the following procedures (A) and (B):

(A) a procedure that classifies the waveform by a mathematical approach using Fourier series expansion; and
(B) a procedure that classifies the waveform based on a combination of one or two or more of the following data (1) to (4) obtained by engineering approaches:

(1) data indicating a shape of a time waveform in a predetermined time range of the waveform of the vibration generated by the cardiac apex beat;
(2) data of a graph of the time waveform, in which frequency and power spectrum are taken on a horizontal axis and a vertical axis, the data being obtained by the frequency analyzing means;
(3) image data in a predetermined time range which is the analysis result of the short time Fourier transform of the time waveform and shows the time, the frequency, and the degree of the power spectrum variation; and
(4) data regarding a type and a period of a waveform, in a predetermined time range, which data is extracted from a correlogram,

to classify waveforms of vibrations generated by the cardiac apex beat of healthy people into five, and
infers whether the measurement-time health condition of the measurement subject is a healthy condition or not based on whether or not the result of the classification of the measurement-time waveform of the measurement subject corresponds to any one of the five results of the waveform classification.

**30.** The computer program according to claim 29, wherein the image data is divided based on a plurality of frequencies, and a plurality of image data are created in the predetermined time range.

**31.** A recording medium in which the computer program according to any one of claims 17 to 30 is recorded.

FIG. 1

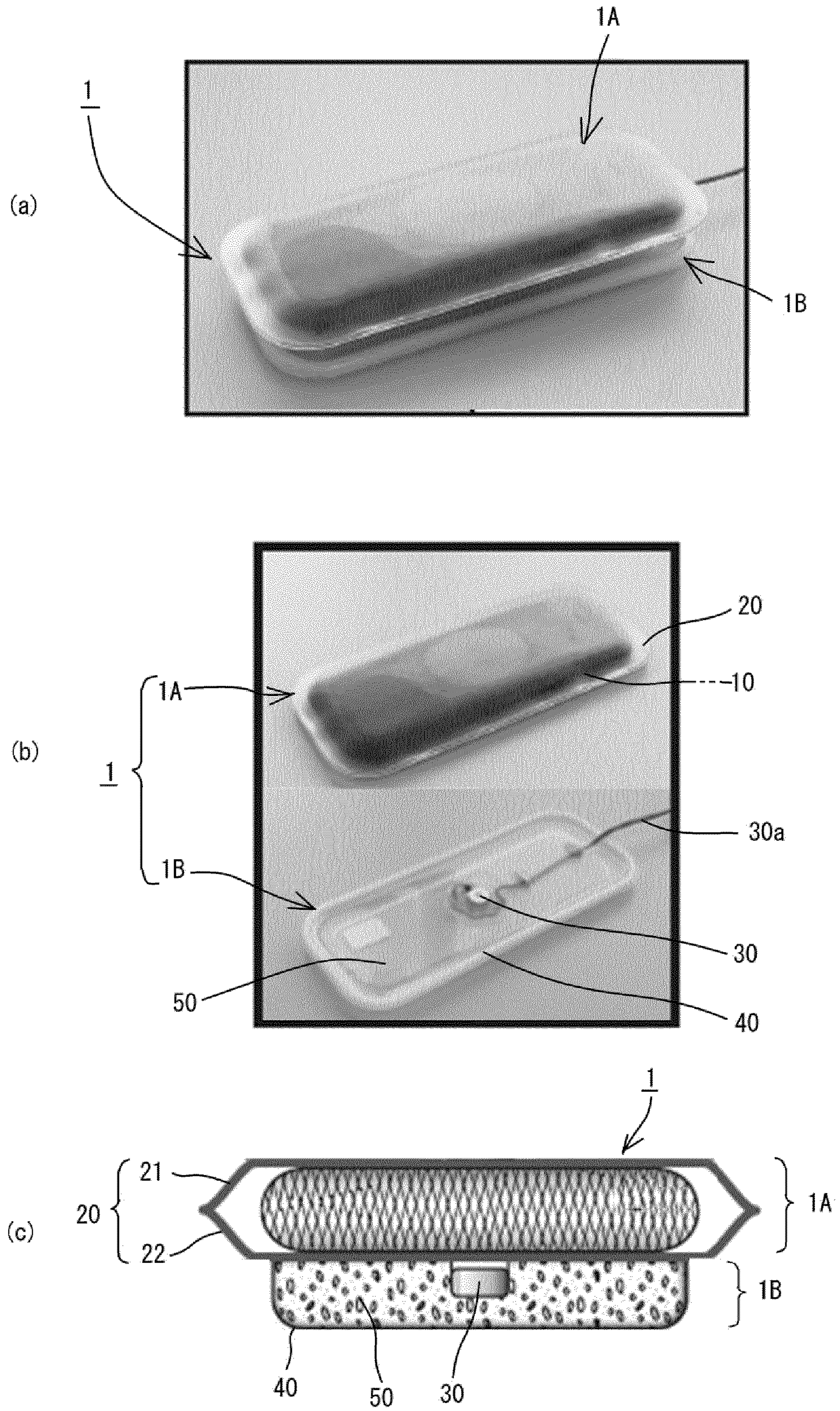

FIG. 2

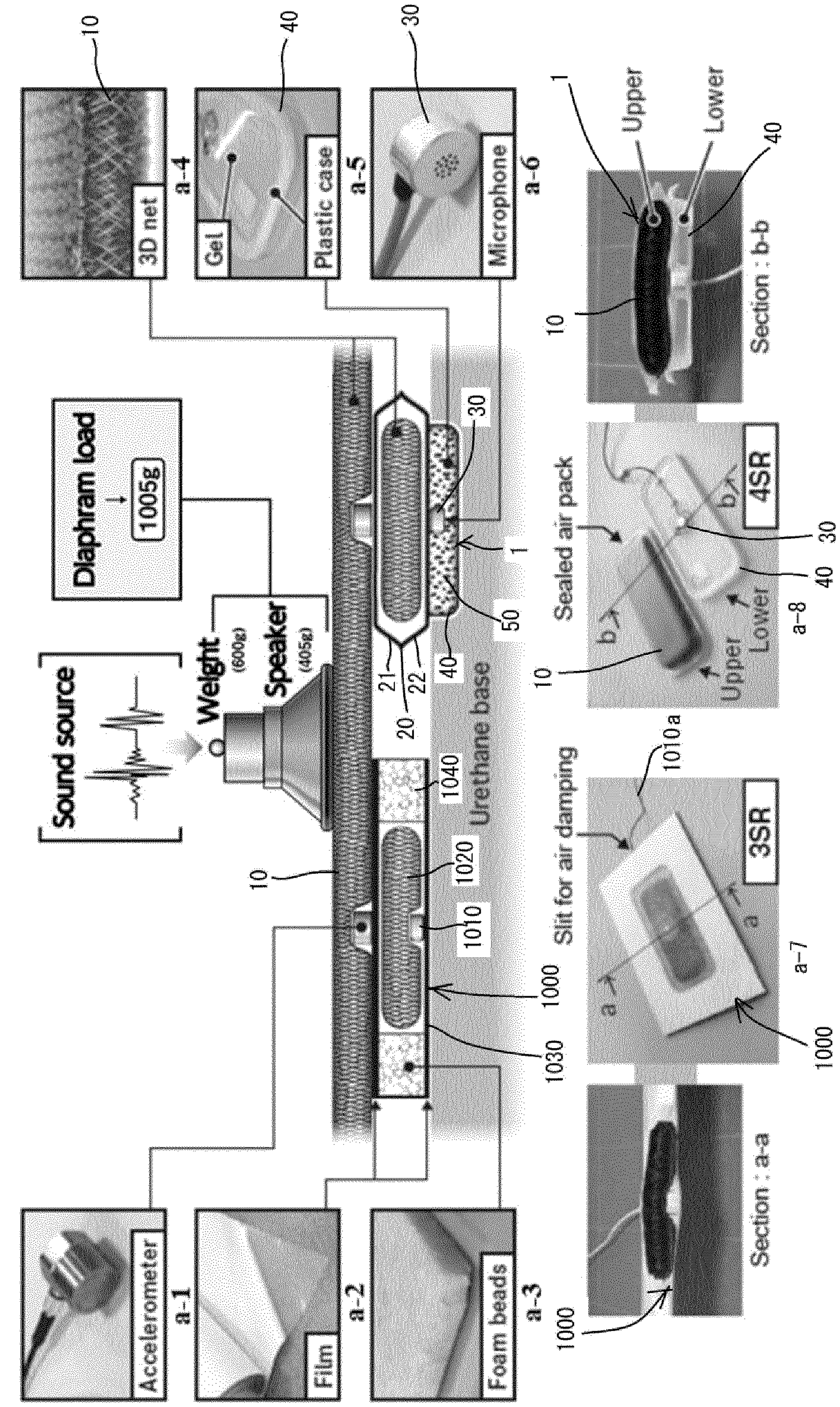
Sound source
Weight (600g)
Speaker (405g)
Diaphram load
1005g
Accelerometer
a-1
Film
a-2
Foam beads
a-3
3D net
a-4
Gel
Plastic case
a-5
Microphone
a-6
Urethane base
Slit for air damping
3SR
a-7
Sealed air pack
Upper
Lower
4SR
a-8
Section : a-a
Section : b-b
1
10
20
21
22
30
40
50
1000
1010
1010a
1020
1030
1040

FIG. 3

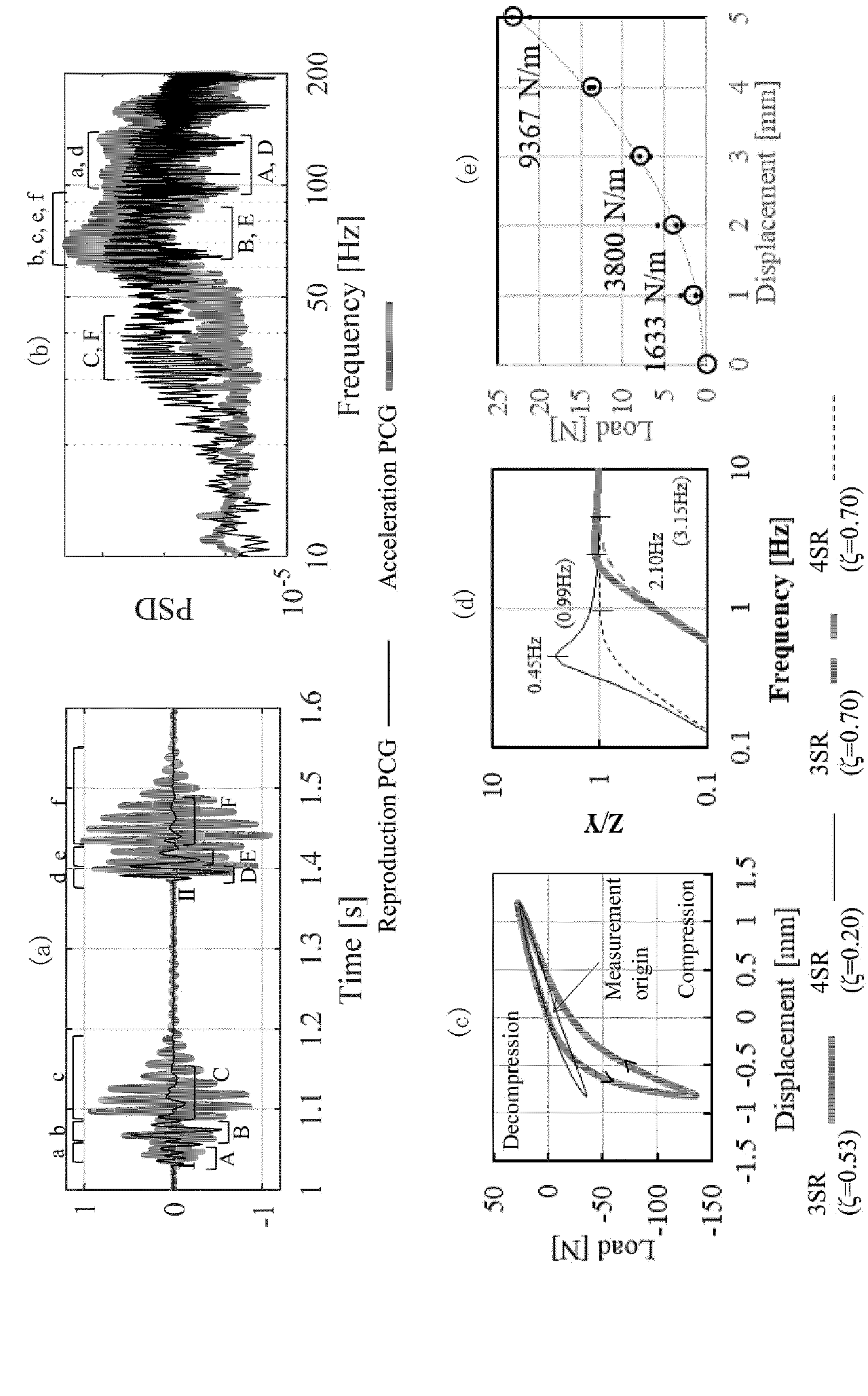

(a)
(b)
(c)
(d)
(e)
Time [s]
Frequency [Hz]
PSD
Reproduction PCG
Acceleration PCG
Load [N]
Displacement [mm]
Decompression
Measurement origin
Compression
Z/Y
Frequency [Hz]
0.45Hz
(0.99Hz)
2.10Hz
(3.15Hz)
3SR (ζ=0.53)
4SR (ζ=0.20)
3SR (ζ=0.70)
4SR (ζ=0.70)
1633 N/m
3800 N/m
9367 N/m

FIG. 4

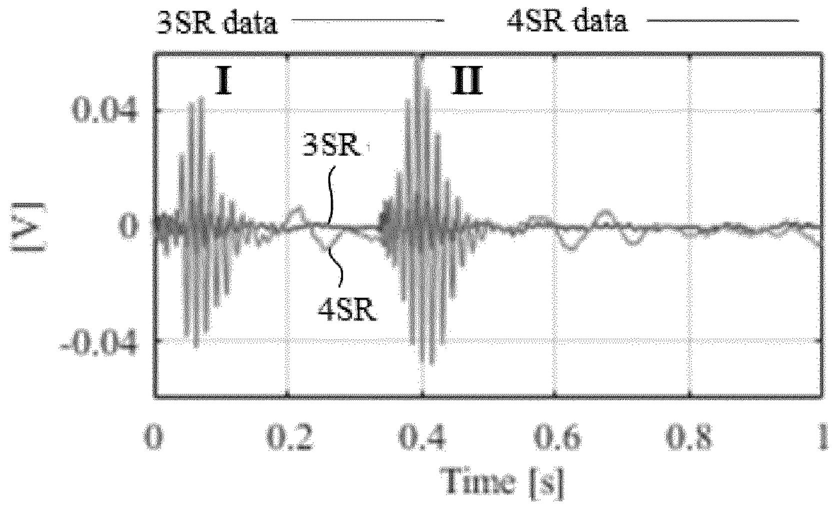


(a)Input: PCG

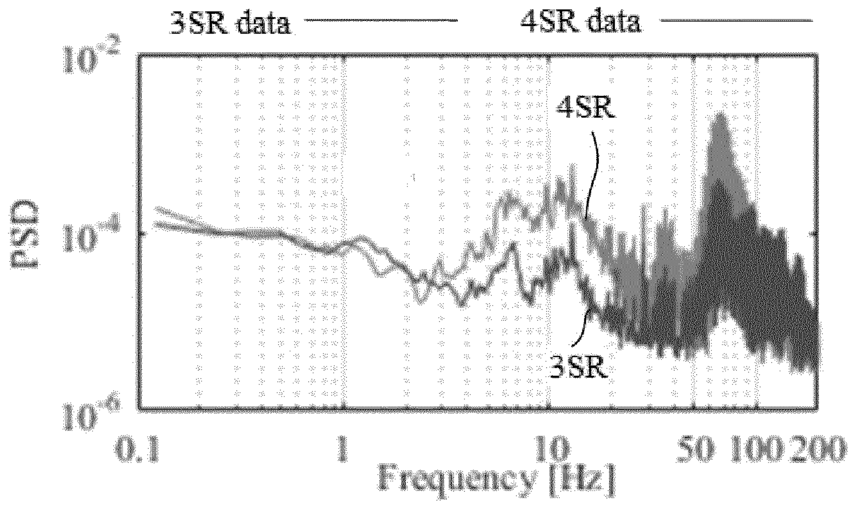


(b) Input: PCG

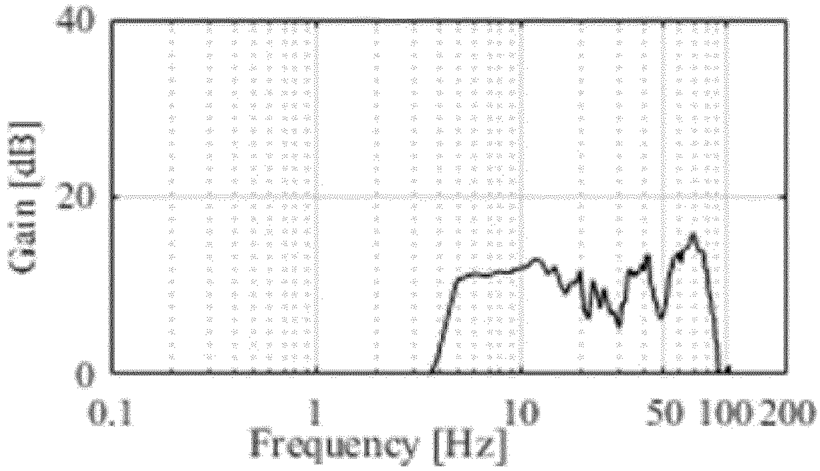


(c) Input: PCG

FIG. 5

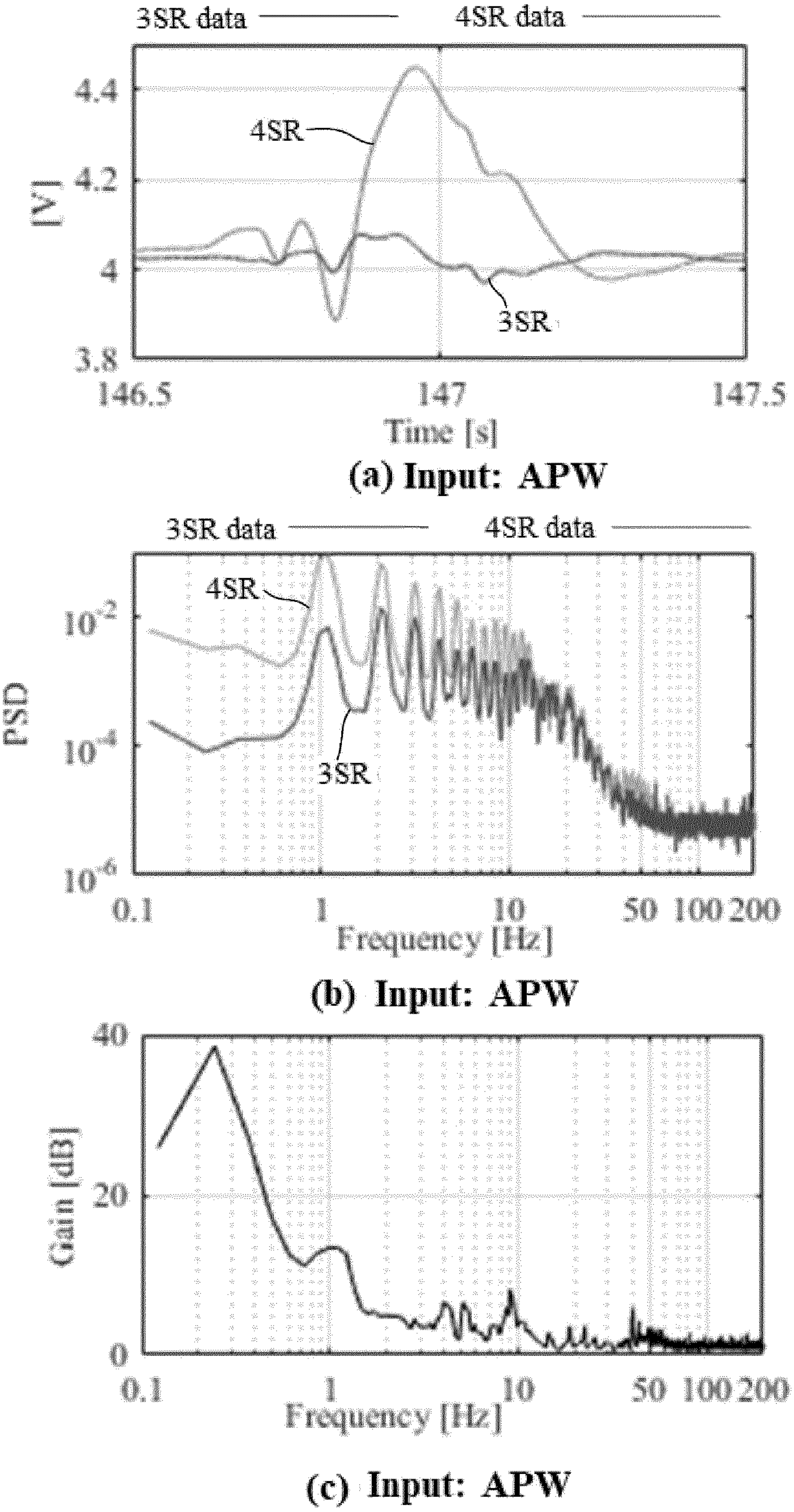
3SR data
4SR data
4.4
4.2
4
3.8
[V]
4SR
3SR
146.5
147
147.5
Time [s]
(a) Input: APW
3SR data
4SR data
$10^{-2}$
$10^{-4}$
$10^{-6}$
PSD
4SR
3SR
0.1
1
10
50
100
200
Frequency [Hz]
(b) Input: APW
40
20
0
Gain [dB]
0.1
1
10
50
100
200
Frequency [Hz]
(c) Input: APW

FIG. 6

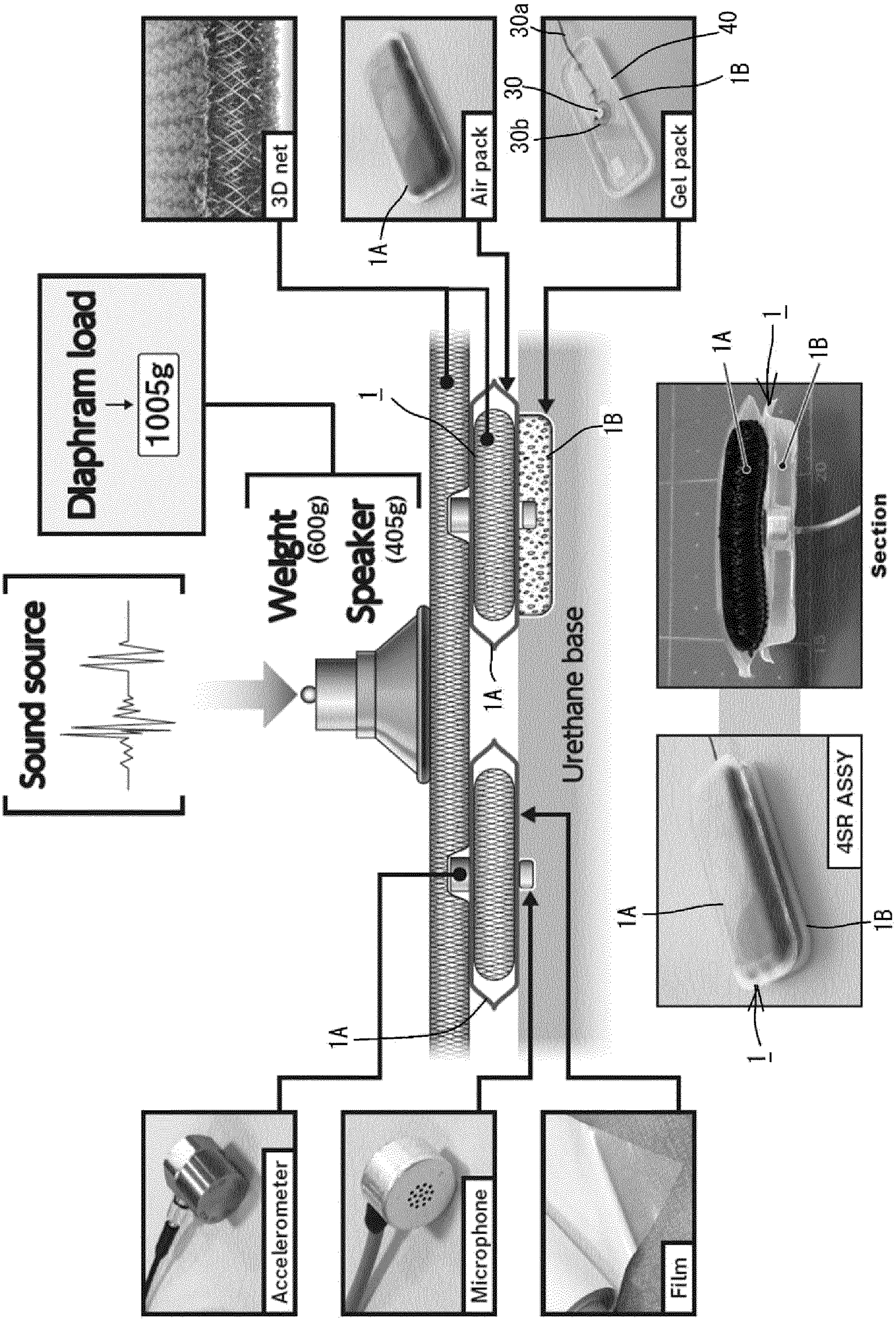
Sound source
Diaphram load
1005g
Weight
(600g)
Speaker
(405g)
3D net
Air pack
Gel pack
30a
30
30b
40
1B
1A
1
Urethane base
Section
4SR ASSY
Accelerometer
Microphone
Film

FIG. 7

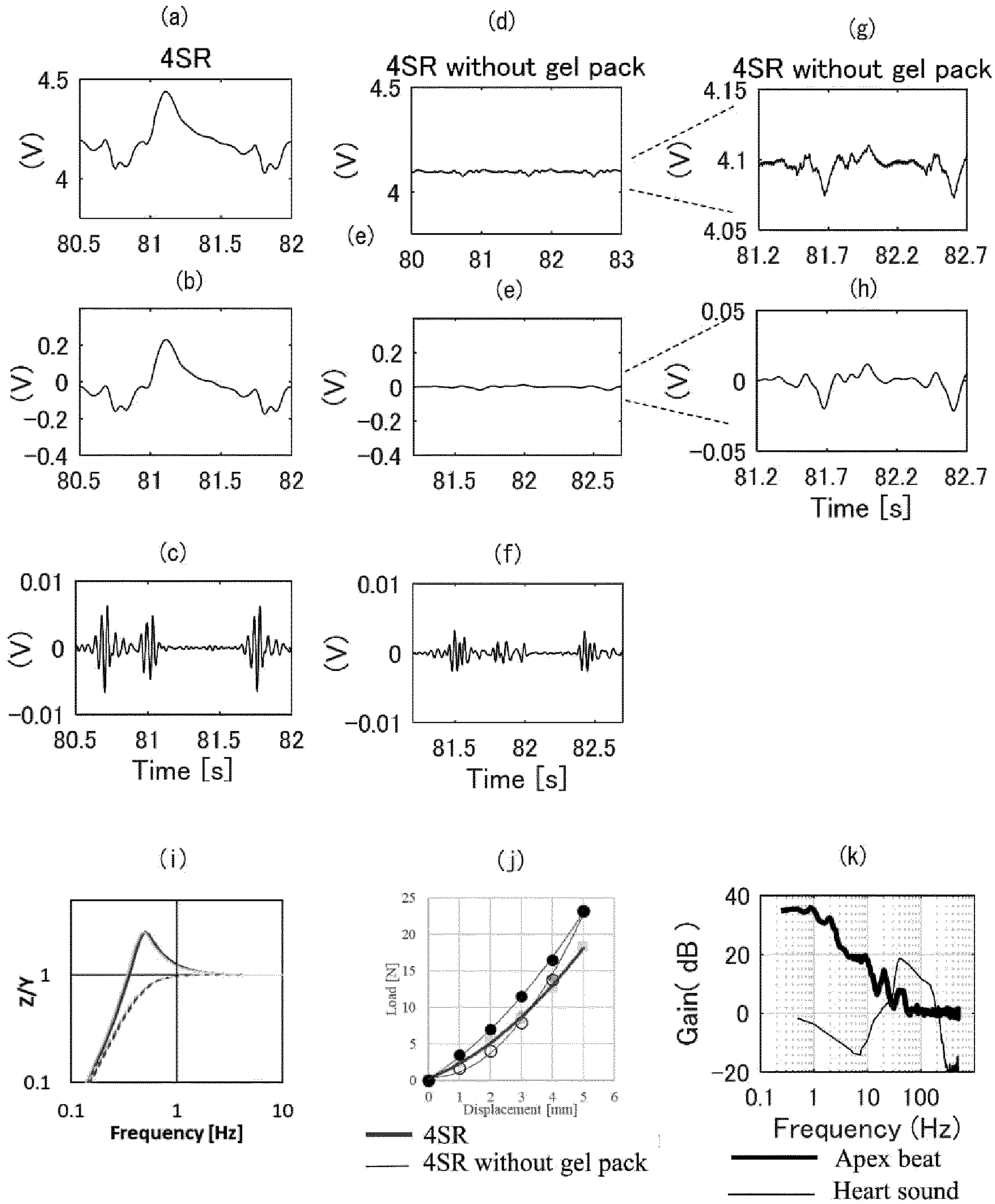
(a)
4SR
(d)
4SR without gel pack
(g)
4SR without gel pack
(b)
(e)
(h)
(c)
(f)
Time [s]
(i)
Z/Y
Frequency [Hz]
(j)
Load [N]
Displacement [mm]
4SR
4SR without gel pack
(k)
Gain( dB )
Frequency (Hz)
Apex beat
Heart sound

FIG. 8

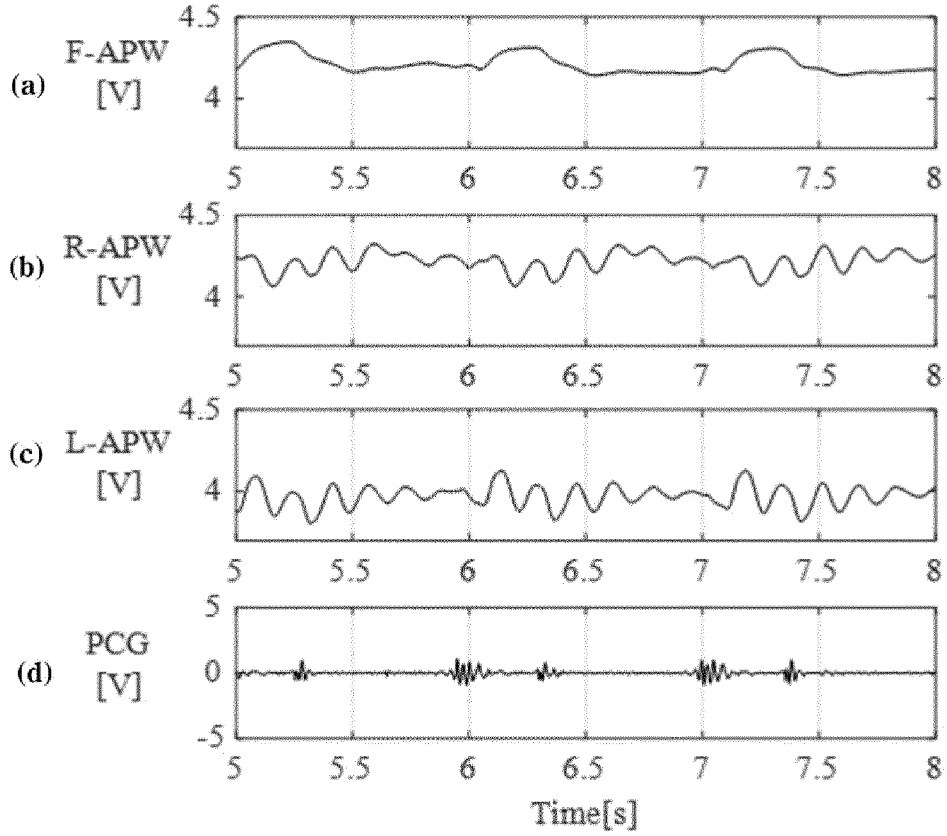


Example of HR 58/min

FIG. 9

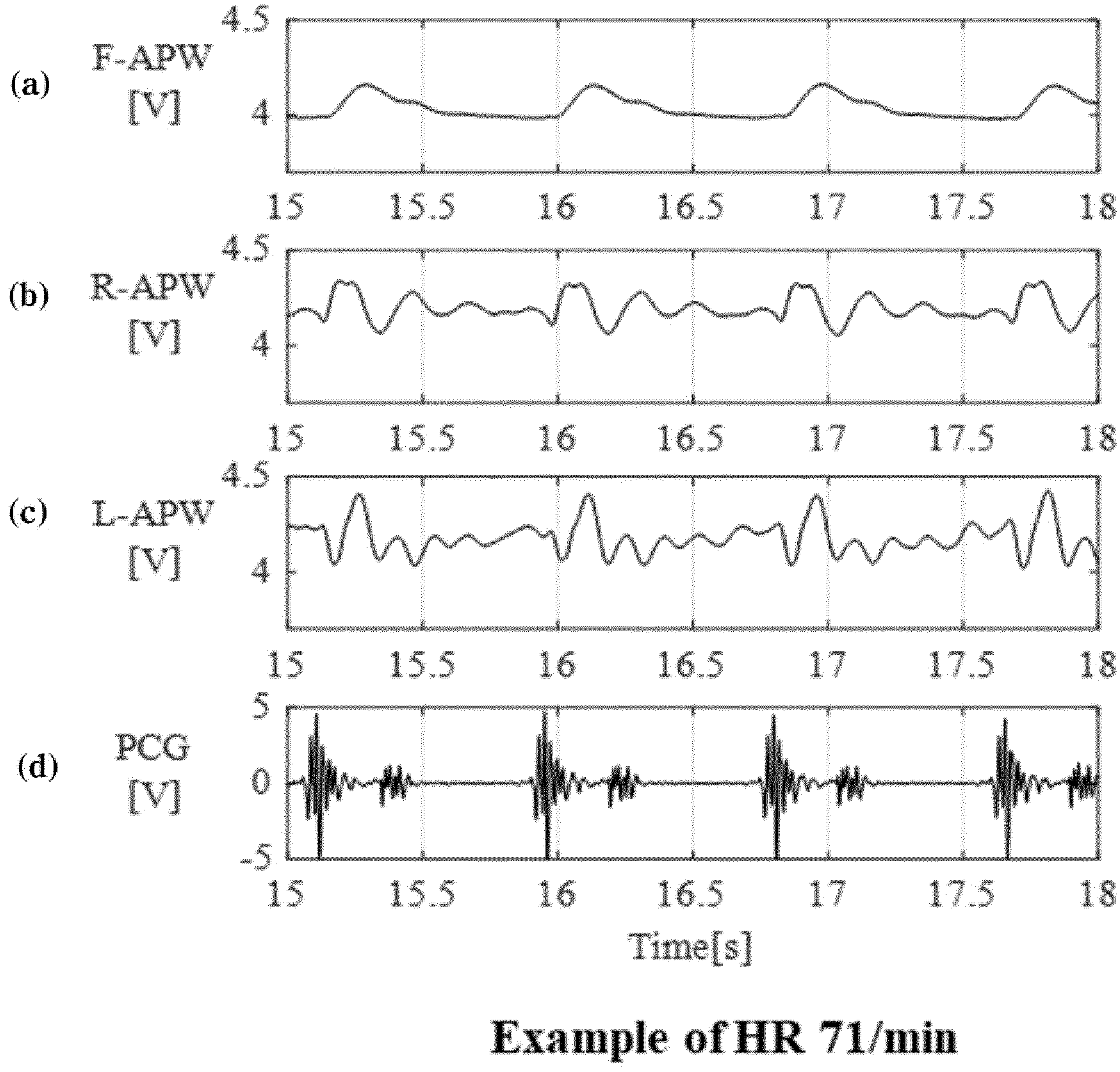
(a)
F-APW
[V]
4.5
4
(b)
R-APW
[V]
4.5
4
(c)
L-APW
[V]
4.5
4
(d)
PCG
[V]
5
0
-5
15
15.5
16
16.5
17
17.5
18
Time[s]
Example of HR 71/min

FIG. 10

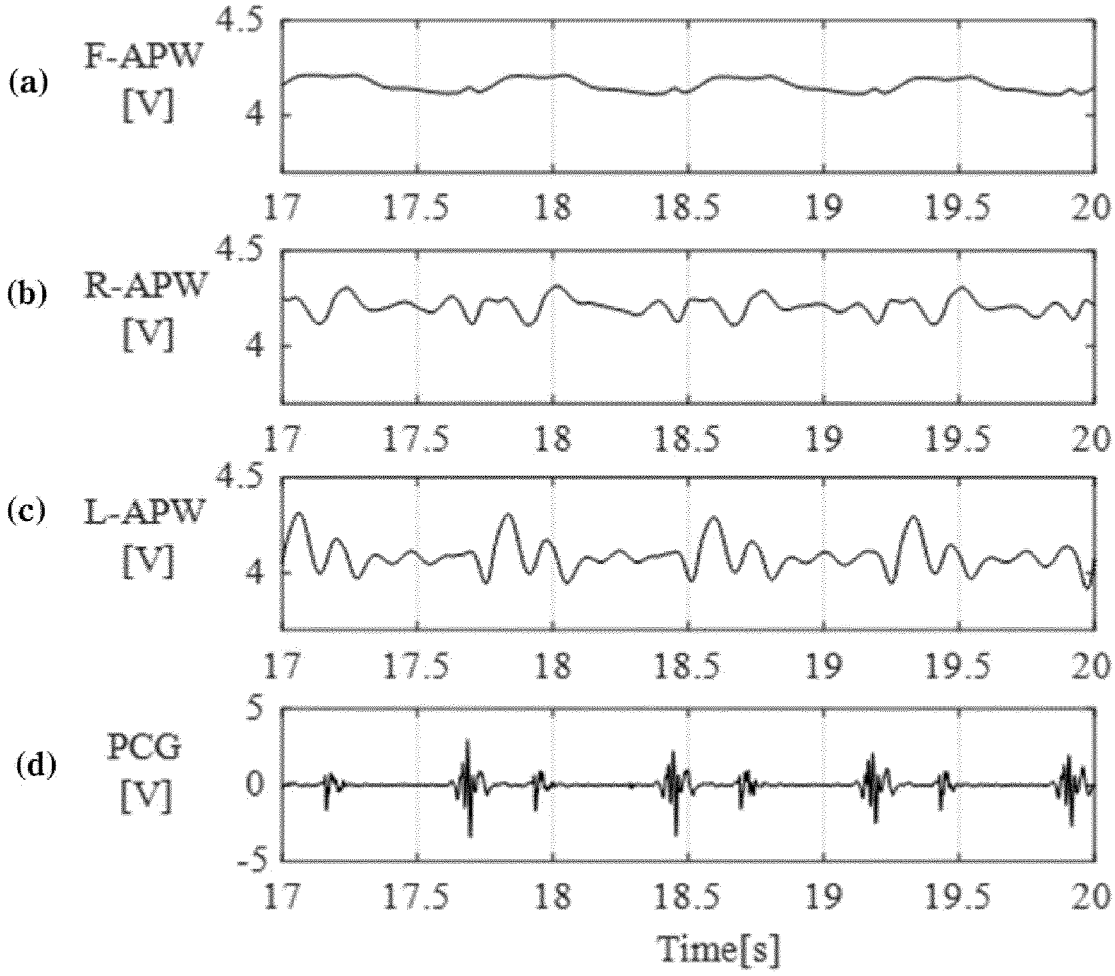


Example of HR 79/min

FIG. 11

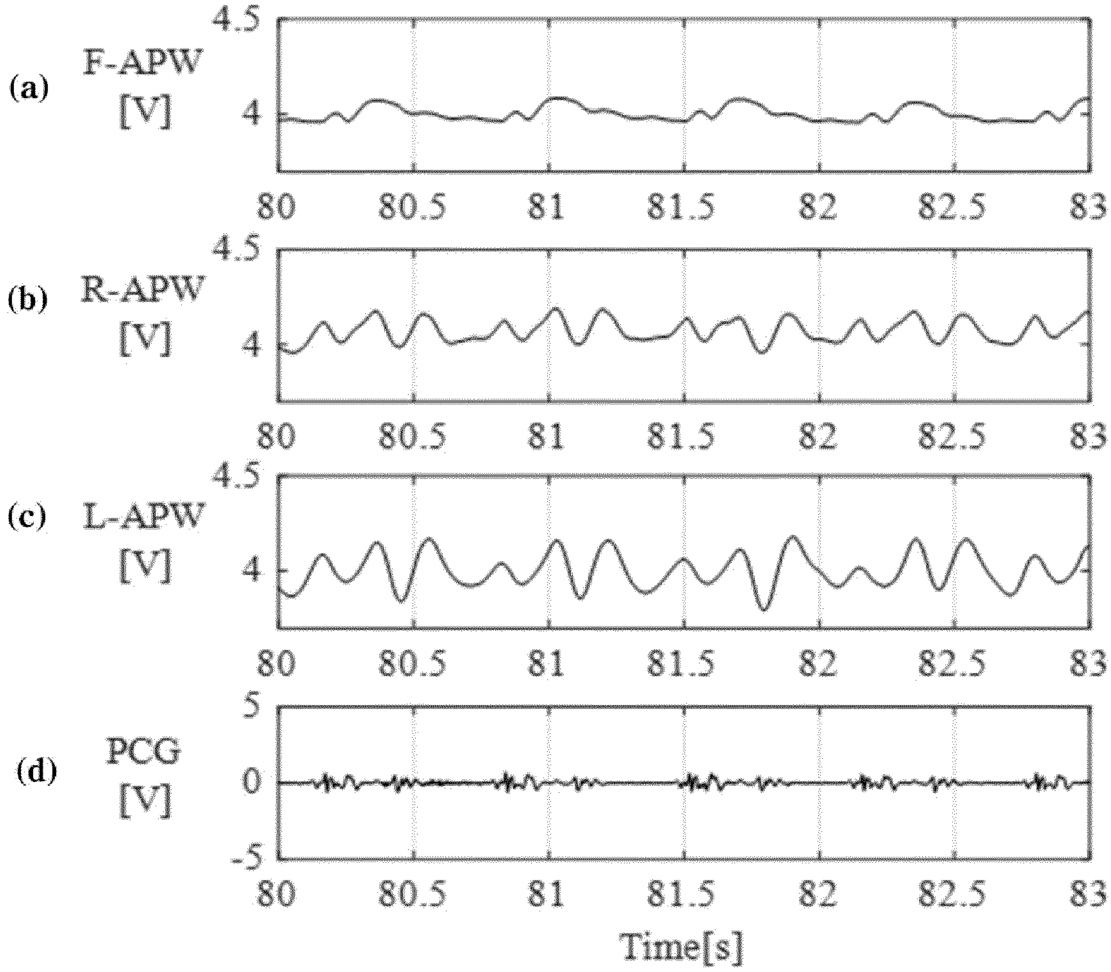


Example of HR 93/min

FIG. 12

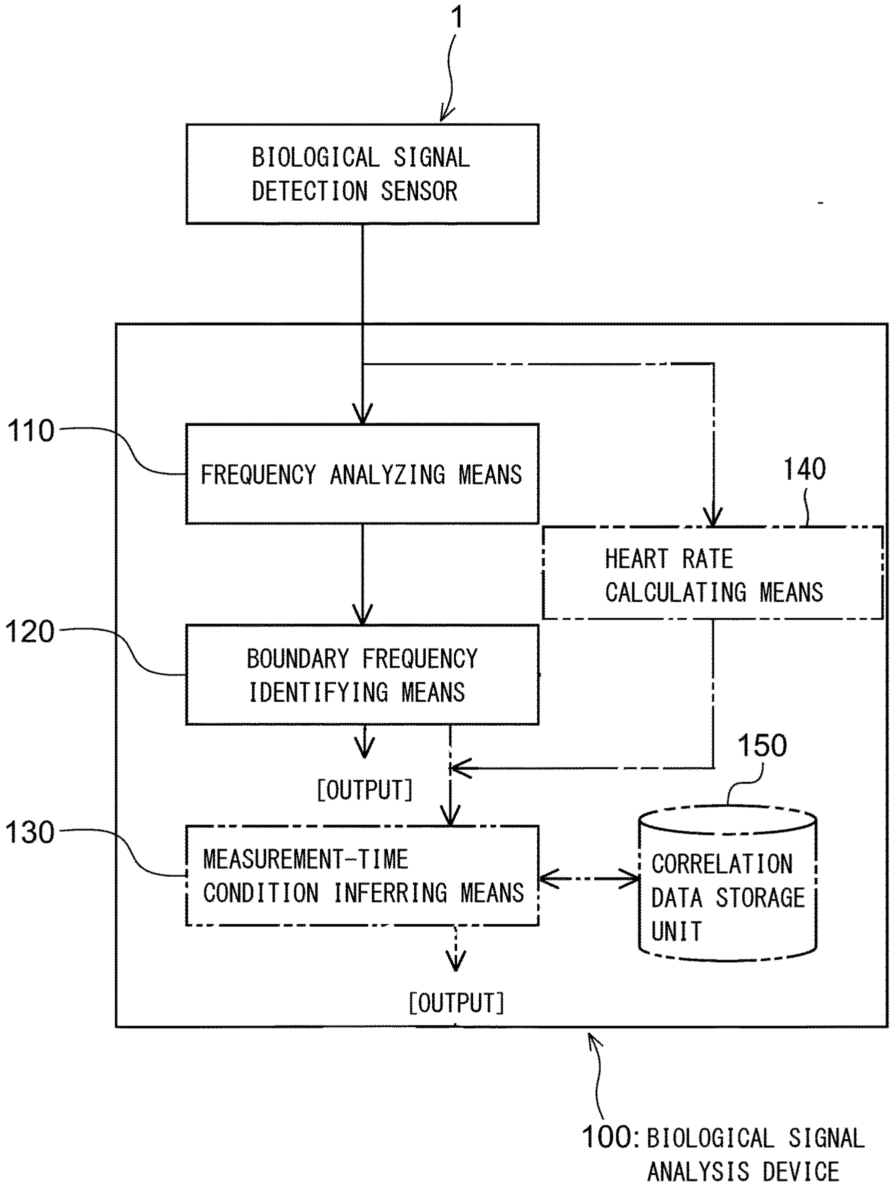
1
BIOLOGICAL SIGNAL DETECTION SENSOR
110
FREQUENCY ANALYZING MEANS
140
HEART RATE CALCULATING MEANS
120
BOUNDARY FREQUENCY IDENTIFYING MEANS
[OUTPUT]
150
130
MEASUREMENT-TIME CONDITION INFERRING MEANS
CORRELATION DATA STORAGE UNIT
[OUTPUT]
100: BIOLOGICAL SIGNAL ANALYSIS DEVICE

FIG. 13

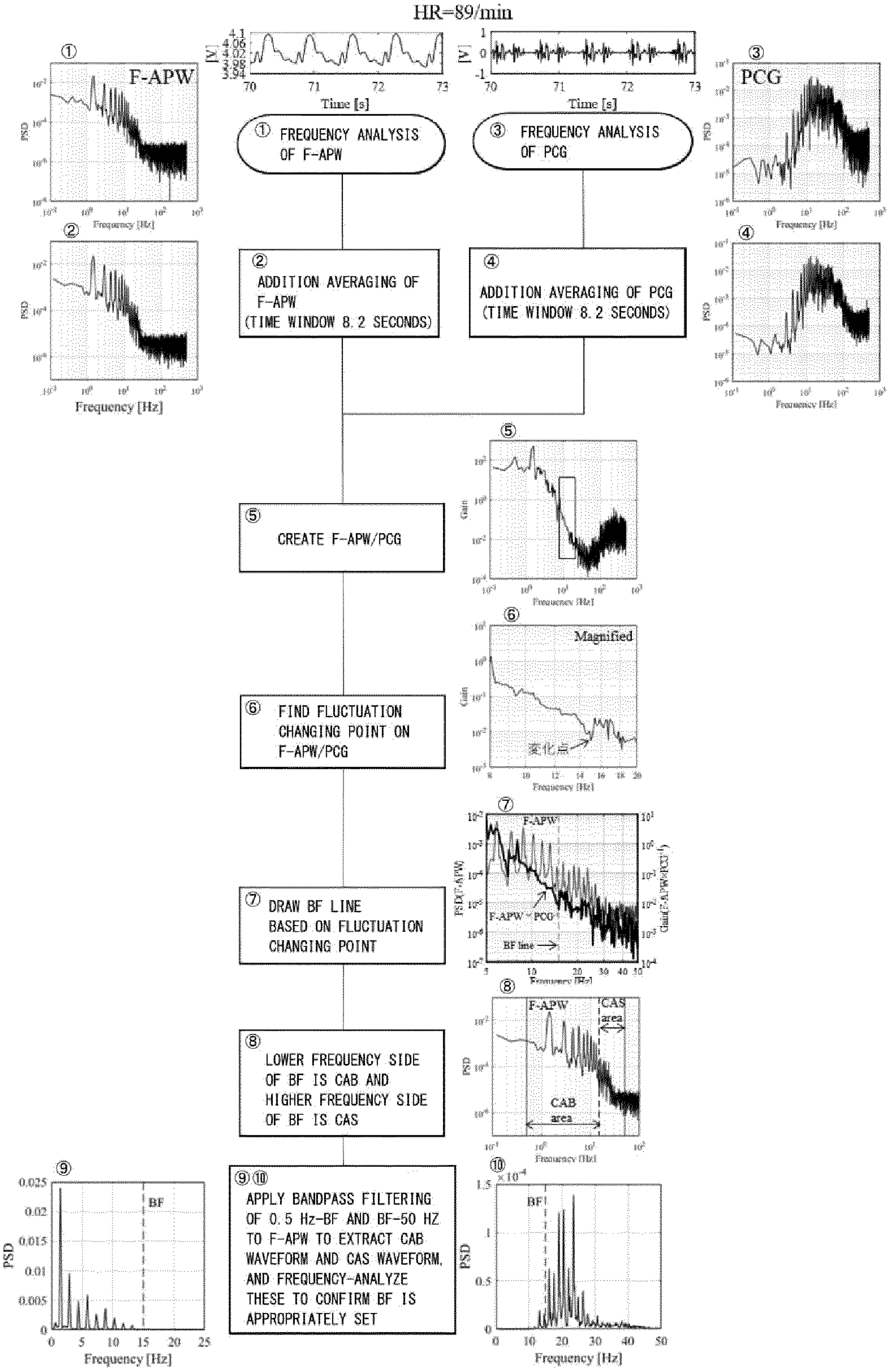
HR=89/min
F-APW
PCG
Time [s]
Frequency [Hz]
① FREQUENCY ANALYSIS OF F-APW
③ FREQUENCY ANALYSIS OF PCG
② ADDITION AVERAGING OF F-APW (TIME WINDOW 8.2 SECONDS)
④ ADDITION AVERAGING OF PCG (TIME WINDOW 8.2 SECONDS)
⑤ CREATE F-APW/PCG
⑥ FIND FLUCTUATION CHANGING POINT ON F-APW/PCG
Magnified
変化点
⑦ DRAW BF LINE BASED ON FLUCTUATION CHANGING POINT
F-APW/PCG
BF line
⑧ LOWER FREQUENCY SIDE OF BF IS CAB AND HIGHER FREQUENCY SIDE OF BF IS CAS
CAS area
CAB area
⑨⑩ APPLY BANDPASS FILTERING OF 0.5 Hz-BF AND BF-50 HZ TO F-APW TO EXTRACT CAB WAVEFORM AND CAS WAVEFORM, AND FREQUENCY-ANALYZE THESE TO CONFIRM BF IS APPROPRIATELY SET
BF
PSD
Gain

FIG. 14

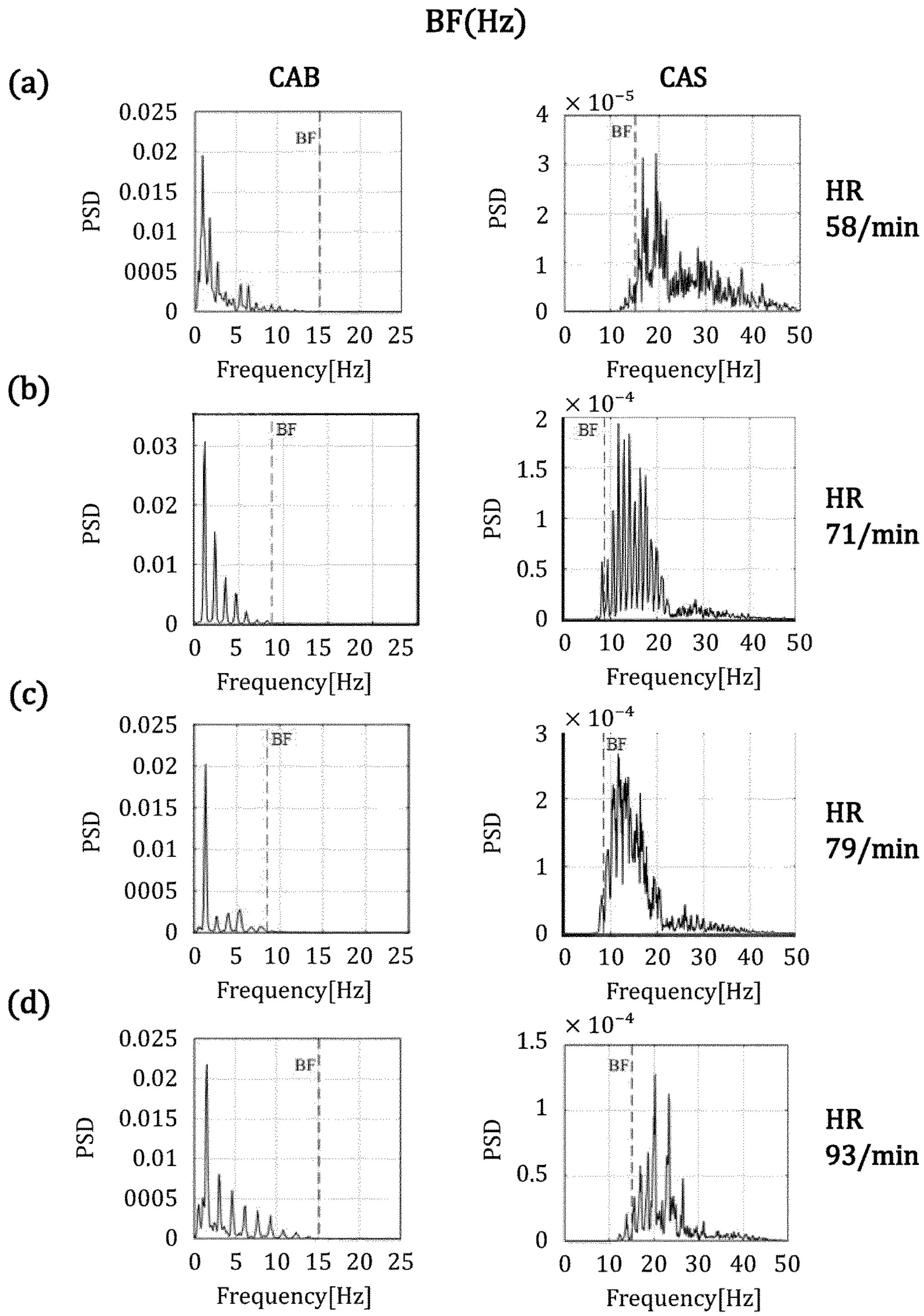
BF(Hz)
(a)
CAB
CAS
HR
58/min
(b)
HR
71/min
(c)
HR
79/min
(d)
HR
93/min
PSD
Frequency[Hz]
BF

FIG. 15

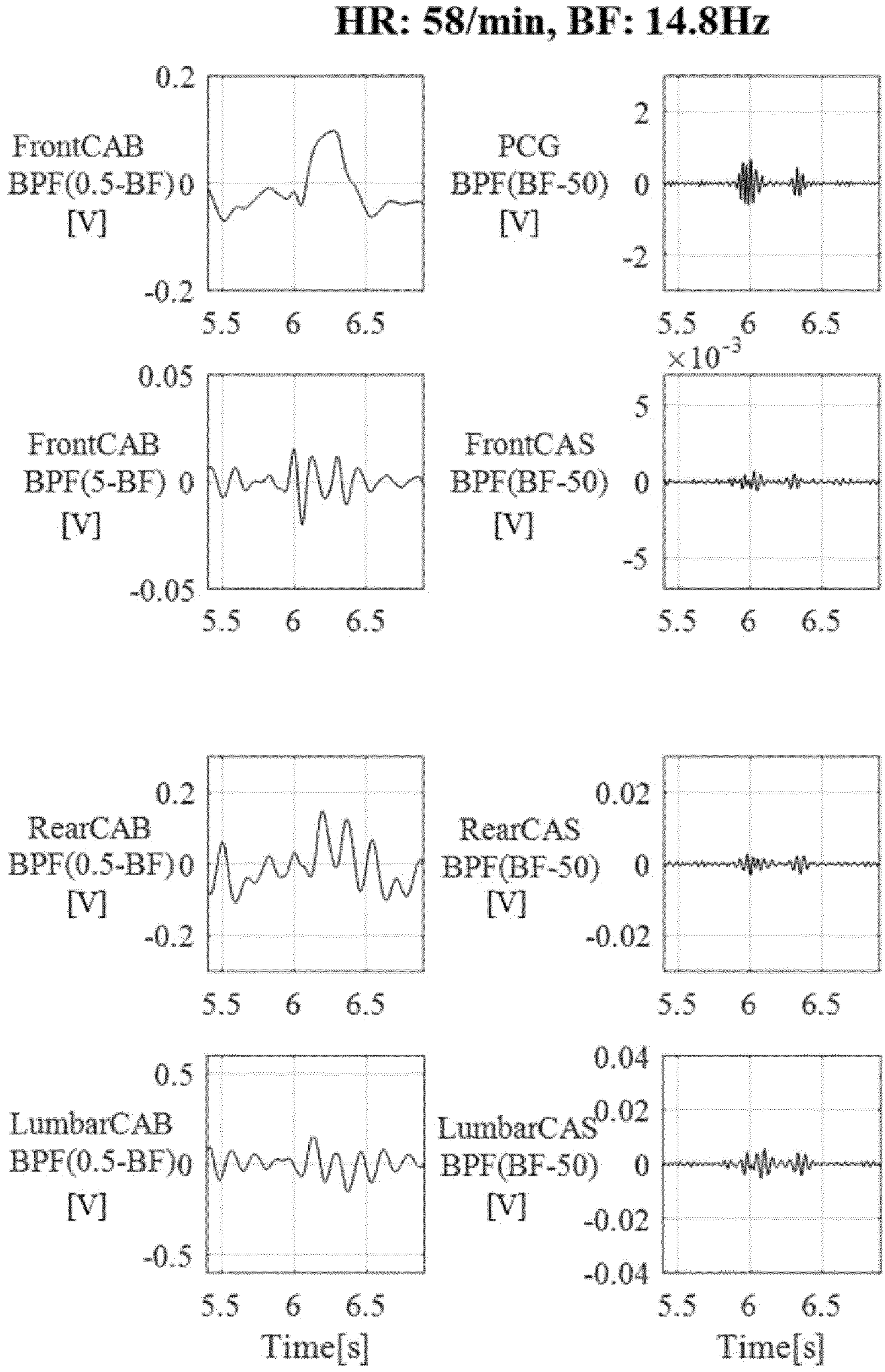
HR: 58/min, BF: 14.8Hz
FrontCAB
BPF(0.5-BF)
[V]
0.2
0
-0.2
PCG
BPF(BF-50)
[V]
2
0
-2
FrontCAB
BPF(5-BF)
[V]
0.05
0
-0.05
×10^-3
FrontCAS
BPF(BF-50)
[V]
5
0
-5
RearCAB
BPF(0.5-BF)
[V]
0.2
0
-0.2
RearCAS
BPF(BF-50)
[V]
0.02
0
-0.02
LumbarCAB
BPF(0.5-BF)
[V]
0.5
0
-0.5
LumbarCAS
BPF(BF-50)
[V]
0.04
0.02
0
-0.02
-0.04
5.5
6
6.5
Time[s]

FIG. 16

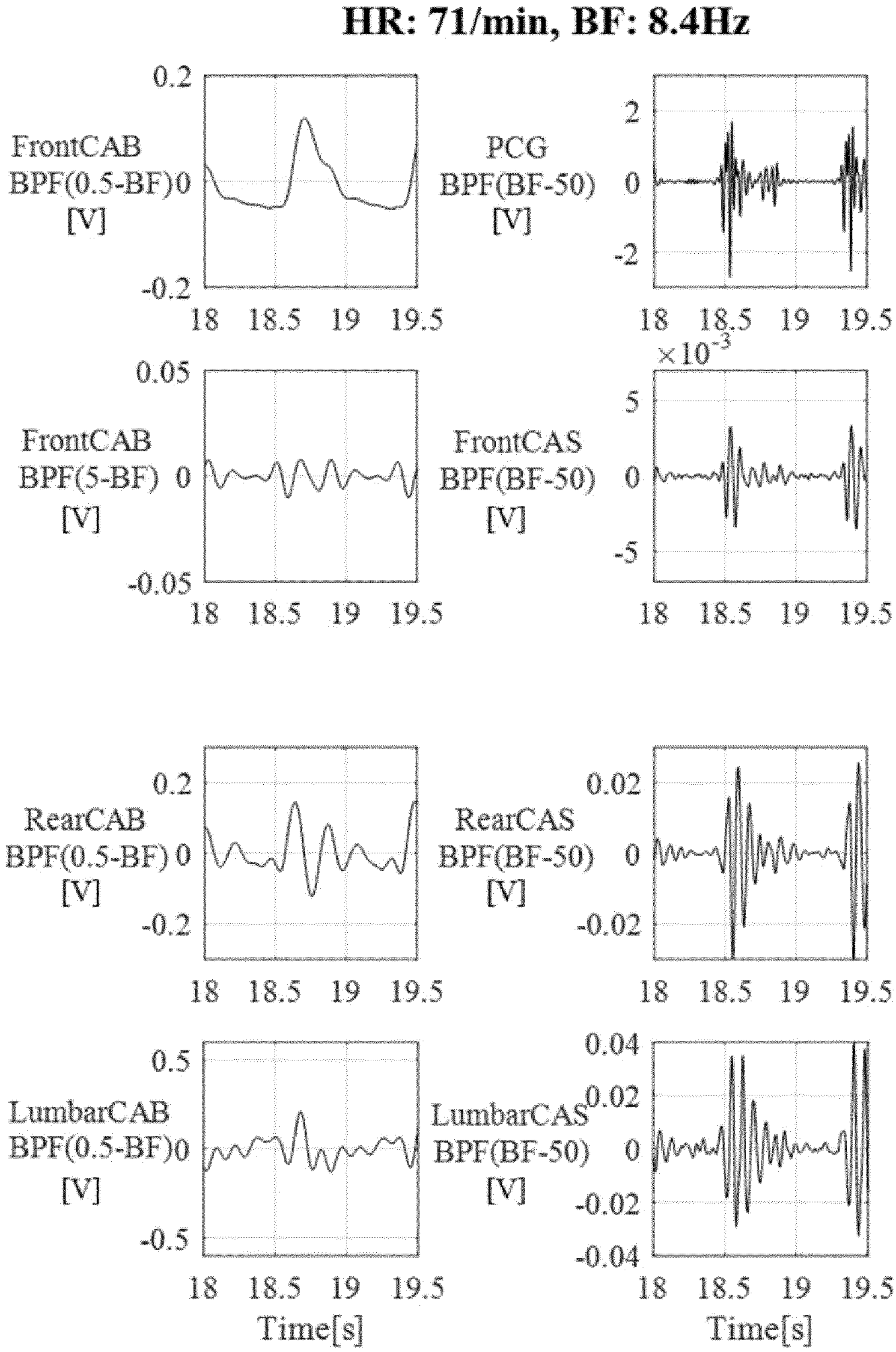
HR: 71/min, BF: 8.4Hz
FrontCAB BPF(0.5-BF) [V]
PCG BPF(BF-50) [V]
FrontCAB BPF(5-BF) [V]
FrontCAS BPF(BF-50) [V]
×10^-3
RearCAB BPF(0.5-BF) [V]
RearCAS BPF(BF-50) [V]
LumbarCAB BPF(0.5-BF) [V]
LumbarCAS BPF(BF-50) [V]
Time[s]
Time[s]

FIG. 17

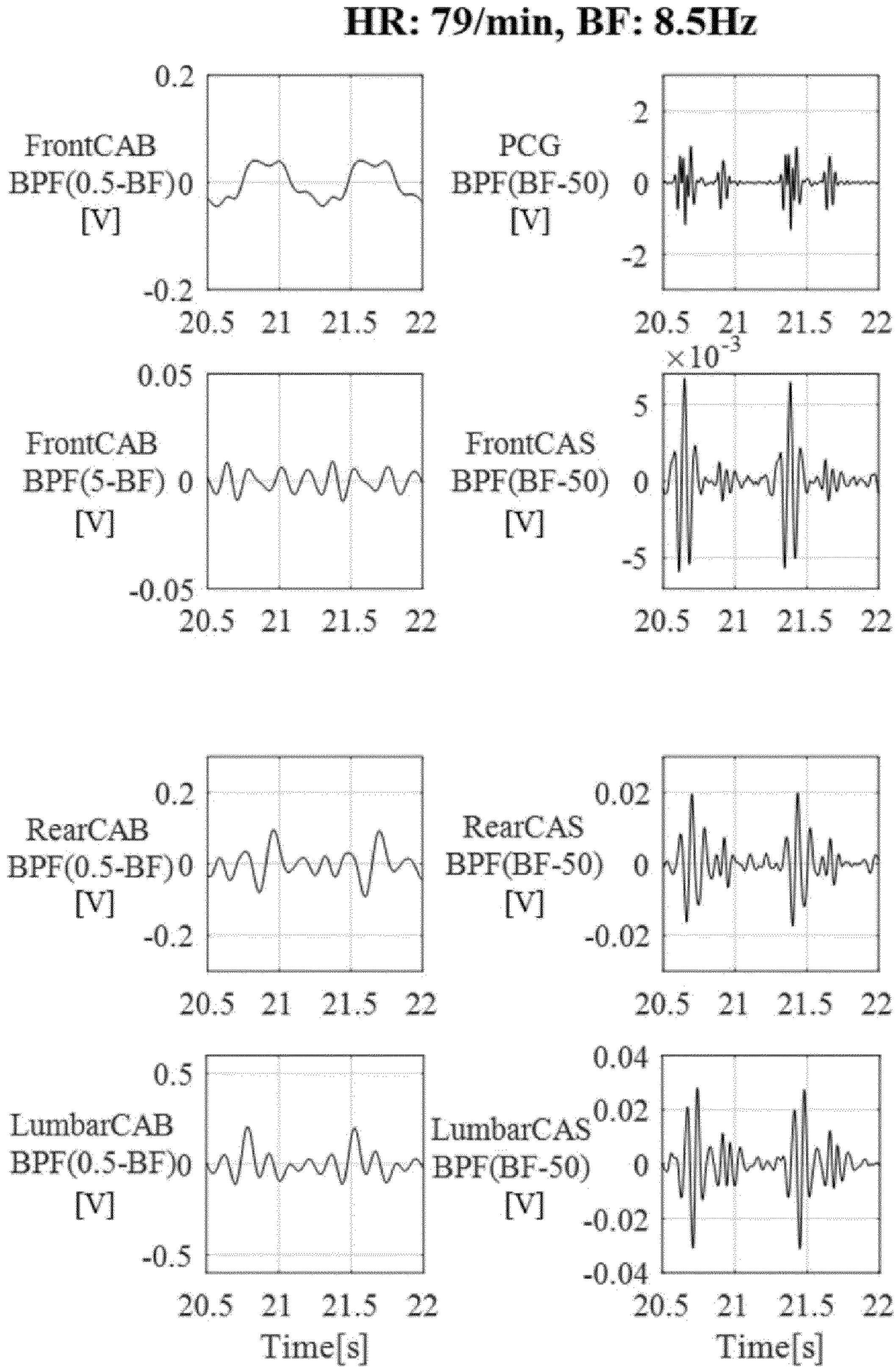
HR: 79/min, BF: 8.5Hz
FrontCAB BPF(0.5-BF) [V]
0.2
0
-0.2
20.5 21 21.5 22
PCG BPF(BF-50) [V]
2
0
-2
20.5 21 21.5 22
FrontCAB BPF(5-BF) [V]
0.05
0
-0.05
20.5 21 21.5 22
FrontCAS BPF(BF-50) [V]
×10^-3
5
0
-5
20.5 21 21.5 22
RearCAB BPF(0.5-BF) [V]
0.2
0
-0.2
20.5 21 21.5 22
RearCAS BPF(BF-50) [V]
0.02
0
-0.02
20.5 21 21.5 22
LumbarCAB BPF(0.5-BF) [V]
0.5
0
-0.5
20.5 21 21.5 22
Time[s]
LumbarCAS BPF(BF-50) [V]
0.04
0.02
0
-0.02
-0.04
20.5 21 21.5 22
Time[s]

FIG. 18

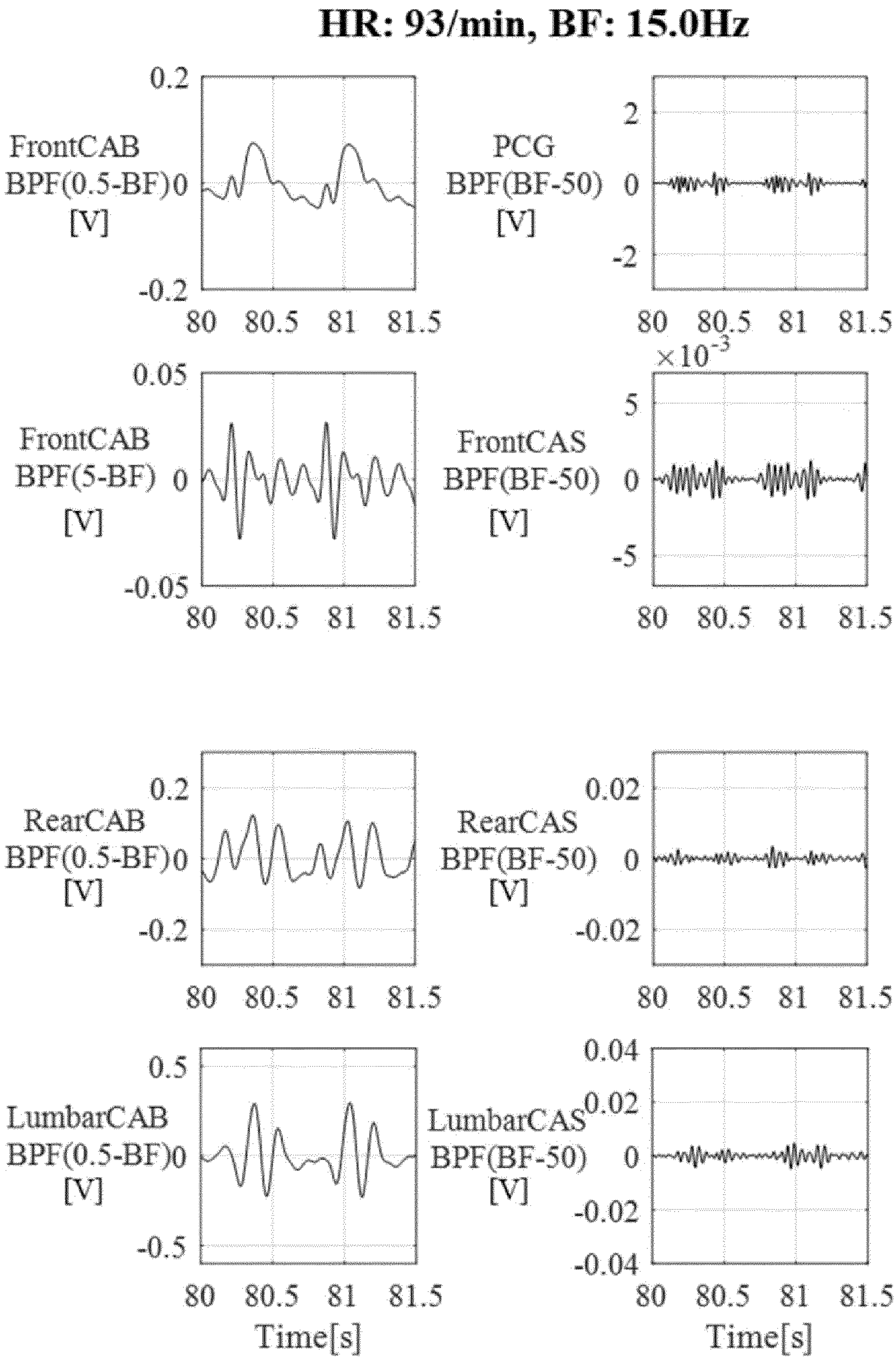
HR: 93/min, BF: 15.0Hz
FrontCAB BPF(0.5-BF) [V]
PCG BPF(BF-50) [V]
FrontCAB BPF(5-BF) [V]
FrontCAS BPF(BF-50) [V]
×10^-3
RearCAB BPF(0.5-BF) [V]
RearCAS BPF(BF-50) [V]
LumbarCAB BPF(0.5-BF) [V]
LumbarCAS BPF(BF-50) [V]
Time[s]
Time[s]

FIG. 19

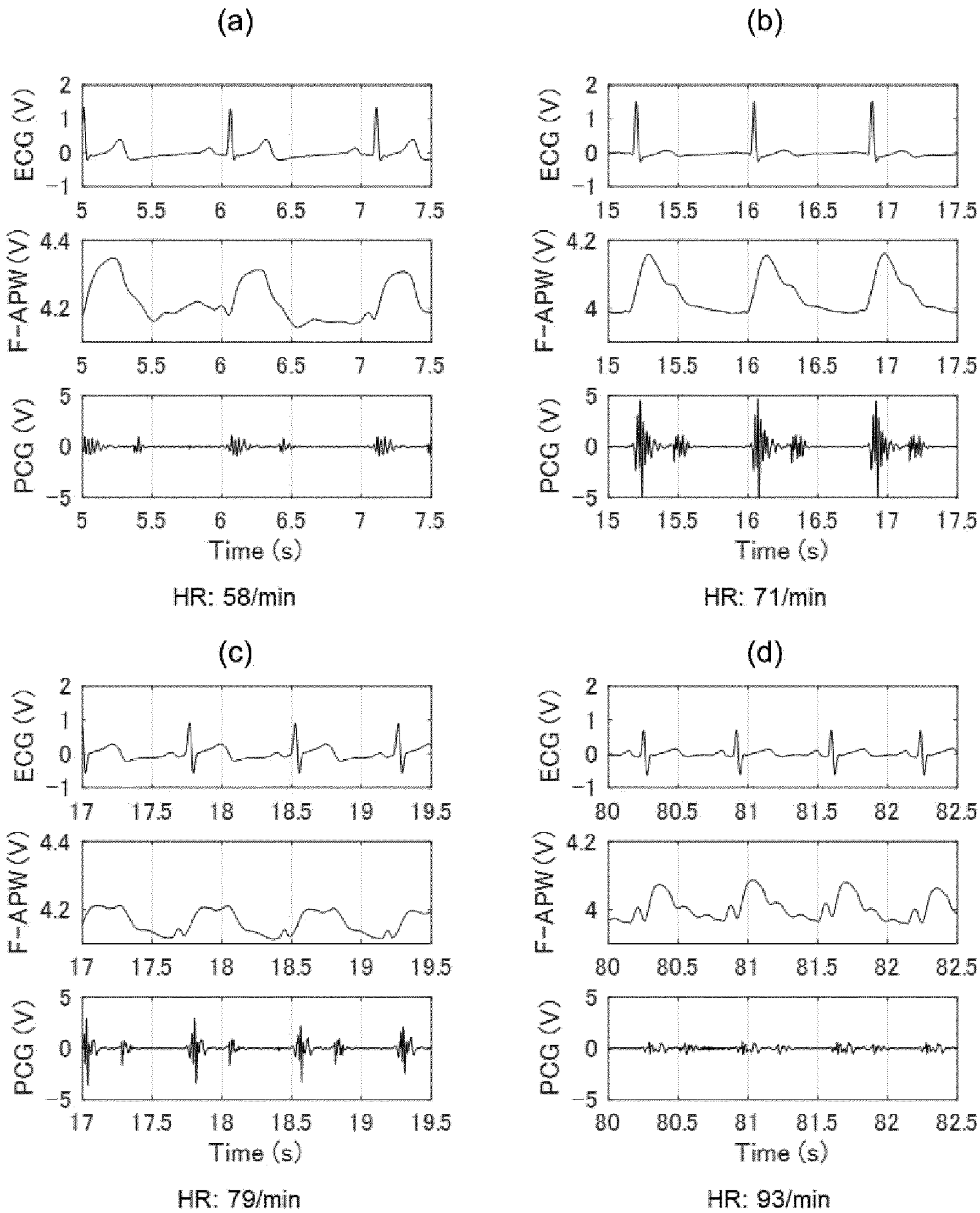
(a)
ECG (V)
F-APW (V)
PCG (V)
Time (s)
HR: 58/min
(b)
HR: 71/min
(c)
HR: 79/min
(d)
HR: 93/min

Fig. 20

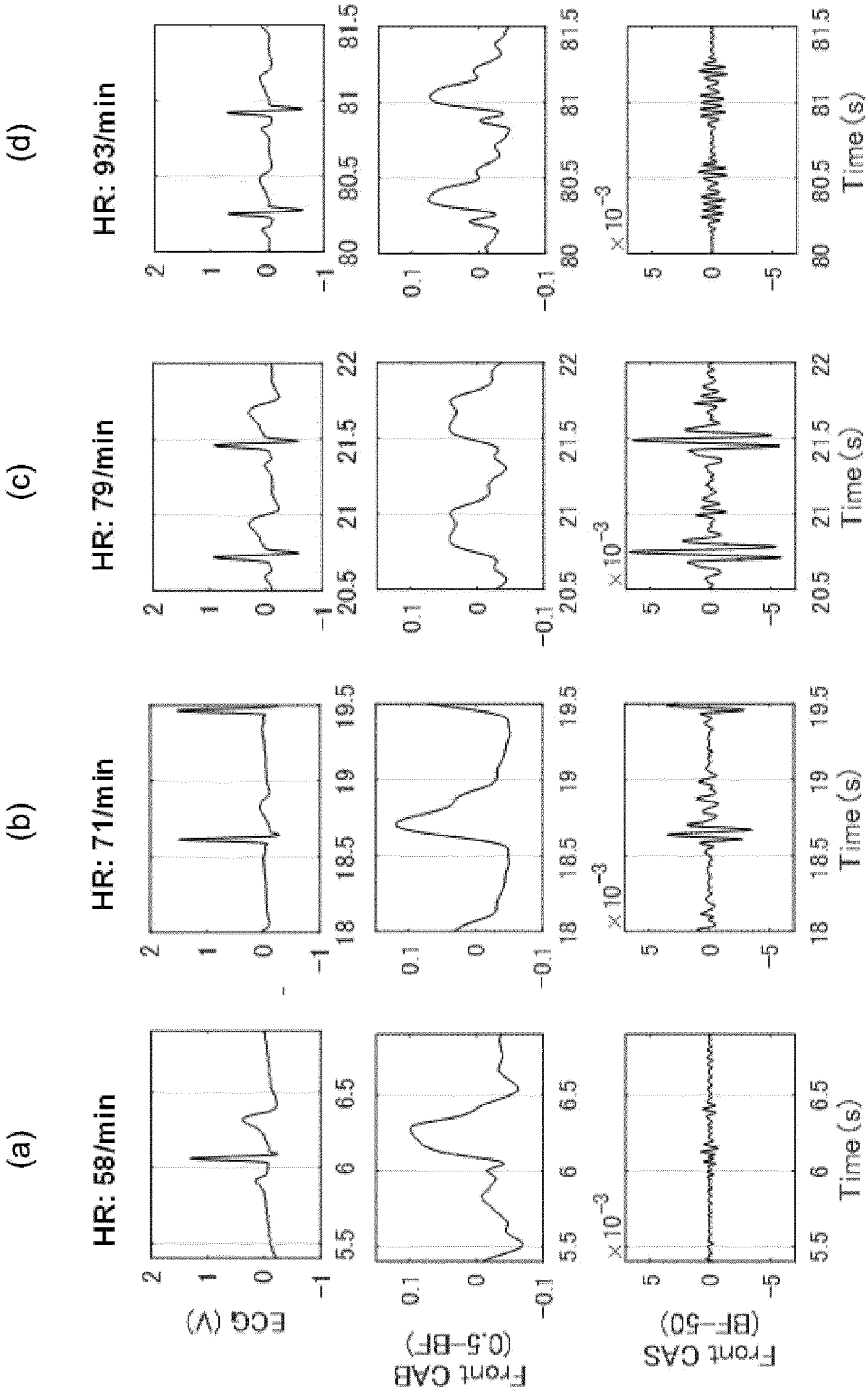
(a)
HR: 58/min
(b)
HR: 71/min
(c)
HR: 79/min
(d)
HR: 93/min
ECG (V)
Front CAB
(0.5–BF)
Front CAS
(BF–50)
×10^-3
Time (s)

FIG. 21

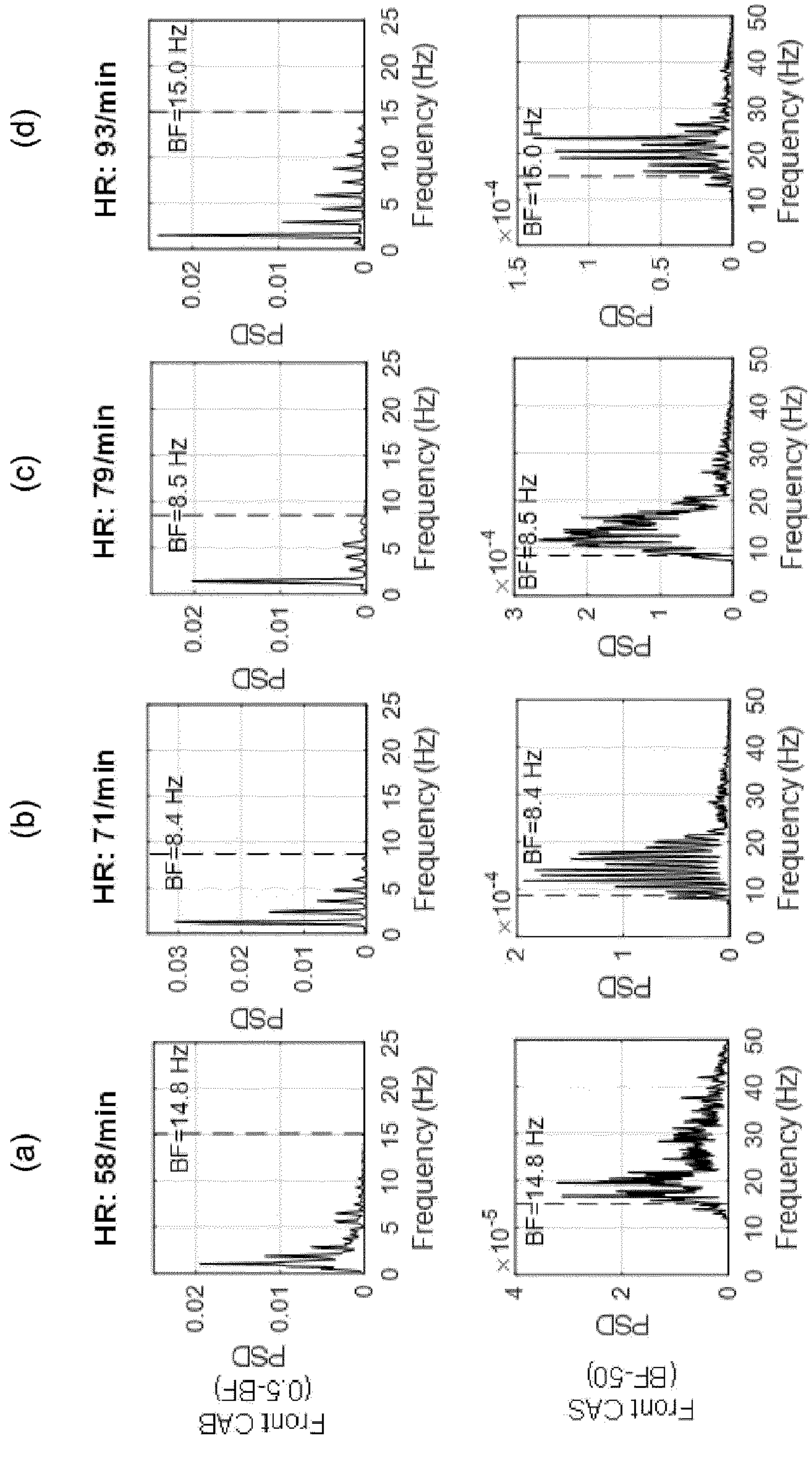

(a)
HR: 58/min
(b)
HR: 71/min
(c)
HR: 79/min
(d)
HR: 93/min
Front CAB
(0.5-BF)
Front CAS
(BF-50)
PSD
Frequency (Hz)
BF=14.8 Hz
BF=8.4 Hz
BF=8.5 Hz
BF=15.0 Hz

FIG. 22

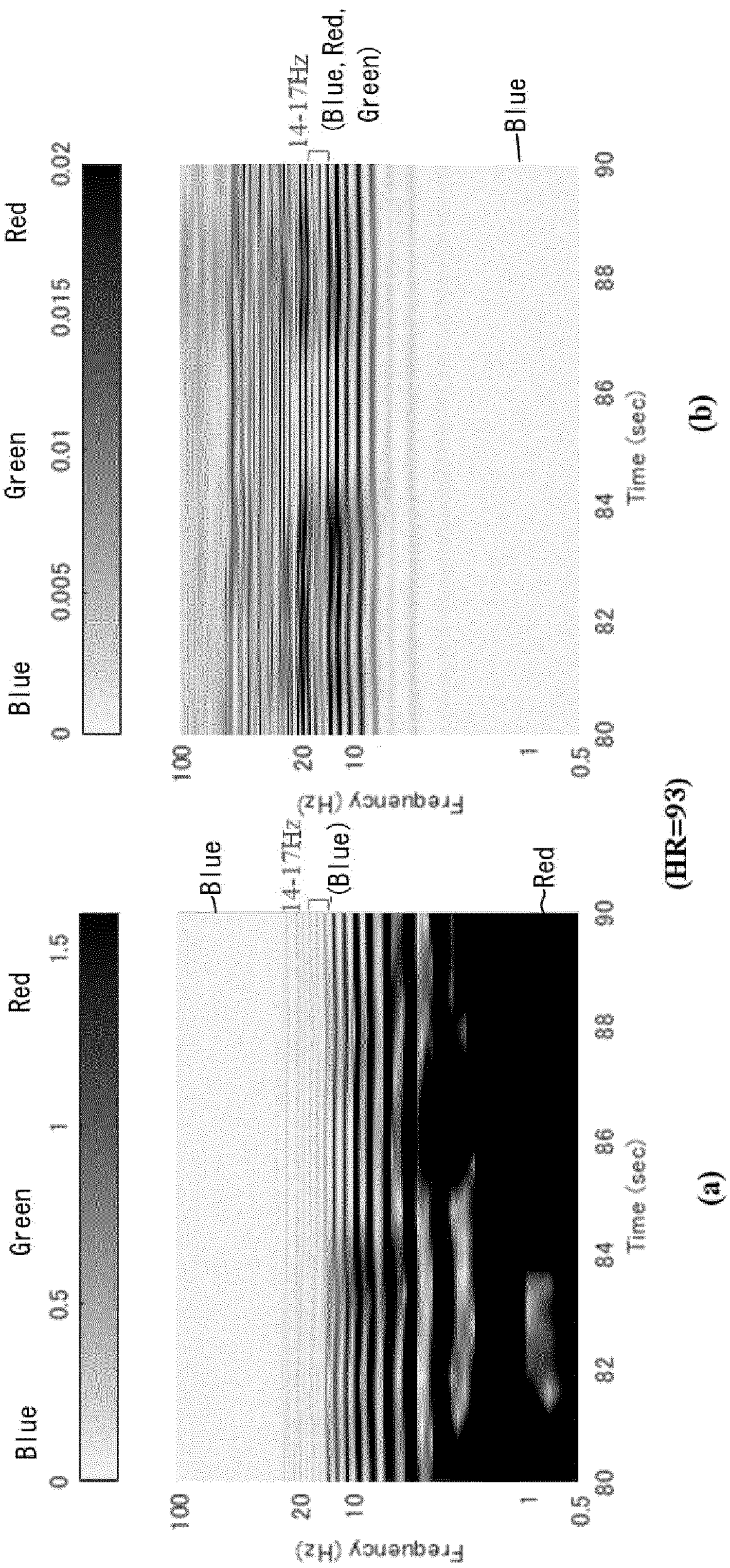
Blue
Green
Red
0
0.005
0.01
0.015
0.02
14-17Hz
(Blue, Red, Green)
Blue
Frequency (Hz)
100
20
10
1
0.5
80
82
84
86
88
90
Time (sec)
(b)
(HR=93)
Blue
Green
Red
0
0.5
1
1.5
Blue
14-17Hz
(Blue)
Red
Frequency (Hz)
100
20
10
1
0.5
80
82
84
86
88
90
Time (sec)
(a)

FIG. 23

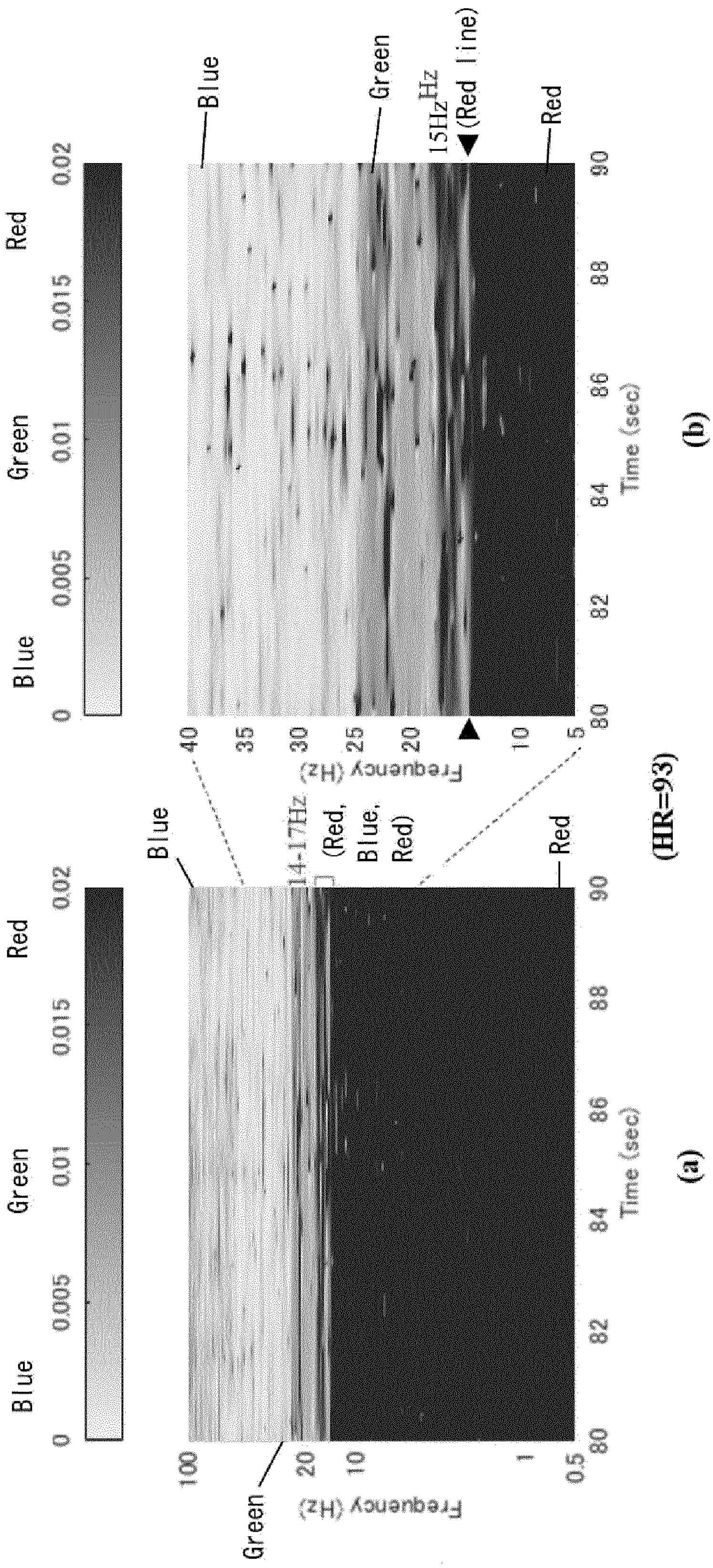
Blue
Green
Red
0
0.005
0.01
0.015
0.02
100
20
10
1
0.5
Frequency (Hz)
80
82
84
86
88
90
Time (sec)
14-17Hz
(Red, Blue, Red)
(a)
(HR=93)
40
35
30
25
15Hz
(Red line)
5
(b)

FIG. 24

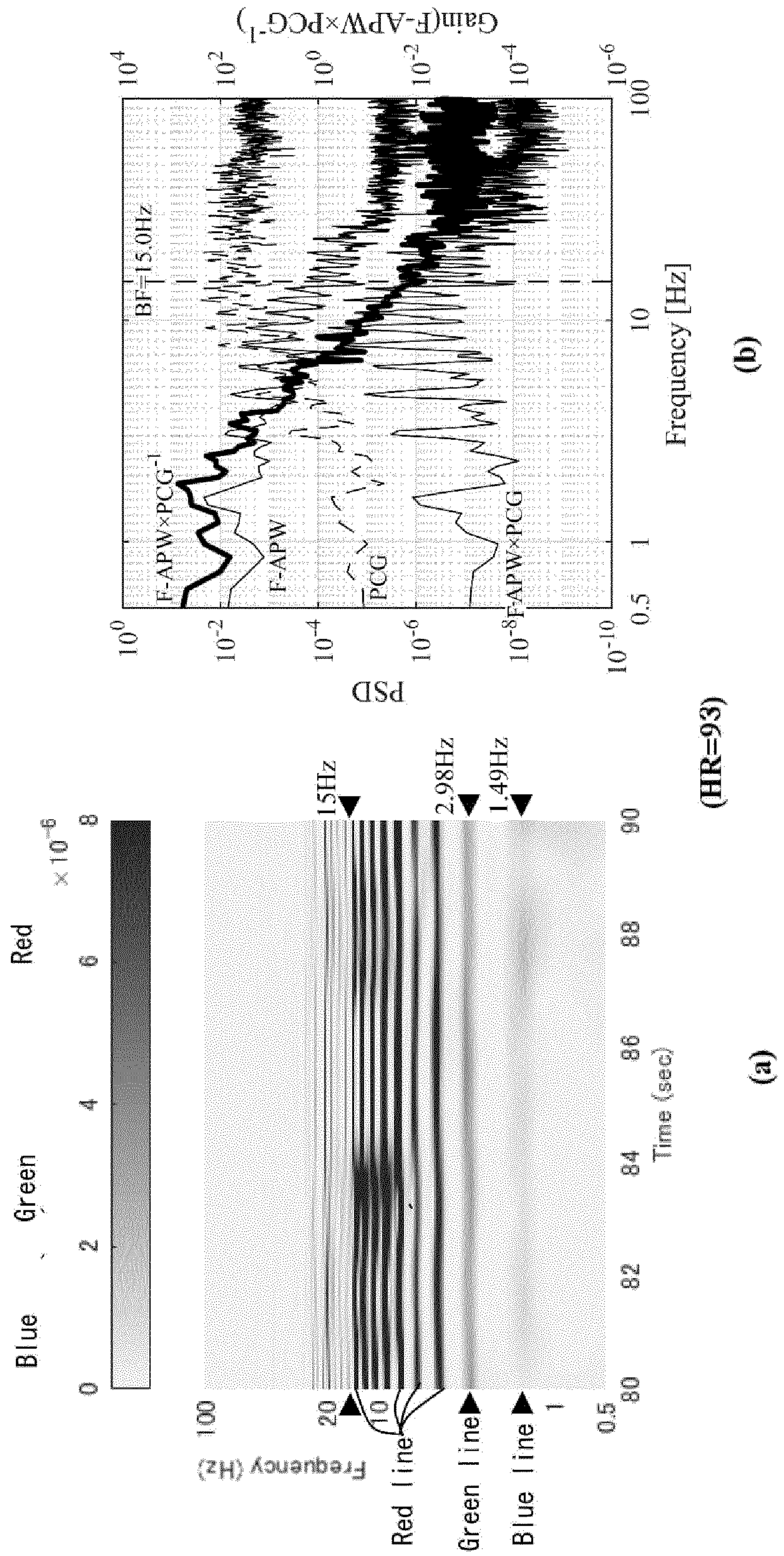
Blue
Green
Red
Frequency (Hz)
Time (sec)
Red line
Green line
Blue line
15Hz
2.98Hz
1.49Hz
(a)
(HR=93)
PSD
Frequency [Hz]
Gain(F-APW×PCG-1)
F-APW×PCG-1
F-APW
PCG
F-APW×PCG
BF=15.0Hz
(b)

FIG. 25

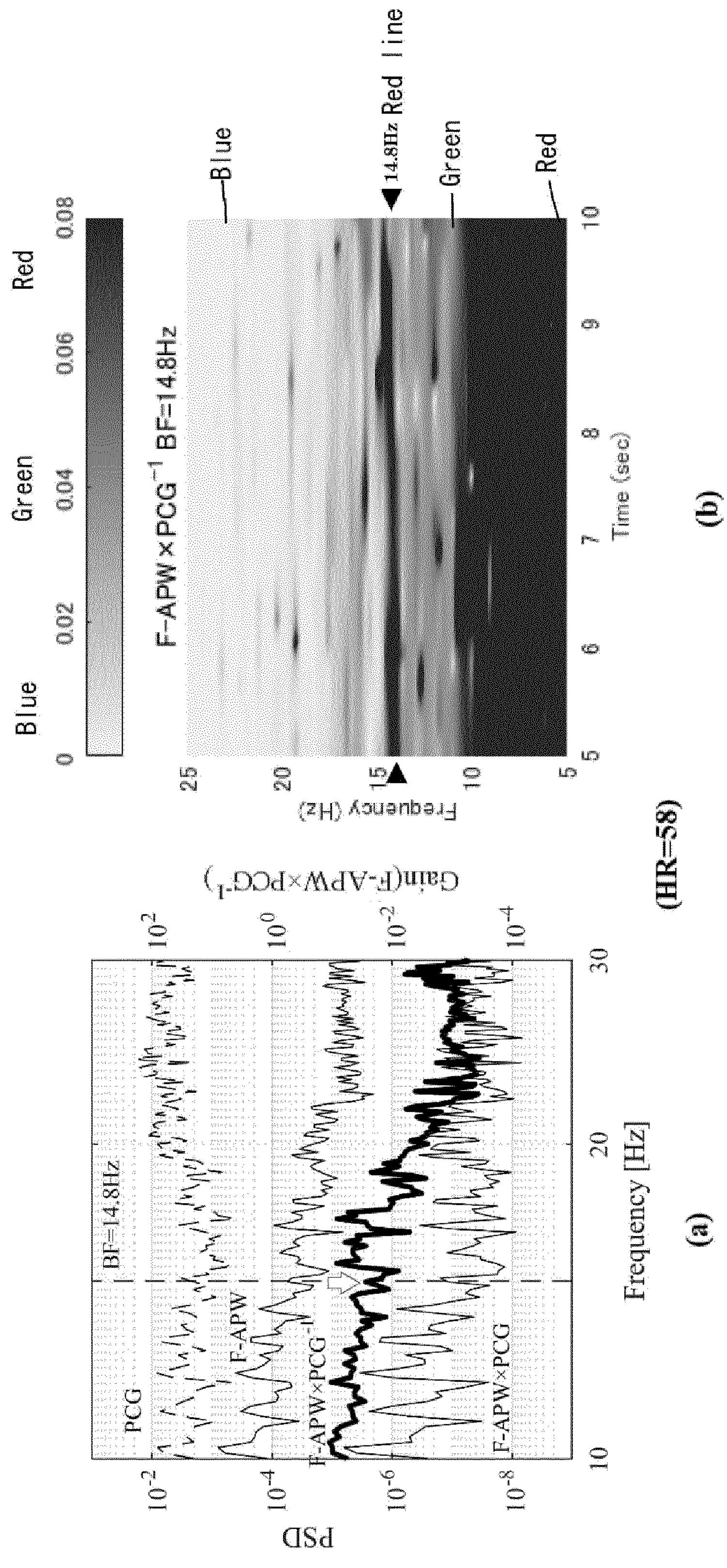
PSD
Gain(F-APW×PCG-1)
PCG
F-APW
F-APW×PCG-1
F-APW×PCG
BF=14.8Hz
Frequency [Hz]
(a)
(HR=58)
Blue
Green
Red
F-APW×PCG-1 BF=14.8Hz
14.8Hz Red line
Frequency (Hz)
Time (sec)
(b)

FIG. 26

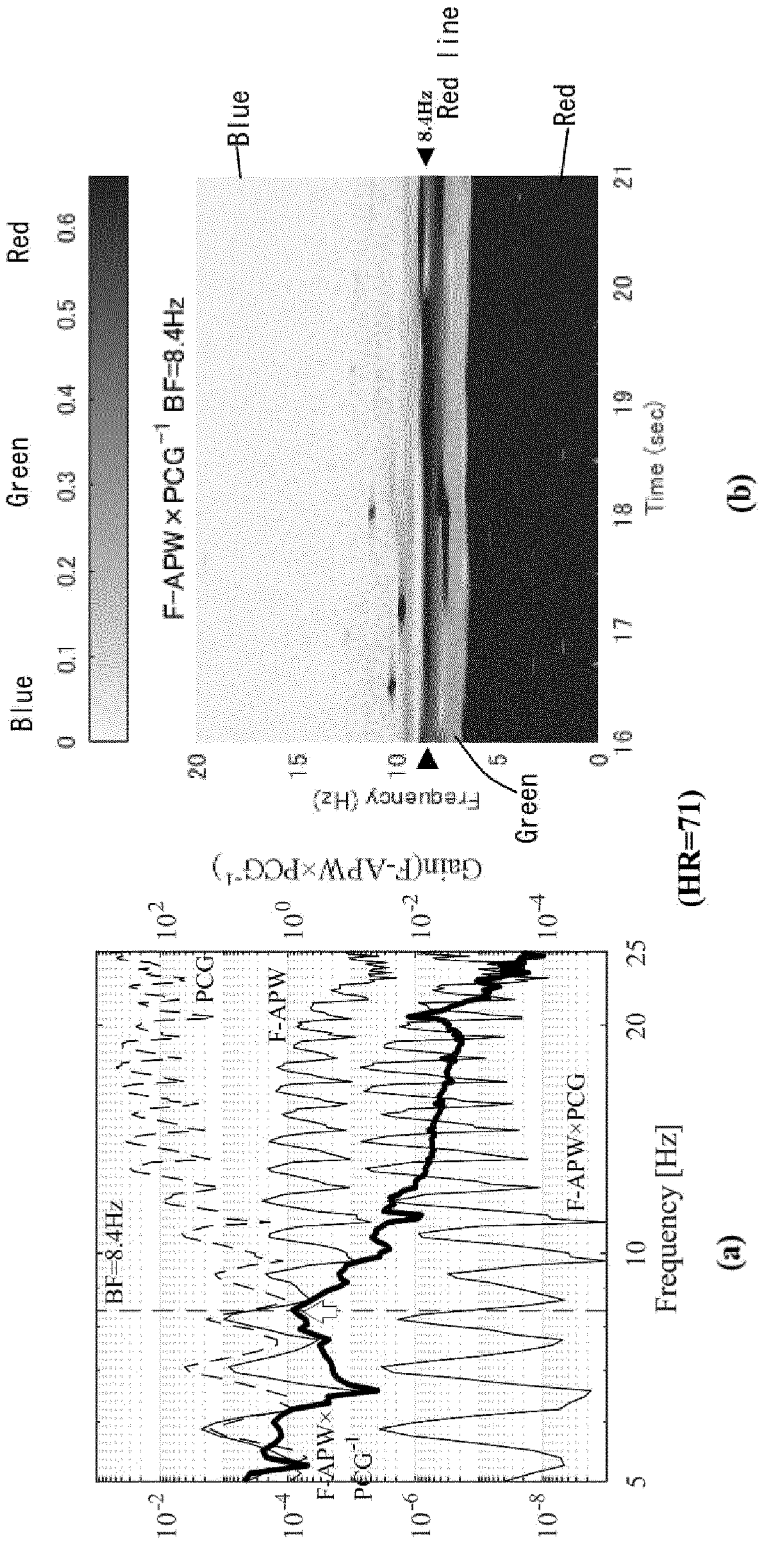
Blue
Green
Red
0
0.1
0.2
0.3
0.4
0.5
0.6
F−APW×PCG−1 BF=8.4Hz
Blue
8.4Hz
Red line
Red
Green
Frequency (Hz)
Time (sec)
(b)
(HR=71)
PSD
Gain(F-APW×PCG-1)
BF=8.4Hz
PCG
F-APW
F-APW×PCG
F-APW×PCG-1
Frequency [Hz]
(a)

FIG. 27

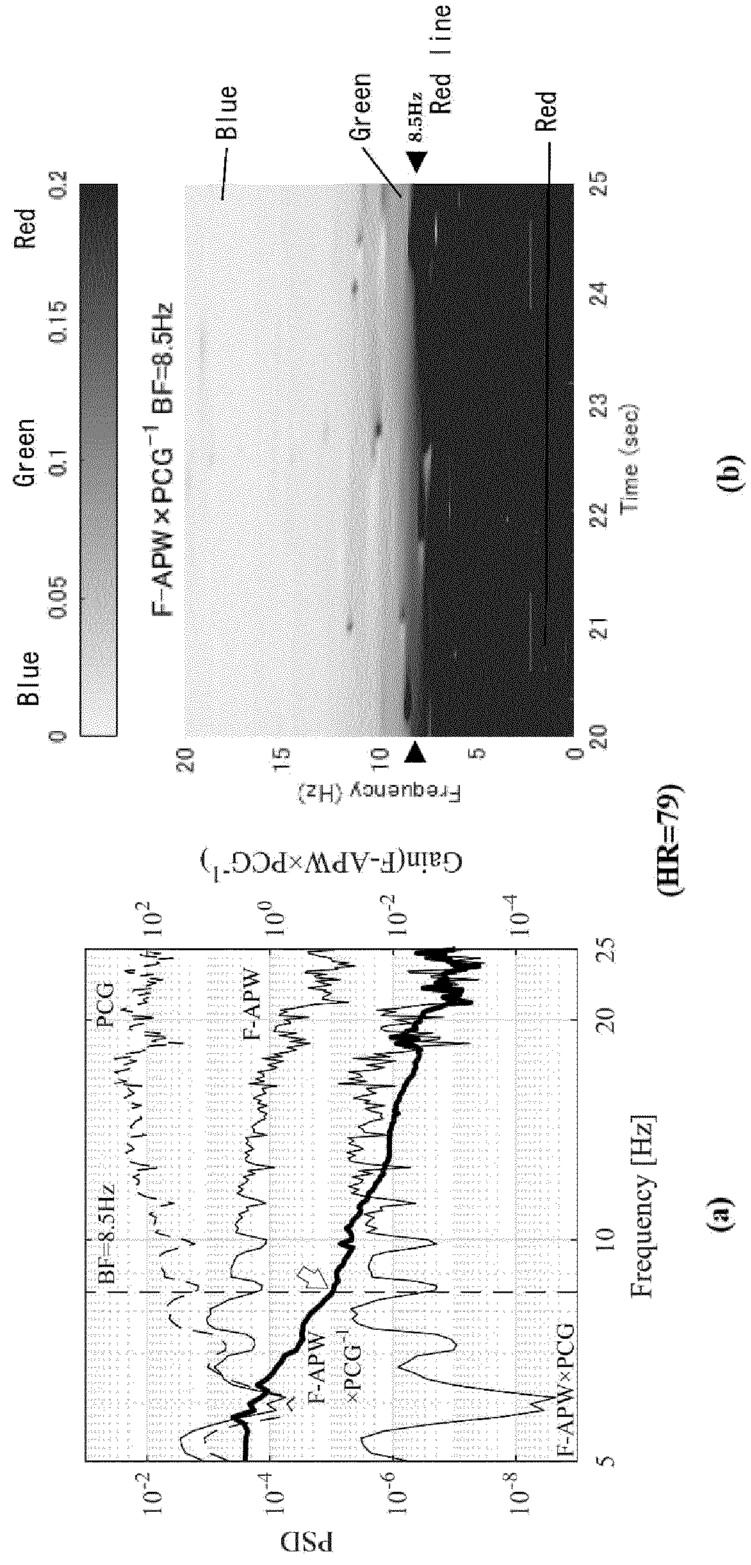

(a)
PSD
Frequency [Hz]
Gain(F-APW×PCG-1)
PCG
F-APW
BF=8.5Hz
F-APW ×PCG-1
F-APW×PCG
(HR=79)
(b)
F-APW×PCG-1 BF=8.5Hz
Blue
Green
Red
Red line
8.5Hz
Frequency (Hz)
Time (sec)

FIG. 28

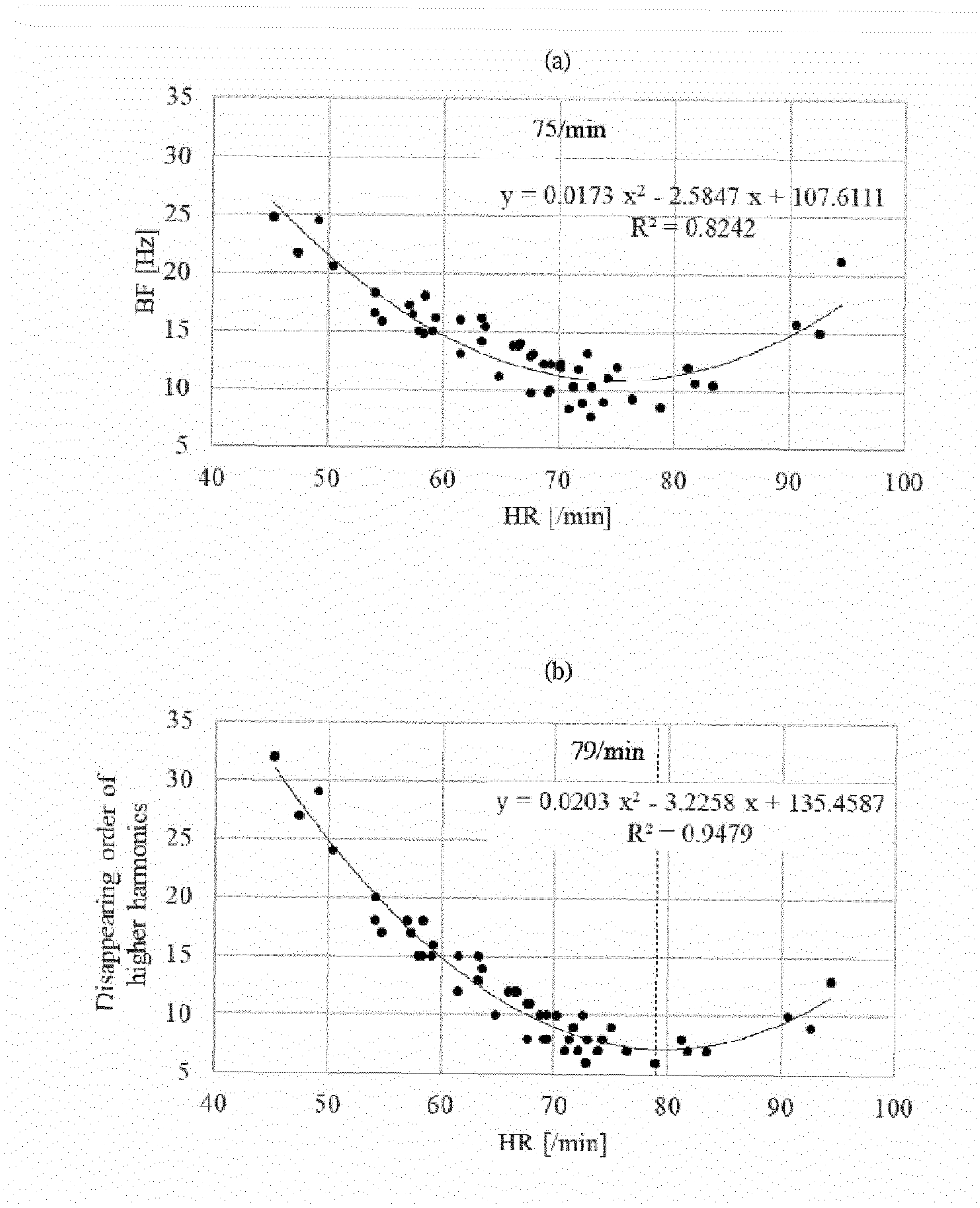
(a)
75/min
y = 0.0173 x² - 2.5847 x + 107.6111
R² = 0.8242
BF [Hz]
35
30
25
20
15
10
5
40
50
60
70
80
90
100
HR [/min]
(b)
79/min
y = 0.0203 x² - 3.2258 x + 135.4587
R² = 0.9479
Disappearing order of higher harmonics
35
30
25
20
15
10
5
40
50
60
70
80
90
100
HR [/min]

FIG. 29

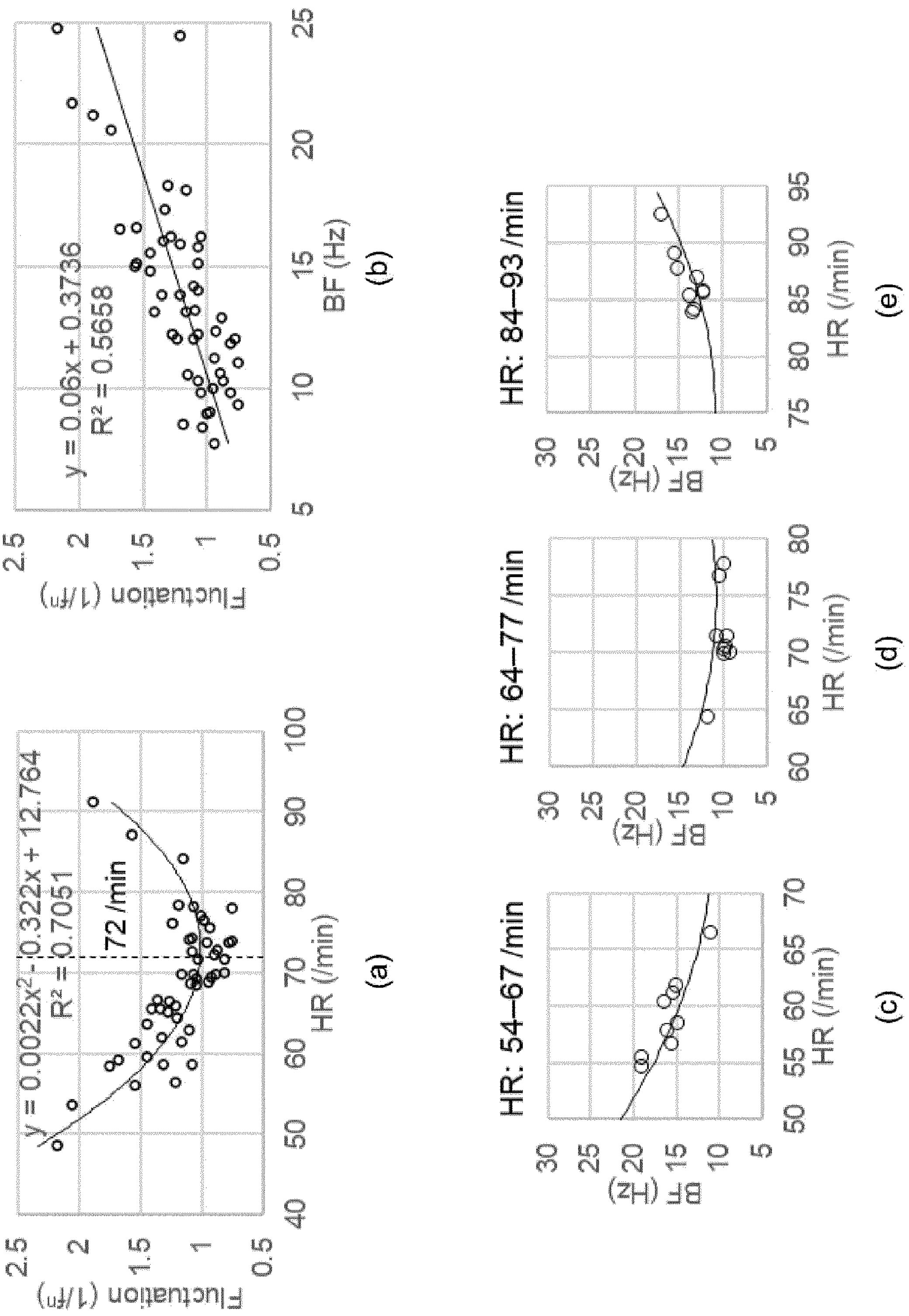
y = 0.0022$x^2$ - 0.322x + 12.764
$R^2$ = 0.7051
72 /min
Fluctuation (1/$f^n$)
HR (/min)
(a)
y = 0.06x + 0.3736
$R^2$ = 0.5658
Fluctuation (1/$f^n$)
BF (Hz)
(b)
HR: 54–67 /min
BF (Hz)
HR (/min)
(c)
HR: 64–77 /min
BF (Hz)
HR (/min)
(d)
HR: 84–93 /min
BF (Hz)
HR (/min)
(e)

FIG. 30

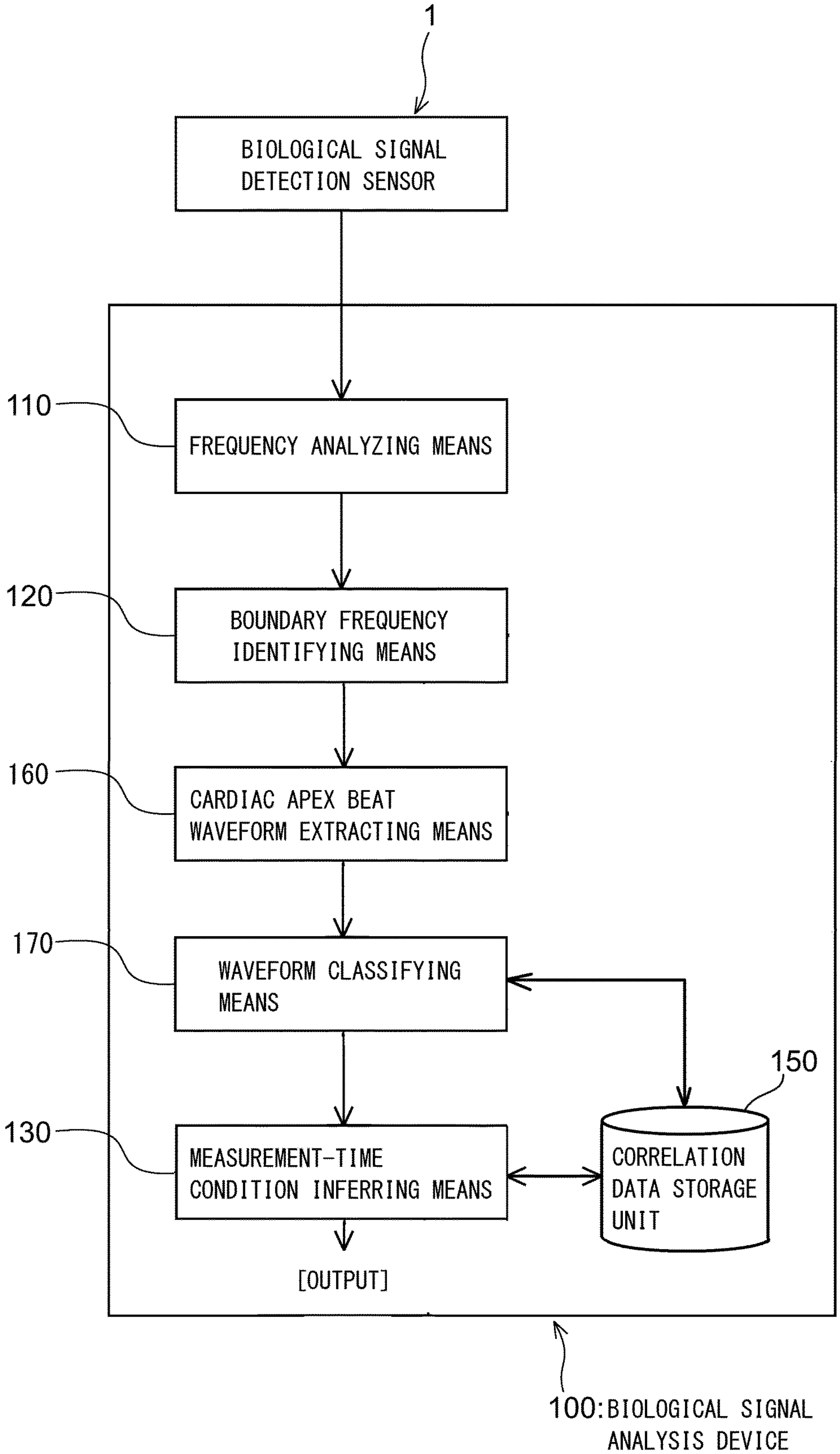
1
BIOLOGICAL SIGNAL DETECTION SENSOR
110
FREQUENCY ANALYZING MEANS
120
BOUNDARY FREQUENCY IDENTIFYING MEANS
160
CARDIAC APEX BEAT WAVEFORM EXTRACTING MEANS
170
WAVEFORM CLASSIFYING MEANS
150
130
MEASUREMENT-TIME CONDITION INFERRING MEANS
CORRELATION DATA STORAGE UNIT
[OUTPUT]
100:BIOLOGICAL SIGNAL ANALYSIS DEVICE

FIG. 31

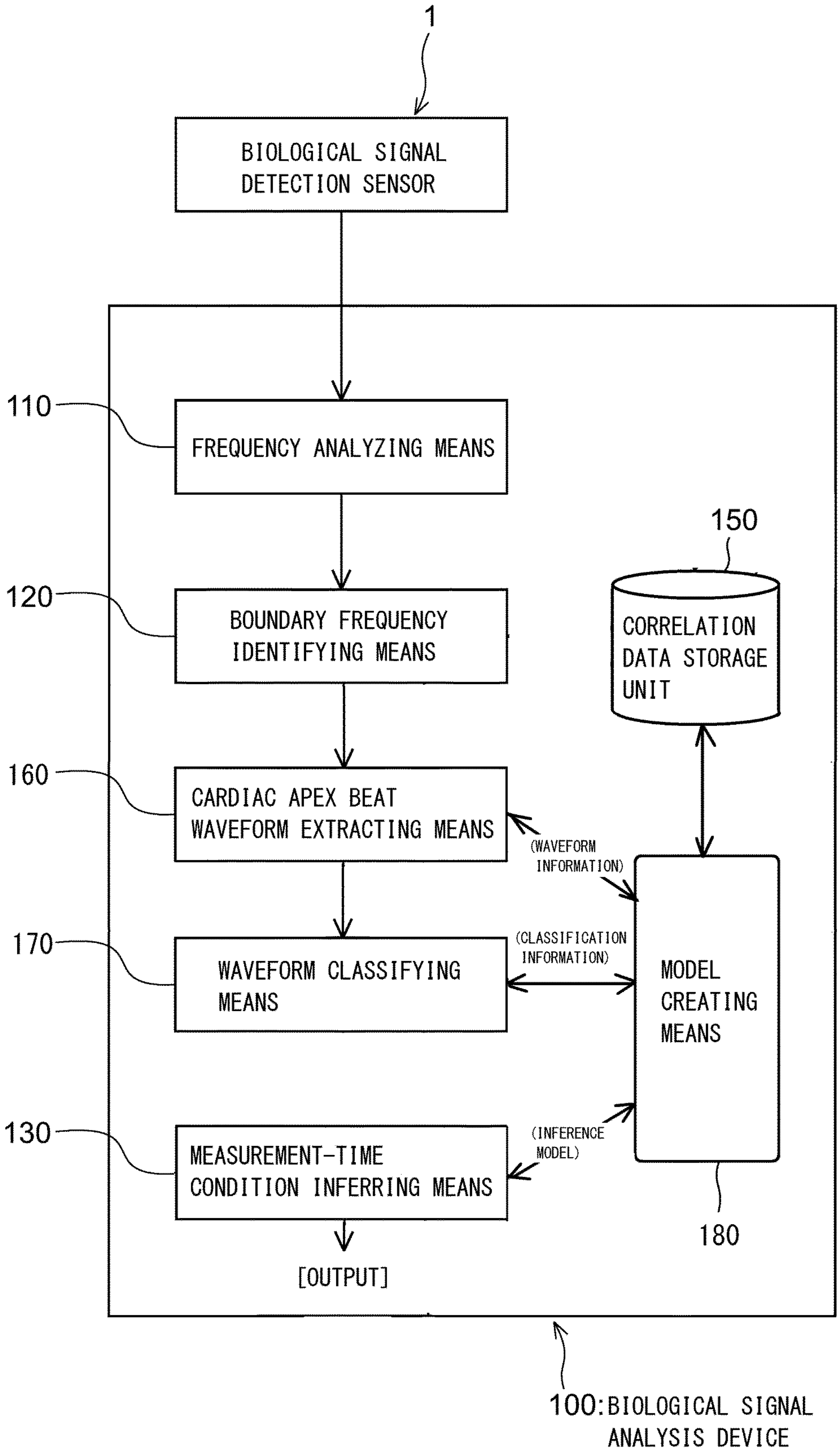
1
BIOLOGICAL SIGNAL DETECTION SENSOR
110
FREQUENCY ANALYZING MEANS
120
BOUNDARY FREQUENCY IDENTIFYING MEANS
150
CORRELATION DATA STORAGE UNIT
160
CARDIAC APEX BEAT WAVEFORM EXTRACTING MEANS
(WAVEFORM INFORMATION)
170
WAVEFORM CLASSIFYING MEANS
(CLASSIFICATION INFORMATION)
MODEL CREATING MEANS
130
MEASUREMENT-TIME CONDITION INFERRING MEANS
(INFERENCE MODEL)
180
[OUTPUT]
100:BIOLOGICAL SIGNAL ANALYSIS DEVICE

FIG. 32

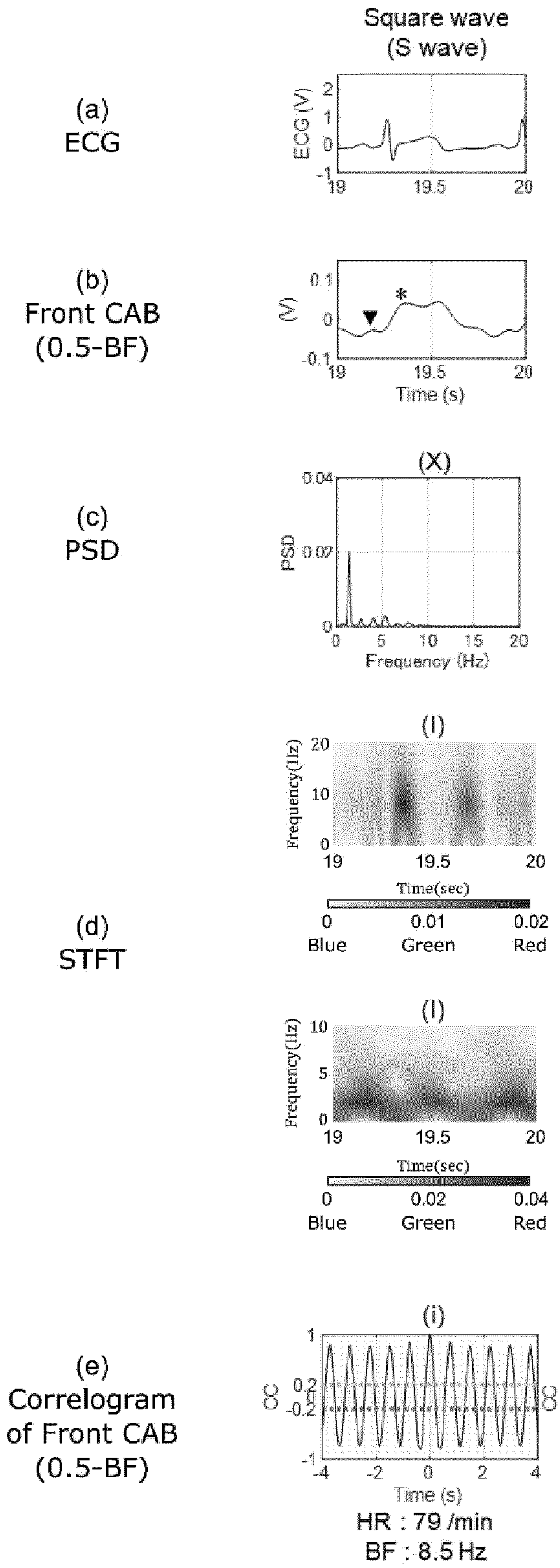

Square wave
(S wave)
(a)
ECG
ECG (V)
19.5
Time (s)
(b)
Front CAB
(0.5-BF)
(V)
(X)
(c)
PSD
PSD
Frequency (Hz)
(I)
Frequency(Hz)
Time(sec)
(d)
STFT
Blue
Green
Red
(e)
Correlogram
of Front CAB
(0.5-BF)
(i)
CC
HR : 79 /min
BF : 8.5 Hz

FIG. 33

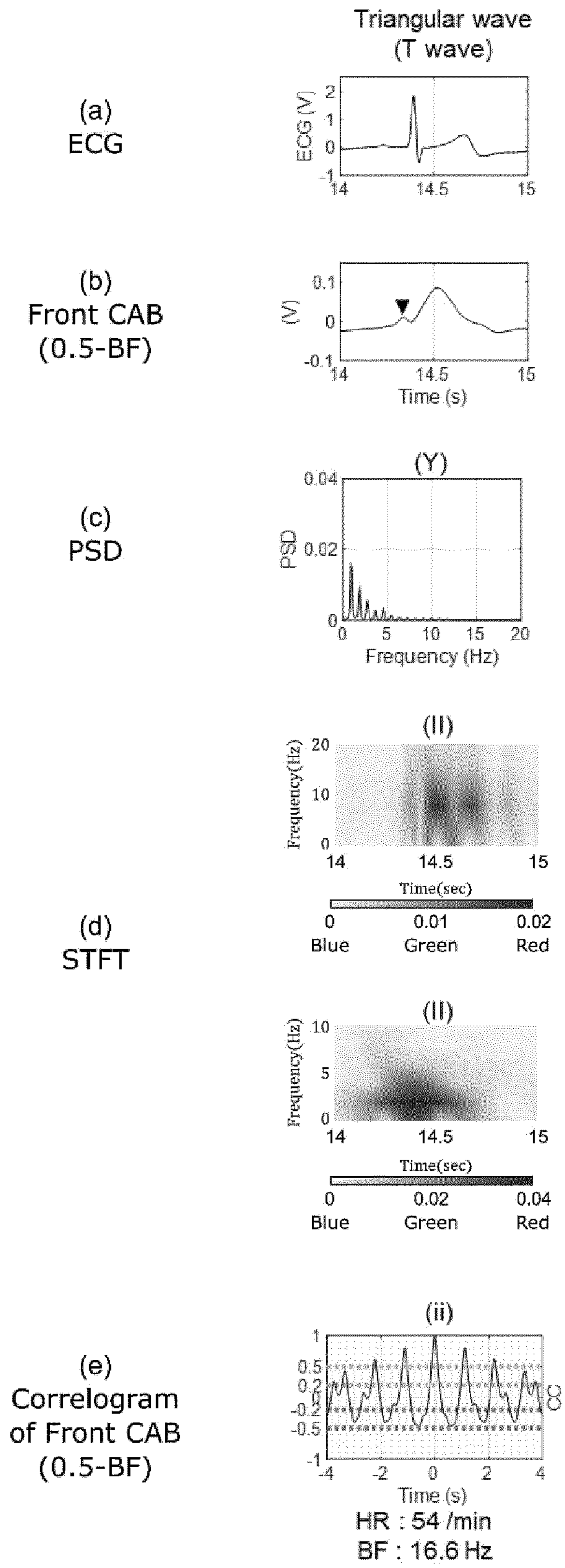
Triangular wave
(T wave)
(a)
ECG
ECG (V)
2
1
0
-1
14
14.5
15
(b)
Front CAB
(0.5-BF)
(V)
0.1
0
-0.1
14
14.5
15
Time (s)
(c)
PSD
(Y)
0.04
PSD
0.02
0
0
5
10
15
20
Frequency (Hz)
(II)
Frequency(Hz)
20
10
0
14
14.5
15
Time(sec)
0
0.01
0.02
Blue
Green
Red
(d)
STFT
(II)
Frequency(Hz)
10
5
0
14
14.5
15
Time(sec)
0
0.02
0.04
Blue
Green
Red
(e)
Correlogram
of Front CAB
(0.5-BF)
(ii)
1
0.5
0.2
0
-0.2
-0.5
-1
CC
-4
-2
0
2
4
Time (s)
HR : 54 /min
BF : 16.6 Hz

FIG. 34

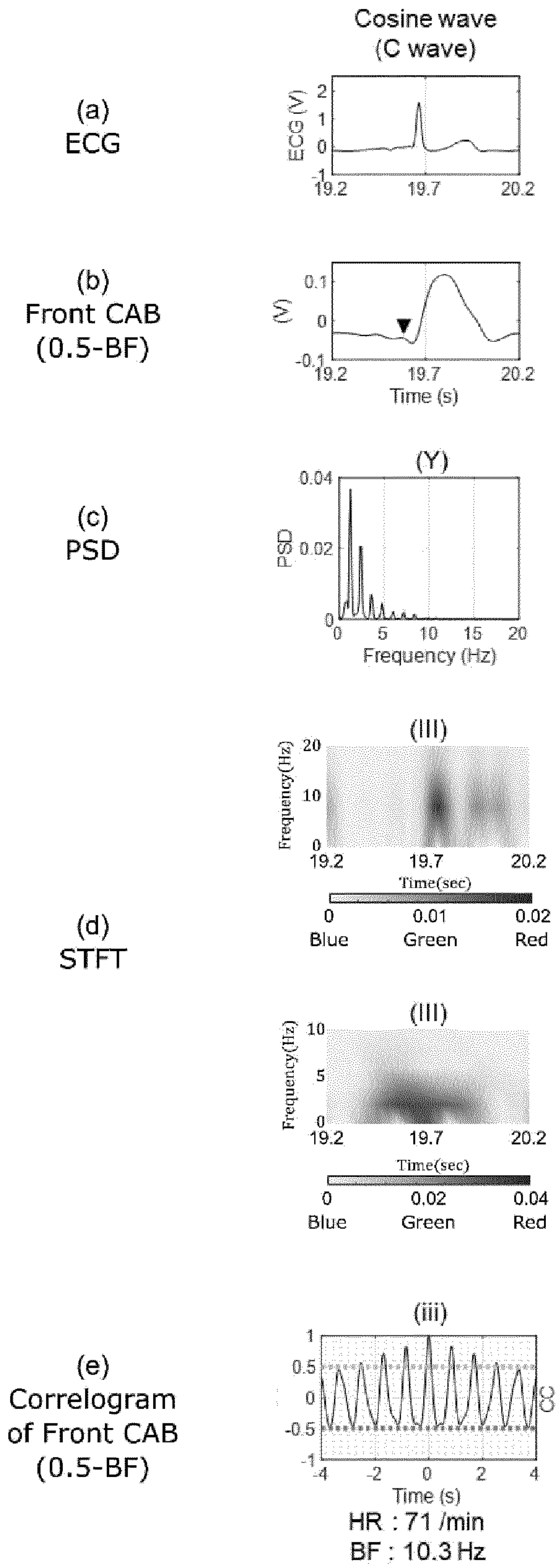
Cosine wave
(C wave)
(a)
ECG
ECG (V)
(b)
Front CAB
(0.5-BF)
(V)
Time (s)
(c)
PSD
(Y)
PSD
Frequency (Hz)
(d)
STFT
(III)
Frequency(Hz)
Time(sec)
0 Blue
0.01 Green
0.02 Red
(III)
Frequency(Hz)
Time(sec)
0 Blue
0.02 Green
0.04 Red
(e)
Correlogram
of Front CAB
(0.5-BF)
(iii)
CC
Time (s)
HR : 71 /min
BF : 10.3 Hz

FIG. 35

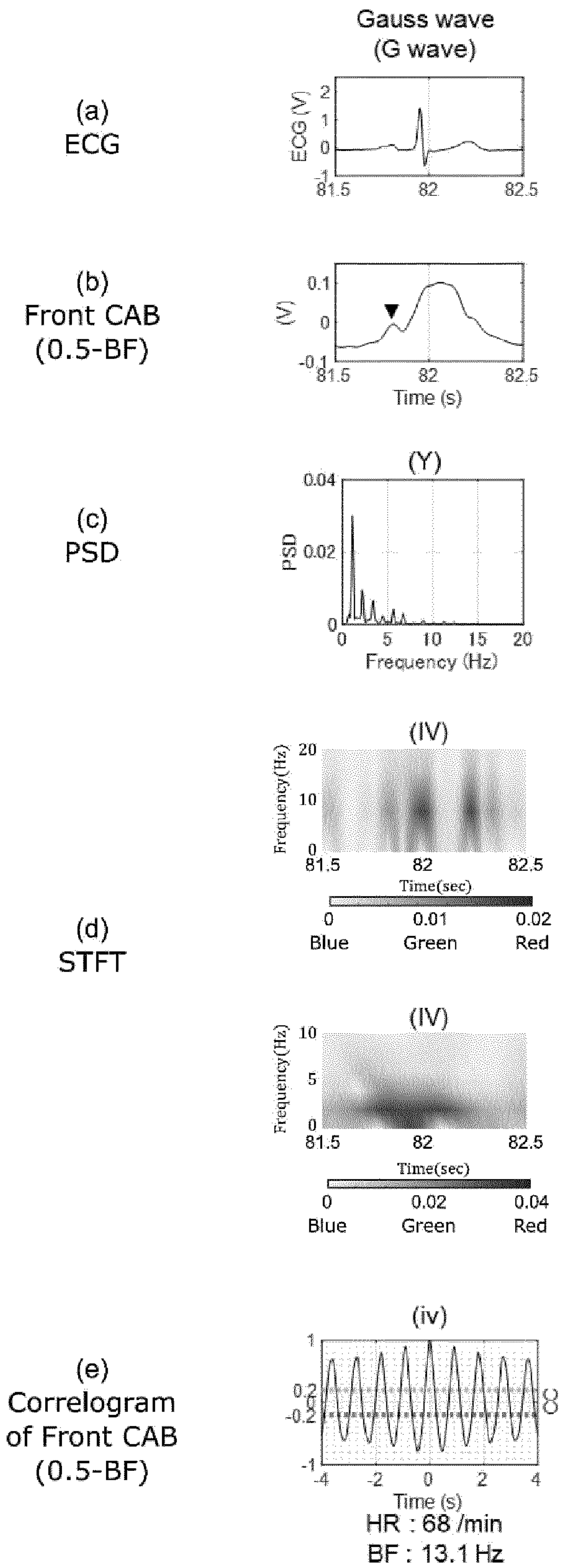
Gauss wave
(G wave)
(a)
ECG
ECG (V)
2
1
0
-1
81.5
82
82.5
(b)
Front CAB
(0.5-BF)
(V)
0.1
0
-0.1
81.5
82
82.5
Time (s)
(c)
PSD
(Y)
0.04
PSD
0.02
0
0
5
10
15
20
Frequency (Hz)
(d)
STFT
(IV)
Frequency(Hz)
20
10
0
81.5
82
82.5
Time(sec)
0
0.01
0.02
Blue
Green
Red
(IV)
Frequency(Hz)
10
5
0
81.5
82
82.5
Time(sec)
0
0.02
0.04
Blue
Green
Red
(e)
Correlogram
of Front CAB
(0.5-BF)
(iv)
1
0.2
0
-0.2
-1
-4
-2
0
2
4
Time (s)
HR : 68 /min
BF : 13.1 Hz

FIG. 36

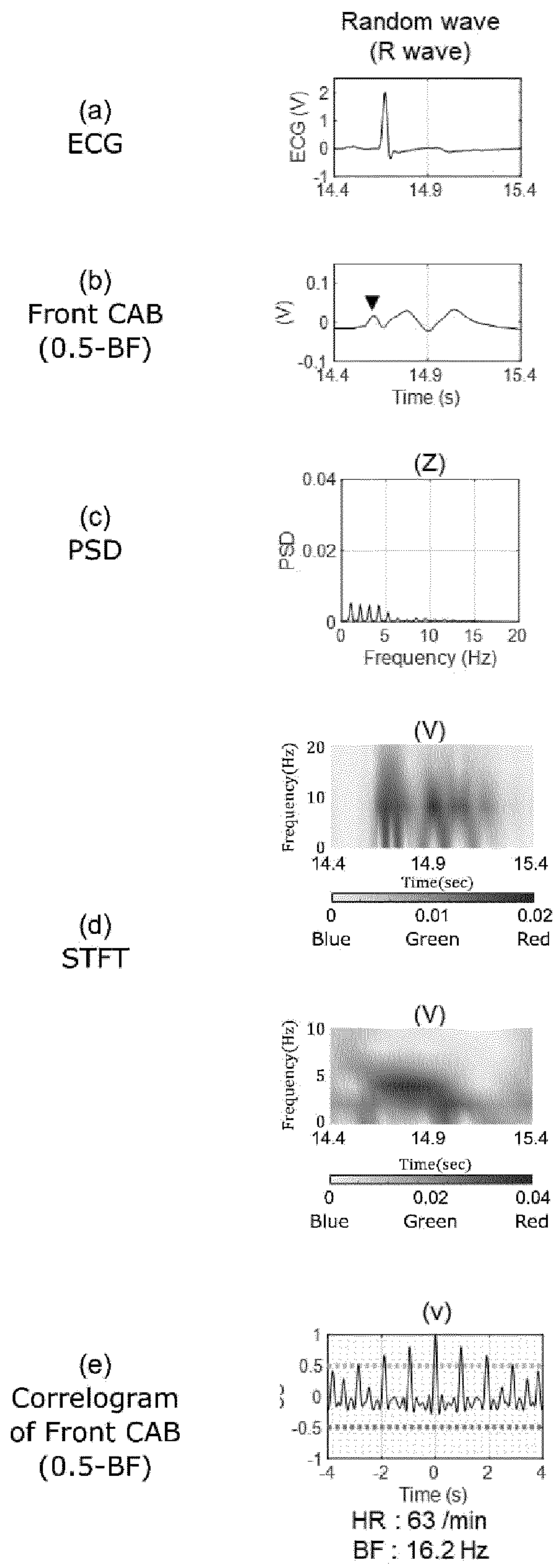
Random wave
(R wave)
(a)
ECG
ECG (V)
2
1
0
-1
14.4
14.9
15.4
(b)
Front CAB
(0.5-BF)
(V)
0.1
0
-0.1
14.4
14.9
15.4
Time (s)
(Z)
(c)
PSD
PSD
0.04
0.02
0
0
5
10
15
20
Frequency (Hz)
(V)
Frequency(Hz)
20
10
0
14.4
14.9
15.4
Time(sec)
0
0.01
0.02
Blue
Green
Red
(d)
STFT
(V)
Frequency(Hz)
10
5
0
14.4
14.9
15.4
Time(sec)
0
0.02
0.04
Blue
Green
Red
(v)
(e)
Correlogram
of Front CAB
(0.5-BF)
1
0.5
0
-0.5
-1
-4
-2
0
2
4
Time (s)
HR : 63 /min
BF : 16.2 Hz

FIG. 37

| Wave / STFT | S wave | T wave | G wave | G wave | R wave | Total |
|---|---|---|---|---|---|---|
| | 4 | 8 | 15 | 11 | 12 | 50 |
| I | **4** | 0 | 0 | 0 | 1 | 5 |
| II | 0 | **8** | 0 | 1 | 1 | 10 |
| III | 0 | 0 | **14** | 0 | 2 | 16 |
| IV | 0 | 0 | 0 | **10** | 0 | 10 |
| V | 0 | 0 | 1 | 0 | **8** | 9 |

$P=2.2 \times 10^{-16}$

| Wave / Power spectrum | S wave | T wave | G wave | G wave | R wave | Total |
|---|---|---|---|---|---|---|
| | 4 | 8 | 15 | 11 | 12 | 50 |
| X | **3** | 1 | 0 | 1 | 0 | 5 |
| Y | 1 | **5** | **15** | **10** | 7 | 38 |
| Z | 0 | 2 | 0 | 0 | **5** | 7 |

$P=7.9 \times 10^{-5}$

| Wave / Correlo-gram | S wave | T wave | G wave | G wave | R wave | Total |
|---|---|---|---|---|---|---|
| | 4 | 8 | 15 | 11 | 12 | 50 |
| i | **4** | 0 | 0 | 0 | 0 | 4 |
| ii | 0 | **7** | 1 | 0 | 1 | 9 |
| iii | 0 | 0 | **14** | 1 | 3 | 18 |
| iv | 0 | 0 | 0 | **10** | 0 | 10 |
| v | 0 | 1 | 0 | 0 | **8** | 9 |

$P=2.2 \times 10^{-16}$

**Classification of waveform**

FIG. 38

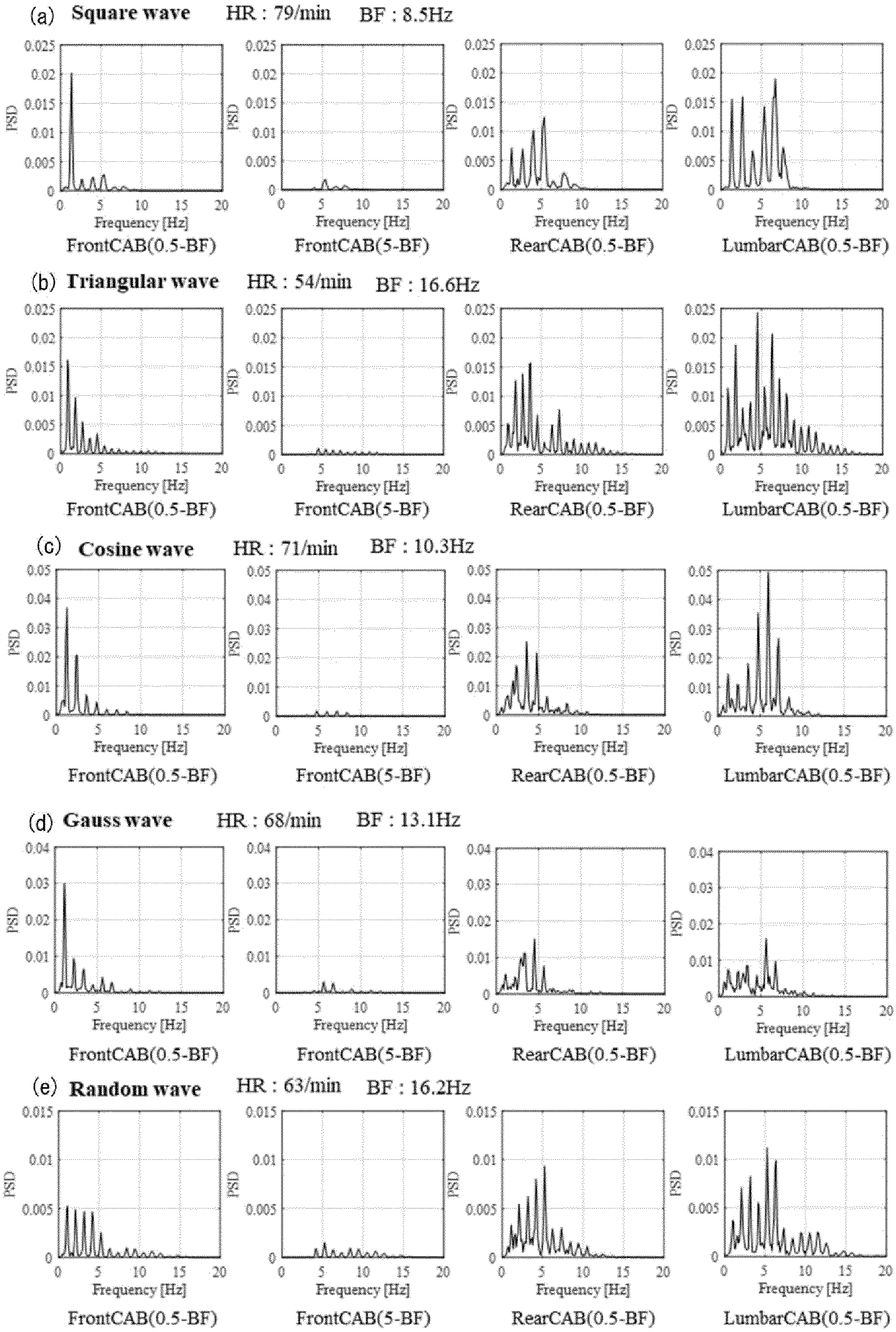
(a) Square wave HR : 79/min BF : 8.5Hz
FrontCAB(0.5-BF)
FrontCAB(5-BF)
RearCAB(0.5-BF)
LumbarCAB(0.5-BF)
(b) Triangular wave HR : 54/min BF : 16.6Hz
FrontCAB(0.5-BF)
FrontCAB(5-BF)
RearCAB(0.5-BF)
LumbarCAB(0.5-BF)
(c) Cosine wave HR : 71/min BF : 10.3Hz
FrontCAB(0.5-BF)
FrontCAB(5-BF)
RearCAB(0.5-BF)
LumbarCAB(0.5-BF)
(d) Gauss wave HR : 68/min BF : 13.1Hz
FrontCAB(0.5-BF)
FrontCAB(5-BF)
RearCAB(0.5-BF)
LumbarCAB(0.5-BF)
(e) Random wave HR : 63/min BF : 16.2Hz
FrontCAB(0.5-BF)
FrontCAB(5-BF)
RearCAB(0.5-BF)
LumbarCAB(0.5-BF)
PSD
Frequency [Hz]

FIG. 39

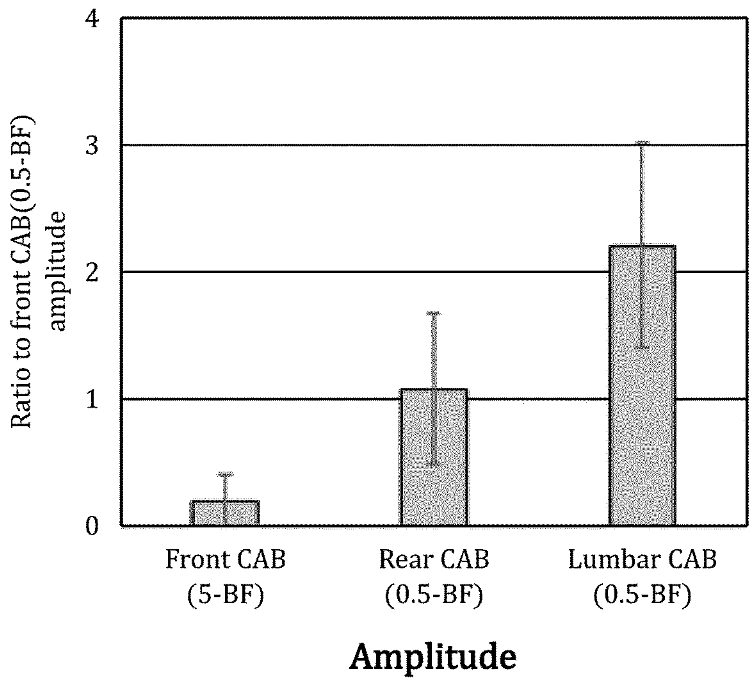
4
3
2
1
0
Ratio to front CAB(0.5-BF) amplitude
Front CAB (5-BF)
Rear CAB (0.5-BF)
Lumbar CAB (0.5-BF)
Amplitude

FIG. 40

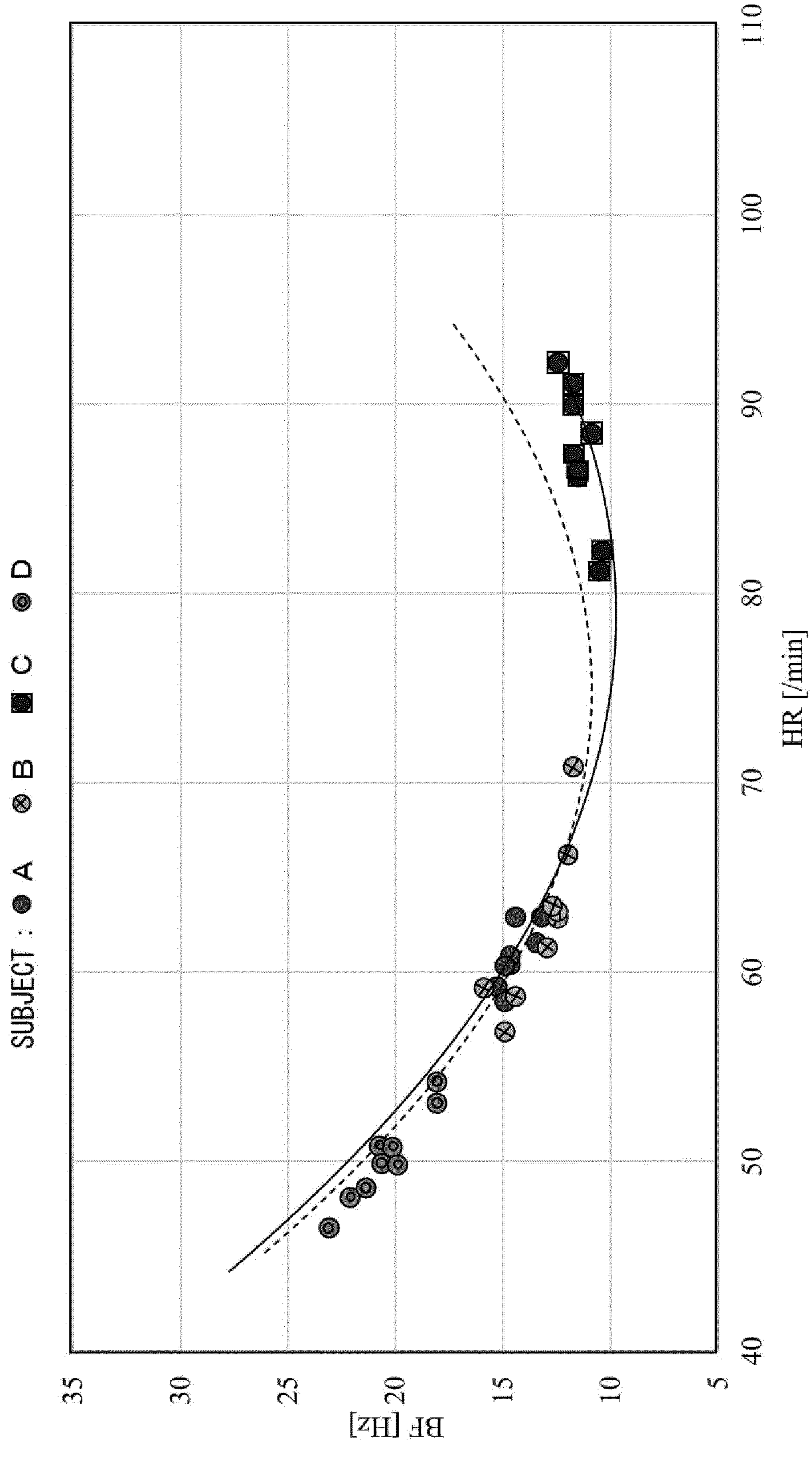
SUBJECT : A B C D
HR [/min]
BF [Hz]
40
50
60
70
80
90
100
110
5
10
15
20
25
30
35

FIG. 41

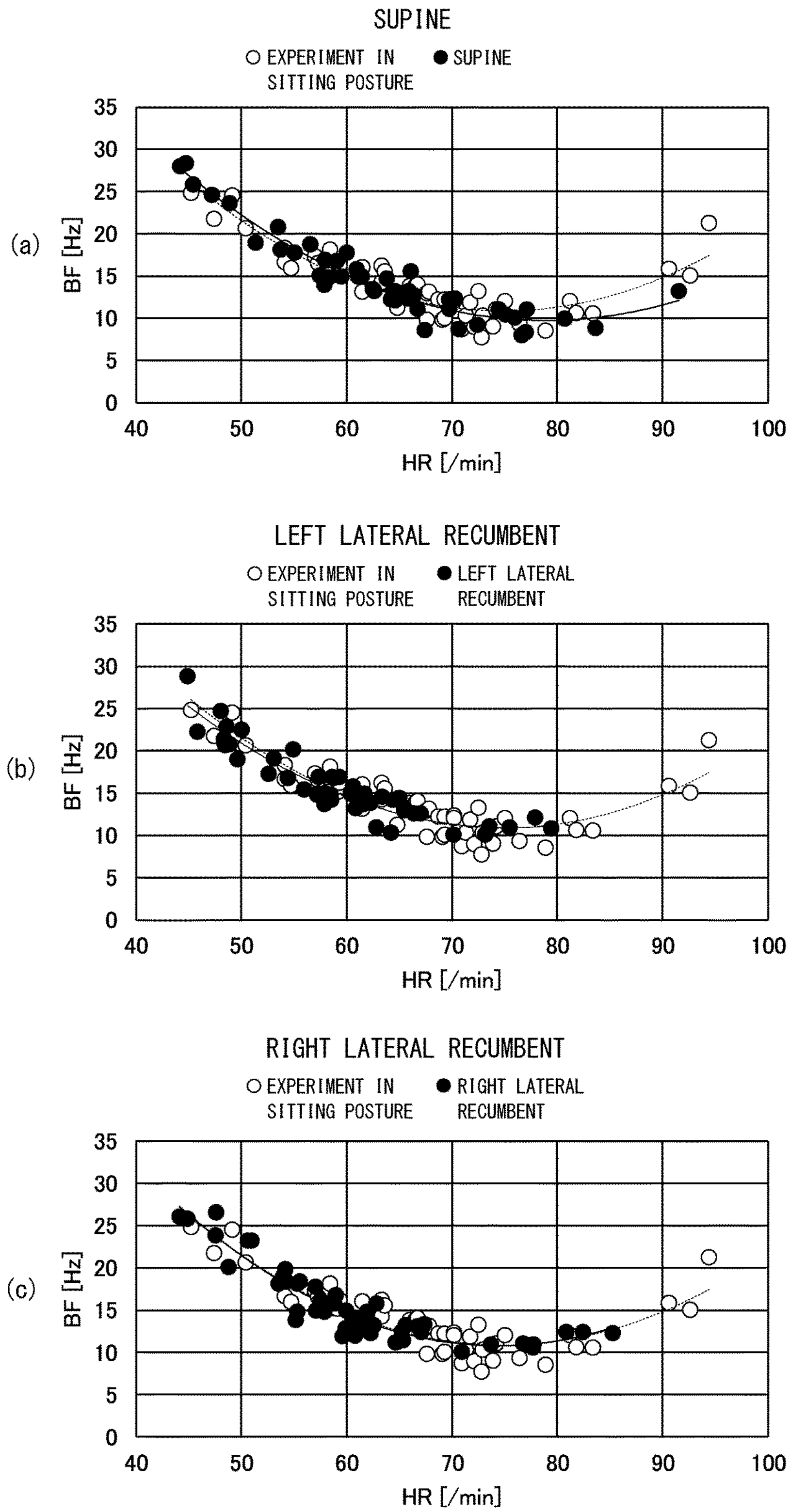
SUPINE
EXPERIMENT IN SITTING POSTURE
SUPINE
(a)
BF [Hz]
HR [/min]
LEFT LATERAL RECUMBENT
EXPERIMENT IN SITTING POSTURE
LEFT LATERAL RECUMBENT
(b)
BF [Hz]
HR [/min]
RIGHT LATERAL RECUMBENT
EXPERIMENT IN SITTING POSTURE
RIGHT LATERAL RECUMBENT
(c)
BF [Hz]
HR [/min]

## INTERNATIONAL SEARCH REPORT

International application No.
**PCT/JP2021/039924**

**A. CLASSIFICATION OF SUBJECT MATTER**

***A61B 5/02***(2006.01)i; ***A61B 5/0245***(2006.01)i; ***A61B 5/11***(2006.01)i
FI: A61B5/11 100; A61B5/0245 100E; A61B5/02 350

According to International Patent Classification (IPC) or to both national classification and IPC

**B. FIELDS SEARCHED**

Minimum documentation searched (classification system followed by classification symbols)

A61B5/02; A61B5/0245; A61B5/11

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2020-65818 A (DELTA TOOLING CO., LTD.) 30 April 2020 (2020-04-30) paragraphs [0019]-[0056] | 1, 17, 31 |
| Y | paragraphs [0019]-[0056] | 15-16 |
| A | paragraphs [0019]-[0056] | 2-14, 18-30 |
| Y | JP 2008-511396 A (WELCH ALLYN INC.) 17 April 2008 (2008-04-17) paragraphs [0029], [0030], fig. 7, 7a | 15-16 |
| A | JP 46-6279 B1 (RION CO., LTD.) 17 February 1971 (1971-02-17) entire text, all drawings | 1-31 |
| A | JP 2020-69249 A (KUROKI, Shigehiro) 07 May 2020 (2020-05-07) entire text, all drawings | 1-31 |
| A | US 2010/0087746 A1 (RADZIEVSKY, Naira) 08 April 2010 (2010-04-08) entire text, all drawings | 1 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 January 2022** | **18 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

## INTERNATIONAL SEARCH REPORT

International application No.
**PCT/JP2021/039924**

**C. DOCUMENTS CONSIDERED TO BE RELEVANT**

| Category* | Citation of document, with indication, where appropriate, of the relevant passages | Relevant to claim No. |
|---|---|---|
| P, A | WO 2020/262563 A1 (DELTA KOGYO CO., LTD.) 30 December 2020 (2020-12-30) entire text, all drawings | 1 |
| P, Y | paragraph [0025] | 15-16 |

Form PCT/ISA/210 (second sheet) (January 2015)

**INTERNATIONAL SEARCH REPORT**
**Information on patent family members**

International application No.
**PCT/JP2021/039924**

| Patent document cited in search report | Publication date (day/month/year) | Patent family member(s) | Publication date (day/month/year) |
|---|---|---|---|
| JP 2020-65818 A | 30 April 2020 | CN 112911995 A<br>paragraphs [0072]-[0119]<br>WO 2020/085512 A1<br>paragraphs [0019]-[0056] | |
| JP 2008-511396 A | 17 April 2008 | US 2006/0047215 A1<br>paragraphs [0041], [0042], fig. 7A<br>WO 2006/028687 A1<br>CA 2577751 A1 | |
| JP 46-6279 B1 | 17 February 1971 | (Family: none) | |
| JP 2020-69249 A | 07 May 2020 | (Family: none) | |
| US 2010/0087746 A1 | 08 April 2010 | WO 2008/072233 A1<br>entire text, all drawings<br>CN 101594826 A | |
| WO 2020/262563 A1 | 30 December 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012165660 A
- JP 2011167362 A
- JP 2014117425 A
- JP 2014223271 A
- JP 2016026516 A


### Non-patent literature cited in the description


- **NOBUHIRO, Y et al.** Development of Physical Condition Fluctuation Prediction Model Using Trunk Biosignals. *Proceeding of Asia-Pacific Vibration Conference,* 2019, vol. 2019
- **YOSHIKAWA, J et al.** Physical Examination in Cardiology. Bunkodo Shoten Co., Ltd, 2005, 79-95
- **KOBAYASHI M. ; MUSHA T.** 1/f fluctuation of heart beat period. IEEE Transactions on biomedical engineering. *BME,* 1982, vol. 29, 45-457